# EUROPEAN PATENT APPLICATION

(11) **EP 4 606 794 A1**
(43) Date of publication of application: **27.08.2025**
(21) Application number: 23878627.1
(22) Date of filing: 18.05.2023
(51) Int. Cl.: C07D 295/033, A61K 31/495, A61P 23/00

(54) **PIPERAZINE SUBSTITUTED PHENOL DERIVATIVE AND USE THEREOF**

(30) Priority: 19.10.2022 CN 202211281594
(71) Applicant: CHENGDU MFS PHARMA. CO., LTD., Chengdu, Sichuan 611135 (CN)
(72) Inventor: ZHANG, Hancheng, Chengdu, Sichuan 611135 (CN); LIU, Jin, Chengdu, Sichuan 611135 (CN); ZHANG, Wensheng, Chengdu, Sichuan 611135 (CN); CHEN, Guangwu, Chengdu, Sichuan 611135 (CN); MA, Haijun, Chengdu, Sichuan 611135 (CN)
(74) Representative: Altmann Stößel Dick Patentanwälte PartG mbB
(86) International application number: PCT/CN2023/095079
(87) International publication number: WO 2024/082629

(57) **Abstract**

This invention discloses a piperazine substituted phenol derivative and its applications in pharmaceutical chemistry. The compound, depicted by formula I, exhibits excellent salt formation properties and water solubility, meeting formulation requirements. It also has a low minimum anesthetic effective dose, enabling rapid onset and recovery. This addresses the drawbacks of propofol and ciprofol prodrugs and lipid emulsions. The compound shows promise in developing drugs with sedative, hypnotic, and/or anesthetic effects, as well as in drugs for controlling status epilepticus.

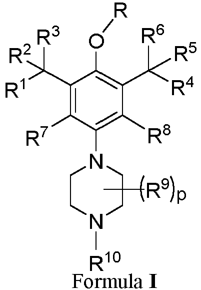

## Description

### Technical Field

The present invention relates to the field of medicinal chemistry, and in particular to a class of novel piperazine substituted phenol derivatives, their synthetic methods, as well as their applications in the preparation of drugs for inducing sedative, hypnotic, and/or anesthetic effects, as well as for controlling status epilepticus.

### Background Art

Propofol is a rapid, short-acting intravenous general anesthetic widely used clinically for the induction and maintenance of general anesthesia and sedation of critically ill patients in ICUs. It offers advantages such as rapid induction of anesthesia, quick recovery with complete functional restoration, and low incidence of postoperative nausea and vomiting.

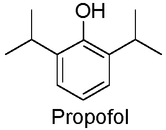

However, clinical practice has demostrated that propofol can cause sereious adverse reactions such as circulatory depression, respiratory depression, injection pain, agitation, and talkativeness etc. To enhance the potency of propofol, researchers have developed a structurally similar drug ciprofol based on propofol, which exhibits improved potency but still does not resolve the afore mentioned issues associated with propofol.

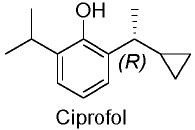

Since propofol and ciprofol are neither soluble in water nor can form salts, their clinical formulations are primarily fat emulsions. These emulsions are prepared by dissolving propofol or ciprofol in vegetable oils (primarily composed of fatty acid triglycerides), using phospholipids as emulsifiers, and adding isotonic agents and water for injection to create a stable oil-in-water (O/W) emulsion suitable for intravenous administration. However, recent studies have found that fat emulsions can easily cause various adverse reactions in clinical practice, including venous inflammatory reactions, acute renal failure, allergic reactions, anaphylactic shock, arrhythmias, and propofol infusion syndrome. To overcome the adverse effects of fat emulsions, researchers have developed water-soluble prodrugs. Fospropofol disodium is a water-soluble prodrug of propofol. After intravenous injection, it is metabolized by alkaline phosphatase on the surface of endothelial cells to produce the active drug propofol, which rapidly reaches equilibrium in brain tissue and exerts a dose-dependent hypnotic and sedative effect. However, compared to propofol, fospropofol disodium has a significantly slower onset time (up to 2.9 minutes), failing to meet the requirement for rapid onset.

To address the aforementioned issues, there is an urgent need to develop novel sedative, hypnotic, and general anesthetic agents that combine excellent water solubility with rapid onset, thereby overcoming or improving the existing problems associated with propofol.

### Summary

The objective of the present invention is to provide a novel series of piperazine substituted phenol derivatives of formula I, their synthetic methods, and their use in the preparation of drugs inducing sedative, hypnotic, and/or anesthetic effects, as well as for controlling status epilepticus.

The present invention provides a compound of formula I, or a stereoisomer thereof, a pharmaceutically acceptable salt thereof, a solvate thereof, a prodrug thereof, a metabolite thereof, or a deuterated derivative thereof:: 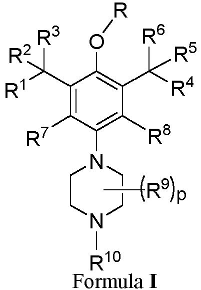
wherein, R is selected from hydrogen, C(O)R^{a}, COCH(NH₂)R^{a}, COOR^{h1}, CH₂COOR^{h1}, protecting group, PO(OR^{h1})(OR^{h2}) or CH₂OPO(OR^{h1}) (OR^{h2}); R^{a} is hydrogen, C₁₋₄ alkyl, C₂₋₄ alkenyl, C₂₋₄ alkynyl, 3-6 membered cycloalkyl, 3-6 membered heterocyclyl, aryl, or heteroaryl; R^{h1}, R^{h2} are each independently selected from hydrogen, C₁₋₄ alkyl, 3-6 membered cycloalkyl, 3-6 membered heterocyclyl, aryl, or heteroaryl;
R¹, R², R³, R⁴, R⁵, and R⁶ are each independently selected from hydrogen, halogen, C₁₋₄ alkyl, or 3-6 membered cycloalkyl, provided that R¹, R², R³, R⁴, R⁵, and R⁶ are not simultaneously methy;
R⁷, R⁸ are each independently selected from hydrogen, halogen or C₁₋₄ alkyl;
p is an integer from 0 to 8, and each R⁹ is independently selected from hydrogen, C₁₋₄ alkyl, halogenated C₁₋₄ alkyl; or p is an integer from 2 to 8, and two R⁹ groups are connected to form a ring, with the remaining R⁹ each independently selected from hydrogen, C₁₋₄ alkyl, halogenated C₁₋₄alkyl; or p is an integer from 1 to 9, and one R⁹ is connected to the nitrogen atom connected to R¹⁰ to form a quaternary ammonium salt, with the remaining R⁹ each independently selected from hydrogen, C₁₋₄ alkyl, halogenated C₁₋₄ alkyl;
R¹⁰ is selected from hydrogen, C₁₋₄ alkyl, (CR^{b1}R^{b2})ₘCOOR^{c}, (CR^{b1}R^{b2})ₘCONR^{d1}R^{d2}, (CR^{b1}k^{b2})ₙOR^{f}, (CR^{b1}R^{b2})ₙNR^{d1}R^{d2}, (CR^{b1}R^{b2})ₙOCOR^{e}, (CR^{b1}R^{b2})ₙNR^{d1}COR^{e}, (CR^{b1}R^{b2})ₘCO(CR^{b3}R^{b4})ₜCOOR^{c}, or (CR^{b1}R^{b2})ₘCO(CR^{b3}R^{b4})ₜCONR^{d1}R^{d2};
wherein, R^{b1}, R^{b2}, R^{b3}, and R^{b4} are each independently selected from hydrogen, halogen, C₁₋₄ alkyl, halogenated C₁₋₄ alkyl, C₂₋₄ alkenyl, C₂₋₄ alkynyl, 3-6 membered cycloalkyl, 3-6 membered heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₄ alkyl, halogenated C₁₋₄ alkyl, C₂₋₄ alkenyl, C₂₋₄ alkynyl, 3-6 membered cycloalkyl, 3-6 membered heterocyclyl, aryl, or heteroaryl;
R^{e} is selected from hydrogen, C₁₋₄ alkyl, C₂₋₄ alkenyl, C₂₋₄ alkynyl, 3-6 membered cycloalkyl, 3-6 membered heterocyclyl, aryl, or heteroaryl;
the alkyl, alkenyl, alkynyl, cycloalkyl, heterocyclyl, aryl, or heteroaryl in R^{b1}, R^{b2}, R^{b3}, R^{b4}, R^{c}, and R^{e} are unsubstituted or optionally substituted with one or more groups selected from halogen, C₁₋₄ alkyl, C₁₋₄ alkoxy, halogenated C₁₋₄ alkyl, C₂₋₄ alkenyl, C₂₋₄ alkynyl, 3-6 membered cycloalkyl, 3-6 membered heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁₋₄ alkyl;
R^{f} is selected from hydrogen, C₁₋₄ alkyl, or halogenated C₁₋₄ alkyl;
Rⁱ is selected from hydrogen, C₁₋₄ alkyl, C₂₋₄ alkenyl, C₂₋₄ alkynyl, 3-6 membered cycloalkyl, or 3-6 membered heterocyclyl;
m is 0, 1, 2, 3, or 4;
n is 2, 3, or 4;
t is 1, 2, 3, or 4.

Further, said compound has a structure of formula II: 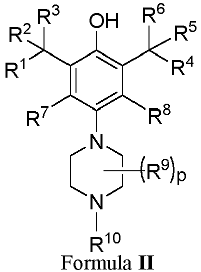
wherein, R¹, R², R³, R⁴, R⁵, and R⁶ are each independently selected from hydrogen, halogen, C₁₋₄ alkyl, or 3-6 membered saturated cycloalkyl;
p is an integer from 0 to 8, and each R⁹ is independently selected from hydrogen, C₁₋₄ alkyl, halogenated C₁₋₄ alkyl; or p is an integer from 2 to 8, and two R⁹ groups are connected to form a ring, with the remaining R⁹ each independently selected from hydrogen, C₁₋₄ alkyl,; or p is an integer from 1 to 9, and one R⁹ is connected to the nitrogen atom attached to R¹⁰ to form a quaternary ammonium salt, with the remaining R⁹ each independently selected from hydrogen, C₁₋₄ alkyl, halogenated C₁₋₄ alkyl;
R¹⁰ is selected from hydrogen, C₁₋₄ alkyl, (CR^{b1}R^{b2})mCOOR^{c}, (CR^{b1}R^{b2})mCONR^{d1}R^{d2}, (CR^{b1}R^{b2})nOR^{f}, (CR^{b1}R^{b2})ₙNR^{d1}R^{d2}, (CR^{b1}R^{b2})nOCOR^{e}, (CR^{b1}R^{b2})nNR^{d1}COR^{e}, (CR^{b1}R^{b2})mCO(CR^{b3}R^{b4})tCOOR^{c}, or (CR^{b1}R^{b2})mCO(CR^{b3}R^{b4})tCONR^{d1}R^{d2};
wherein, R^{b1}, R^{b2}, R^{b3}, and R^{b4} are each independently selected from hydrogen, halogen, C₁₋₄ alkyl, halogenated C₁₋₄ alkyl, C₂₋₄ alkenyl, C₂₋₄ alkynyl, 3-6 membered cycloalkyl, 3-6 membered heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₄ alkyl, halogenated C₁₋₄ alkyl, C₂₋₄ alkenyl, C₂₋₄ alkynyl, 3-6 membered cycloalkyl, 3-6 membered heterocyclyl, aryl, or heteroaryl;
R^{e} is selected from hydrogen, C₁₋₄ alkyl, C₂₋₄ alkenyl, C₂₋₄ alkynyl, 3-6 membered cycloalkyl, 3-6 membered heterocyclyl, aryl, or heteroaryl;
the alkyl, alkenyl, alkynyl, cycloalkyl, heterocyclyl, aryl, or heteroaryl in R^{b1}, R^{b2}, R^{b3}, R^{b4}, R^{c}, and R^{e} are unsubstituted or optionally substituted with one or more groups selected from halogen, C₁-₄ alkyl, C₁-₄ alkoxy, halogenated C₁₋₄ alkyl, C₂₋₄ alkenyl, C₂₋₄ alkynyl, 3-6 membered cycloalkyl, 3-6 membered heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₄ alkyl;
R^{f} is selected from hydrogen, C₁-₄ alkyl, or halogenated C₁-₄ alkyl;
Rⁱ is selected from hydrogen, C₁-₄ alkyl, C₂-₄ alkenyl, C₂-₄ alkynyl, 3-6 membered cycloalkyl, or 3-6 membered heterocyclyl;
m is 0, 1, 2, 3, or 4;
n is 2, 3, or 4;
t is 1, 2, 3, or 4.

Further, said compound has a structure of formula III: 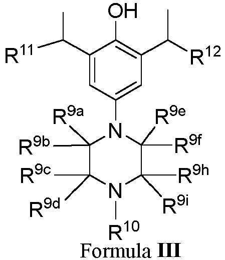
wherein, R^{d1}, R^{d2} are each independently selected from methyl, 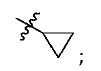
R^{9a}, R^{9b}, R^{9c}, R^{9d}, R^{9e}, R^{9f}, R^{9h}, R⁹ⁱ are each independently selected from hydrogen, C₁-₄ alkyl; or, two groups among R^{9a}, R^{9b}, R^{9c}, R^{9d}, R^{9e}, R^{9f}, R^{9h}, and R⁹ⁱ attached to the same carbon atom may join together to form a spiro ring, with the remaining groups each independently selected from hydrogen or C₁-₄ alkyl; or, two groups among R^{9a}, R^{9b}, R^{9c}, R^{9d}, R^{9e}, R^{9f}, R^{9h}, and R⁹ⁱ attached to different carbon atoms may join together to form a bridged ring or a fused ring, with the remaining groups each independently selected from hydrogen or C₁₋₄ alkyl;
R¹⁰ is as described in formula II.

Further, said compound has a structure of formula IV-1 or formula IV-2: 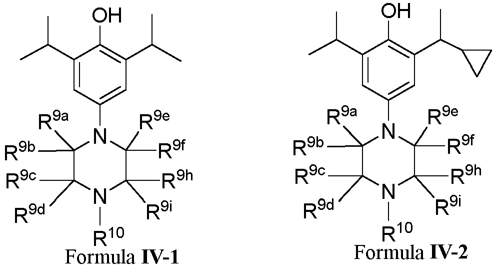
wherein, R^{9a}, R^{9b}, R^{9c}, R^{9d}, R^{9e}, R^{9f}, R^{9h}, and R⁹ⁱ are each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to the same carbon atom may join together to form a spiro ring, with the remaining groups each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to different carbon atoms may join together to form a bridged ring or a fused ring, with the remaining groups each independently selected from hydrogen or C₁-₃ alkyl; said spiro ring, bridged ring, or fused ring contains 2, 3, or 4 heteroatoms selected from N, O, or S;
R¹⁰ is selected from hydrogen, C₁₋₄ alkyl, (CR^{b1}R^{b2})mCOOR^{c}, (CR^{b1}R^{b2})mCONR^{d1}R^{d2}, (CR^{b1}R^{b2})nOR^{f}, (CR^{b1}R^{b2})ₙNR^{d1}R^{d2}, (CR^{b1}R^{b2})nOCOR^{e}, (CR^{b1}R^{b2})nNR^{d1}COR^{e}, (CR^{b1}R^{b2})mCO(CR^{b3}R^{b4})tCOOR^{c}, or (CR^{b1}R^{b2})mCO(CR^{b3}R^{b4})tCONR^{d1}R^{d2};
wherein, R^{b1}, R^{b2}, R^{b3}, and R^{b4} are each independently selected from hydrogen, halogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₄ alkyl, halogenated C₁₋₄ alkyl, C₂₋₄ alkenyl, C₂₋₄ alkynyl, 3-6 membered cycloalkyl, 3-6 membered heterocyclyl, aryl, or heteroaryl;
R^{e} is selected from hydrogen, C₁-₃ alkyl, C₂₋₄ alkenyl, C₂₋₃ alkynyl, 3-6 membered cycloalkyl, 3-6 membered heterocyclyl, aryl, or heteroaryl;
the alkyl, alkenyl, alkynyl, saturated cycloalkyl, saturated heterocyclyls, cycloalkyl, heterocyclyl, aryl, or heteroaryl in R^{b1}, R^{b2}, R^{b3}, R^{b4}, R^{c}, and R^{e} are unsubstituted or optionally substituted with one or more groups selected from halogen, C₁-₃ alkyl, C₁-₃ alkoxy, halogenated C₁-₃ alkyl, C₂₋₃ alkenyl, C₂₋₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₃ alkyl;
R^{f} is selected from hydrogen, C₁-₃ alkyl, or halogenated C₁-₃ alkyl;
Rⁱ is selected from hydrogen, C₁-₃ alkyl, C₂-₃ alkenyl, C₂-₃ alkynyl, 3-6 membered saturated cycloalkyl, or 3-6 membered saturated heterocyclyl;
m is 0, 1, 2, 3, or 4;
n is 2, 3, or 4;
t is 1, 2, 3, or 4.

Further, said compound has a structure of formula IV-1 or formula IV-2: 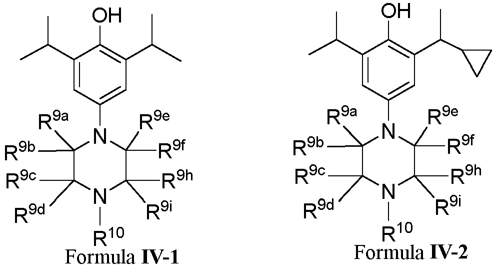
wherein, R^{9a}, R^{9b}, R^{9c}, R^{9d}, R^{9e}, R^{9f}, R^{9h}, and R⁹ⁱ are each independently selected from hydrogen or C₁-₃ alkyl; or two groups among R^{9a}, R^{9b}, R^{9c}, R^{9d}, R^{9e}, R^{9f}, R^{9h}, and R⁹ⁱ attached to the same carbon atom may join together to form a spiro ring, with the remaining groups each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to different carbon atoms may join together to form a bridged ring or a fused ring, with the remaining groups each independently selected from hydrogen or C₁-₃ alkyl; said spiro ring, bridged ring, or fused ring contains 2, 3, or 4 heteroatoms selected from N, O, or S;
R¹⁰ is selected from hydrogen, C₁₋₄ alkyl, (CR^{b1}R^{b2})mCOOR^{c}, (CR^{b1}R^{b2})mCONR^{d1}R^{d2}, (CR^{b1}R^{b2})nOR^{f}, (CR^{b1}R^{b2})ₙNR^{d1}R^{d2}, (CR^{b1}R^{b2})nOCOR^{e}, (CR^{b1}R^{b2})nNR^{d1}COR^{e}, (CR^{b1}R^{b2})mCO(CR^{b3}R^{b4})tCOOR^{c}, or (CR^{b1}R^{b2})mCO(CR^{b3}R^{b4})tCONR^{d1}R^{d2};
wherein, R^{b1}, R^{b2}, R^{b3}, and R^{b4} are each independently selected from hydrogen, halogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, or heteroaryl;
R^{e} is selected from hydrogen, C₁₋₃ alkyl, C₂₋₃ alkenyl, C₂₋₃ alkynyl, 3-6 membered saturated cycloalkyl, 3-6 membered saturated heterocyclyl, aryl, or heteroaryl;
the alkyl, alkenyl, alkynyl, saturated cycloalkyl, saturated heterocyclyl, aryl, or heteroaryl in R^{b1}, R^{b2}, R^{b3}, R^{b4}, R^{c}, and R^{e} are unsubstituted or optionally substituted with one or more groups selected from halogen, C₁-₃ alkyl, C₁-₃ alkoxy, halogenated C₁-₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₃ alkyl;
R^{f} is selected from hydrogen, C₁-₃ alkyl, or halogenated C₁-₃ alkyl;
Rⁱ is selected from hydrogen, C₁-₃ alkyl, C₂-₃ alkenyl, C₂-₃ alkynyl, 3-6 membered saturated cycloalky, or 3-6 membered saturated heterocyclyl;
m is 0, 1, 2, 3, or 4;
n is 2, 3, or 4;
t is 1, 2, 3, or 4.

Further, said 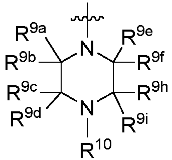 structural fragment is one of the following structures:

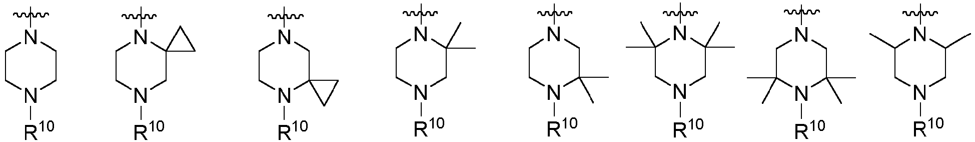

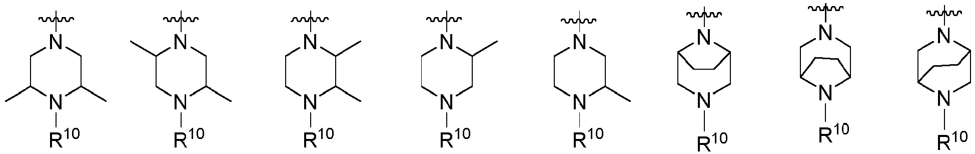

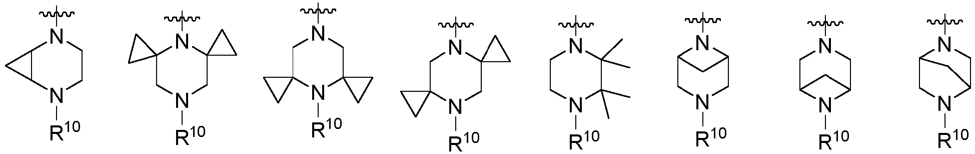

Further, said compound has a structure of formula V- 1 or formula V-2: 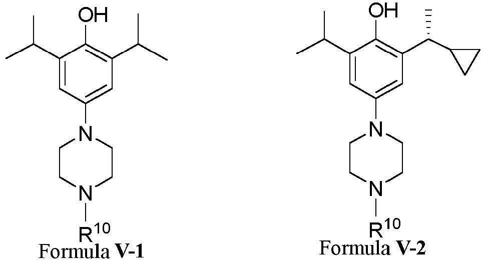
wherein, R¹⁰ is selected from hydrogen, C₁₋₄ alkyl, (CR^{b1}R^{b2})mCOOR^{c}, (CR^{b1}R^{b2})mCONR^{d1}R^{d2}, (CR^{b1}R^{b2})nOR^{f}, (CR^{b1}R^{b2})nNR^{d1}R^{d2}, (CR^{b1}R^{b2})nOCOR^{e}, (CR^{b1}R^{b2})nNR^{d1}COR^{e}, (CR^{b1}R^{b2})mCO(CR^{b3}R^{b4})tCOOR^{c}, or (CR^{b1}R^{b2})mCO(CR^{b3}R^{b4})tCONR^{d1}R^{d2};
wherein, R^{b1}, R^{b2}, R^{b3}, and R^{b4} are each independently selected from hydrogen, halogen, C₁₋₂ alkyl, halogenated C₁₋₂ alkyl, C₂₋₃ alkenyl, C₂₋₃ alkynyl, 3-6 membered saturated cycloalkyl, 3-6 membered saturated heterocyclyl, phenyl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₂ alkyl, halogenated C₁₋₂ alkyl, C₂₋₃ alkenyl, C₂₋₃ alkynyl, 3-6 membered saturated cycloalkyl, 3-6 membered saturated heterocyclyl, phenyl, or heteroaryl;
R^{e} is selected from hydrogen, C₁₋₂ alkyl, C₂₋₃ alkenyl, C₂₋₃ alkynyl, 3-6 membered saturated cycloalkyl, 3-6 membered saturated heterocyclyl, phenyl, or heteroaryl;
the alkyl, alkenyl, alkynyl, saturated cycloalkyl, saturated heterocyclyl, phenyl, or heteroaryl in R^{b1}, R^{b2}, R^{b3}, R^{b4}, R^{c}, and R^{e} are unsubstituted or optionally substituted with one or more groups selected from halogen, C₁-₂ alkyl, C₁-₂ alkoxy, halogenated C₁-₂ alkyl, vinyl, ethynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₂ alkyl;
R^{f} is selected from hydrogen, C₁-₂ alkyl, or halogenated C₁-₂ alkyl;
Rⁱ is selected from hydrogen, C₁-₂ alkyl, vinyl, ethynyl, 3-6 membered saturated cycloalkyl, or 3-6 membered saturated heterocyclyl;
m is 0, 1, 2, 3, or 4;
n is 2, 3, or 4;
t is 1, 2, 3, or 4.

Further, said 3-6 membered saturated cycloalkyl is selected from 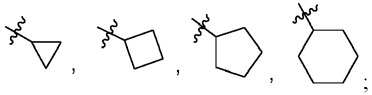 said 3-6 membered saturated heterocyclyl is selected from 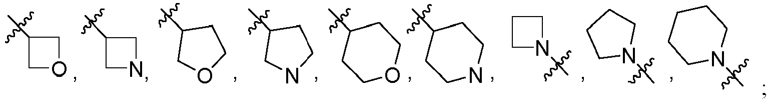 said 3-6- membered cyclic structure is selected from cyclopropyl, cyclobutyl, cyclopentyl, cyclohexyl; said halogen is selected from fluorine, chlorine, bromine.

Further, said compound has a structure of formula VI-1 or formula VI-2: 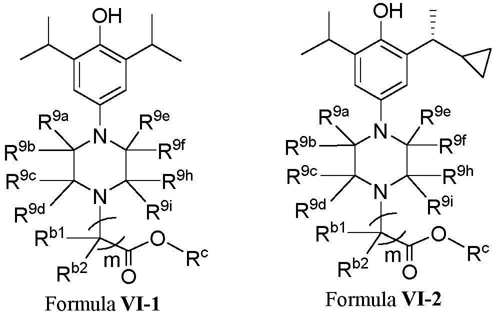
wherein, R^{9a}, R^{9b}, R^{9c}, R^{9d}, R^{9e}, R^{9f}, R^{9h}, and R⁹ⁱ are each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to the same carbon atom may join together to form a spiro ring, with the remaining groups each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to different carbon atoms may join together to form a bridged ring or a fused ring, with the remaining groups each independently selected from hydrogen or C₁-₃ alkyl; said spiro ring, bridged ring, or fused ring contains 2, 3, or 4 heteroatoms selected from N, O, or S;
m is 0, 1, 2, 3, or 4;
R^{b1}, R^{b2} are each independently selected from hydrogen, halogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, or heteroaryl;
the alkyl, alkenyl, alkynyl, saturated cycloalkyl, saturated heterocyclyl, aryl, or heteroaryl in R^{b1}, R^{b2}, and R^{c} are unsubstituted or optionally substituted with one or more groups selected from halogen, C₁-₃ alkyl, C₁-₃ alkoxy, halogenated C₁-₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₃ alkyl;
R^{f} is selected from hydrogen, C₁-₃ alkyl, or halogenated C₁-₃ alkyl;
Rⁱ is selected from hydrogen, C₁-₃ alkyl, C₂-₃ alkenyl, C₂-₃ alkynyl, 3-6 membered saturated cycloalky, or 3-6 membered saturated heterocyclyl.

Further, said compound has a structure of formula VI-1 or formula VI-2: 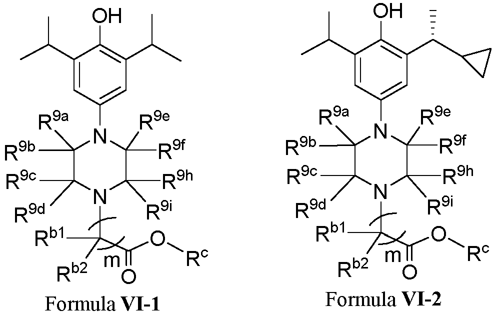
wherein, R^{9a}, R^{9b}, R^{9c}, R^{9d}, R^{9e}, R^{9f}, R^{9h}, and R⁹ⁱ are each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to the same carbon atom may join together to form a spiro ring, with the remaining groups each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to different carbon atoms may join together to form a bridged ring or a fused ring, with the remaining groups each independently selected from hydrogen or C₁-₃ alkyl; said spiro ring, bridged ring, or fused ring contains 2, 3, or 4 heteroatoms selected from N, O, or S;
m is 0, 1, 2, 3, or 4;
R^{b1}, R^{b2} are each independently selected from hydrogen, halogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, heteroaryl, CN, OR^{f}, SR^{f}, or NR^{d1}R^{d2}; or, R^{b1} and R^{b2}, together with the carbon atom to which they are attached, form a 3-6- membered cyclic structure containing 0 or 1 heteroatom selected from N, O, or S;
R^{c} is selected from hydrogen, C₁₋₄ alkyl, halogenated C₁₋₄ alkyl, C₂₋₃ alkenyl, C₂₋₃ alkynyl, 3-6 membered saturated cycloalkyl, 3-6 membered saturated heterocyclyl, aryl, or heteroaryl;
the alkyl, alkenyl, alkynyl, saturated cycloalkyl, saturated heterocyclyl, aryl, or heteroaryl in R^{b1}, R^{b2}, and R^{c} are unsubstituted or optionally substituted with one or more groups selected from halogen, C₁-₃ alkyl, C₁-₃ alkoxy, halogenated C₁-₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₃ alkyl;
R^{f} is selected from hydrogen, C₁-₃ alkyl, or halogenated C₁-₃ alkyl;
Rⁱ is selected from hydrogen, C₁-₃ alkyl, C₂-₃ alkenyl, C₂-₃ alkynyl, 3-6 membered saturated cycloalky, or 3-6 membered saturated heterocyclyl.

Further, said 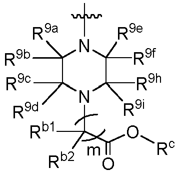 structural fragment is one of the following structures: 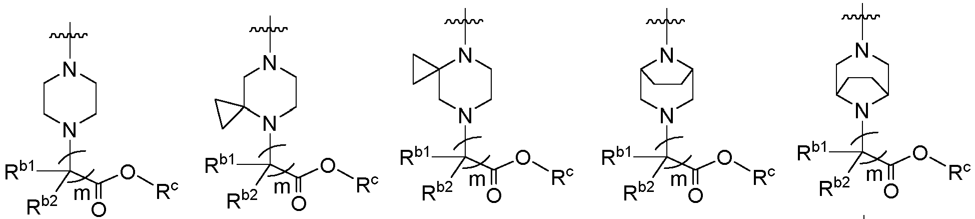 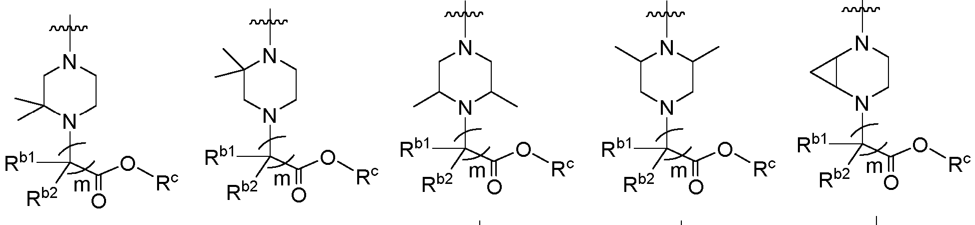 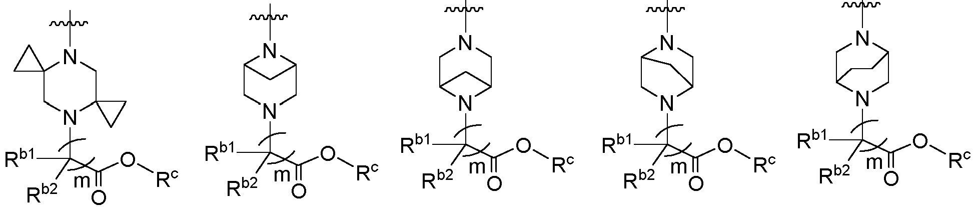
m is 1, 2 or3;
R^{b1}, R^{b2} are each independently selected from hydrogen, halogen, C₁₋₃ alkyl, halogenated C₁₋₃ alkyl, 3-6 membered saturated cycloalkyl, 4-6 membered saturated heterocyclyl; or, R^{b1} and R^{b2}, together with the carbon atom to which they are attached, form a 3-4 membered cyclic structure containing 0 or 1 heteroatomselected from N, O, or S;
R^{c} is selected from hydrogen, C₁₋₄ alkyl, halogenated C₁₋₄ alkyl, 3-6 membered saturated cycloalkyl, 3-6 membered saturated heterocyclyl, aryl, or heteroaryl;
the alkyl, saturated cycloalkyl, saturated heterocyclyl, aryl, or heteroaryl in R^{b1}, R^{b2}, and R^{c} are unsubstituted or optionally substituted with one or more groups selected from halogen, C₁-₃ alkyl, C₁-₃ alkoxy, halogenated C₁-₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2}, R^{f}, and Rⁱ are as described in formula VI-1 or formula VI-2.

Further, said compound has a structure of formula VII-1 or formula VII-2: 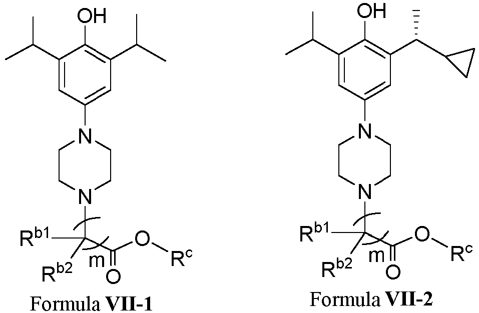 wherein, m is 1, 2;
R^{b1}, R^{b2} are each independently selected from hydrogen, halogen, C₁₋₃ alkyl, halogenated C₁₋₃ alkyl, 3-6 membered saturated cycloalkyl, 4-6 membered saturated heterocyclyl;
R^{c} is selected from hydrogen, C₁₋₄ alkyl, halogenated C₁₋₄ alkyl, 3-6 membered saturated cycloalkyl, 3-6 membered saturated heterocyclyl, aryl, or heteroaryl;
the alkyl, saturated cycloalkyl, saturated heterocyclyl, aryl, or heteroaryl in R^{b1}, R^{b2}, R^{c} are unsubstituted or optionally substituted with one or more groups selected from halogen, C₁-₃ alkyl, C₁-₃ alkoxy, halogenated C₁-₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2}, R^{f}, and Rⁱ are as described in formula VI-1 or formula VI-2.

Further, in formula VI-1, formula VI-2, formula VII-1, or formula VII-2:
m is 1 or 2;
R^{b1}, R^{b2} are each independently selected from hydrogen, methyl, ethyl, n-Propyl, isopropyl, cyclopropyl;
R^{c} is selected from methyl, ethyl, n-Propyl, isopropyl, tert-butyl, CF₃, cyclopropyl,

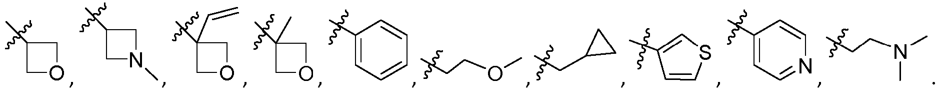

Further, the structure of said compound is selected from: 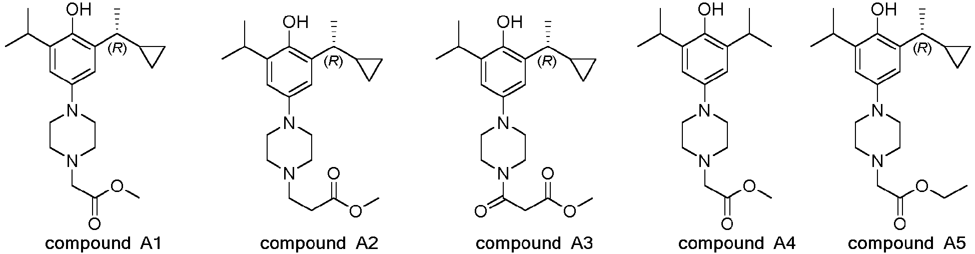 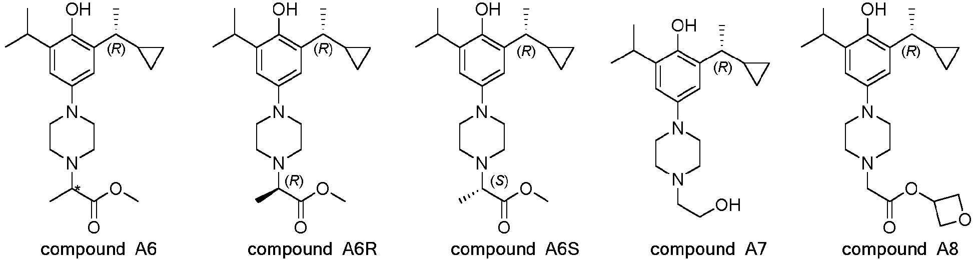 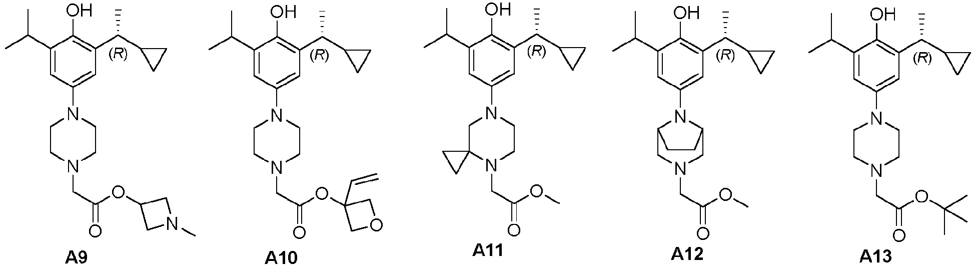 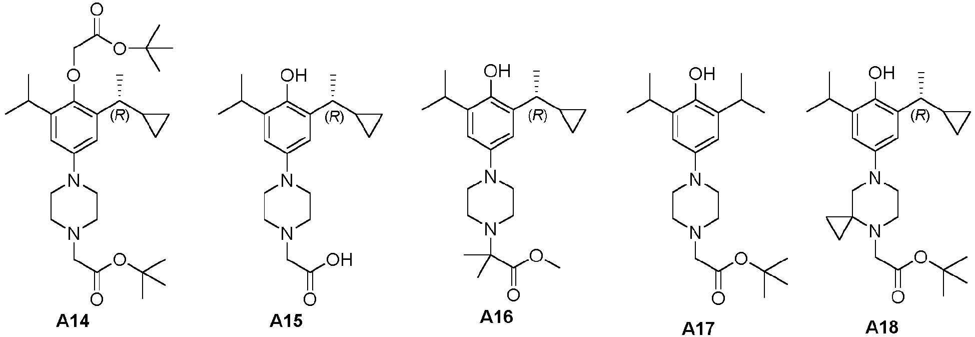 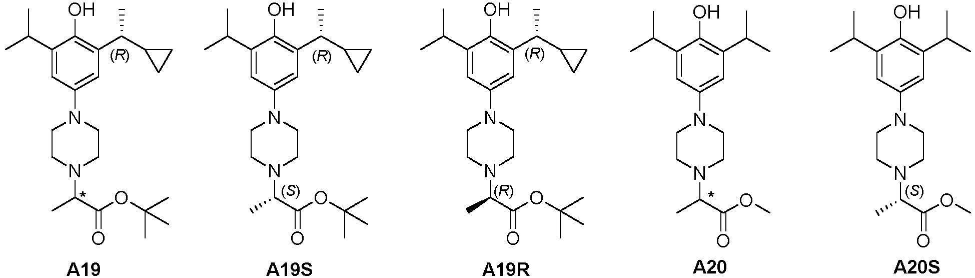 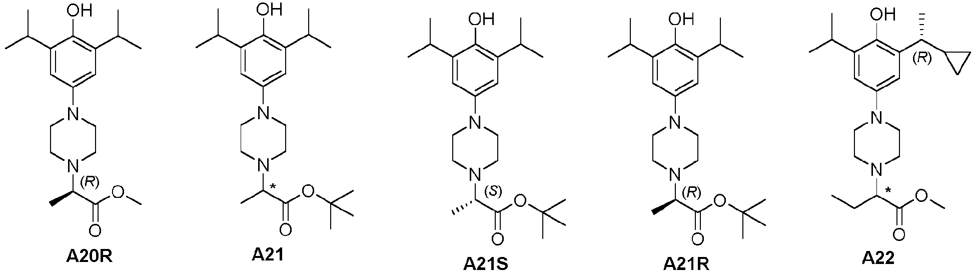 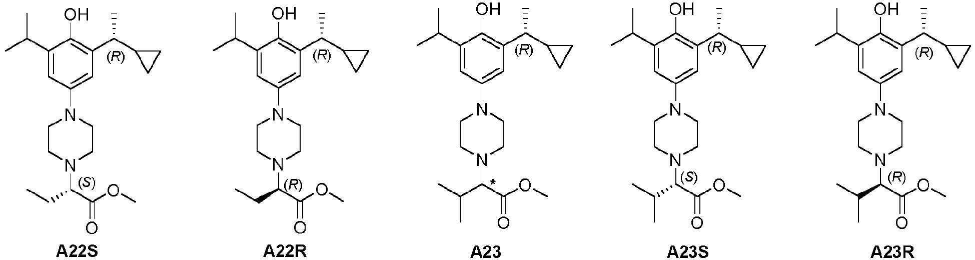 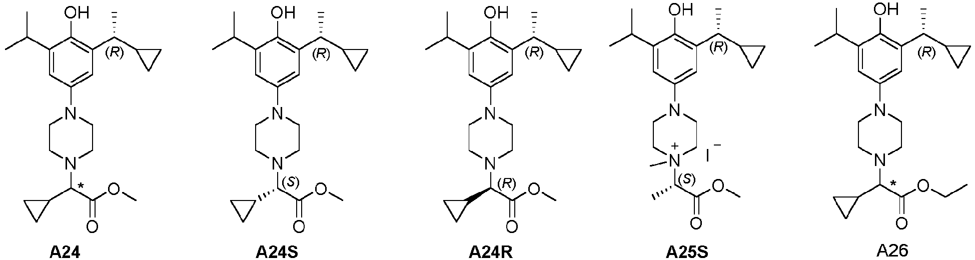 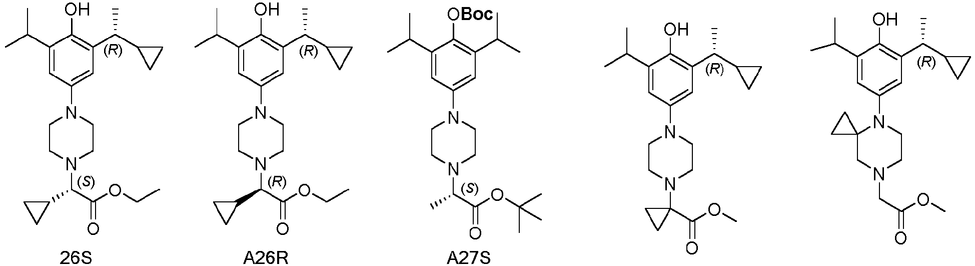 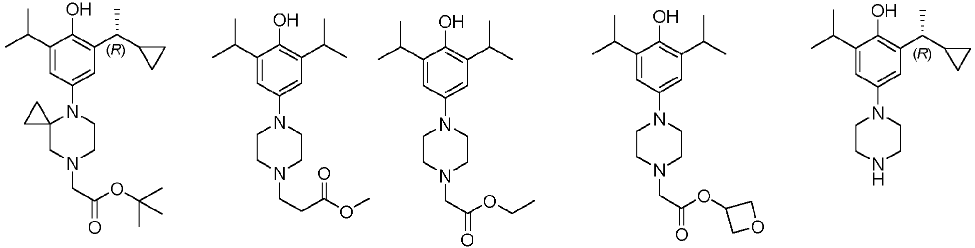 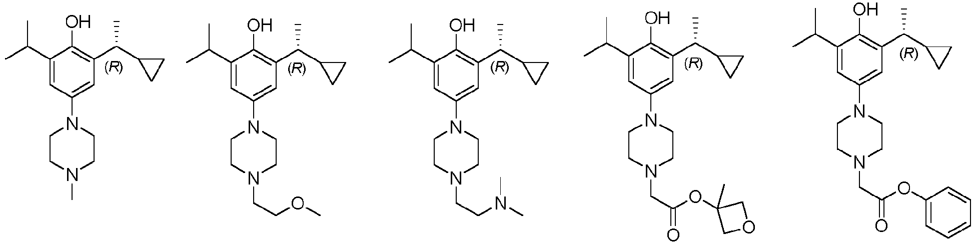 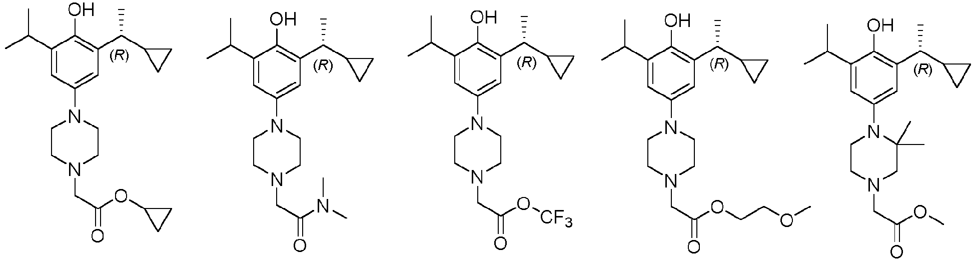 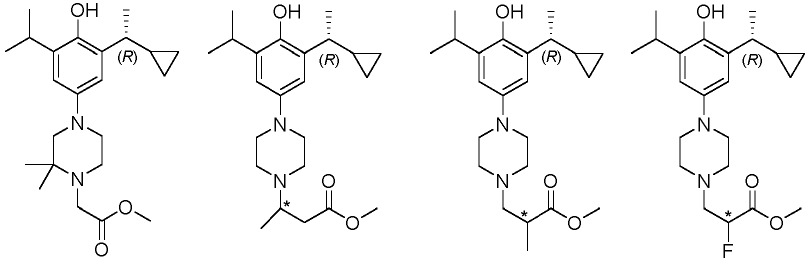 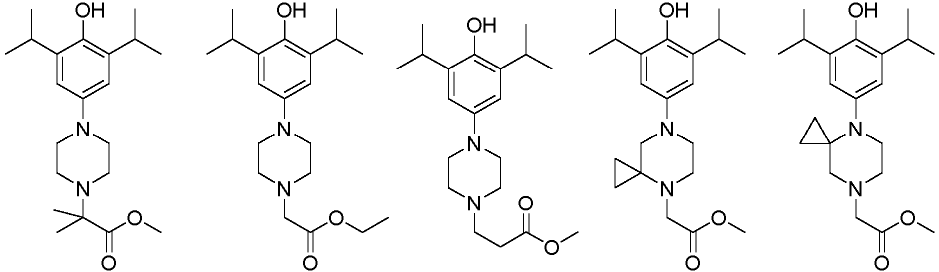 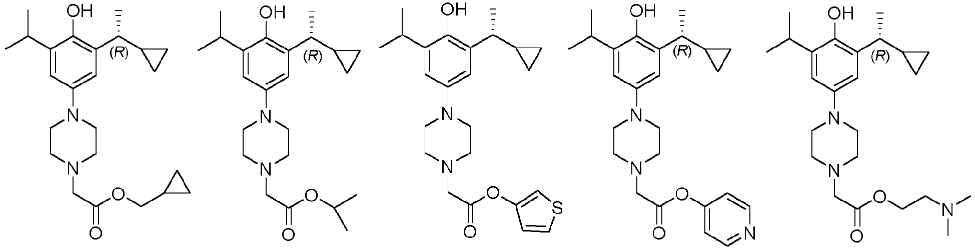 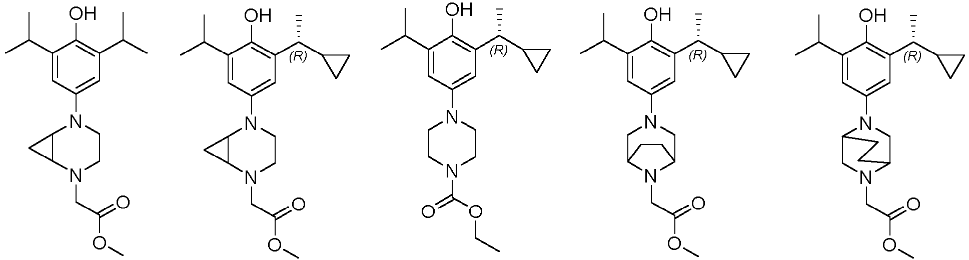 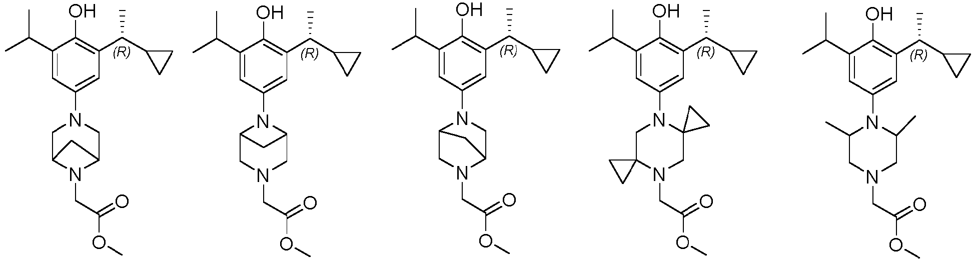 "*" means a chiral center.

Further, the pharmaceutically acceptable salt is selected from acetate, adipate, aspartate, benzoate, benzene sulfonate, bicarbonate, carbonate, bisulfate, sulfate, borate, camphorsulfonate, citrate, cyclamate, edisylate, ethanesulfonate, formate, fumarate, glucoheptonate, gluconate, glucuronate, hexafluorophosphate, hydrochloride, hydrobromide, hydroiodide, hydroxyethylsulfonate, lactate, malate, maleate, malonate, mesylate, methylsulfate, naphthylate, napsylate, nicotinate, nitrate, orotate, oxalate, palmitate, pamoate, phosphate, hydrogen phosphate, dihydrogen phosphate, pyroglutamate, saccharate, stearate, succinate, tannate, tartrate, tosylate, trifluoroacetate, xinafoate, methanesulfonate or p-toluenesulfonate.

Further, the present invention also provides a drug, it is prepared by using the compound according to any one of the above, or a stereoisomer thereof, or a pharmaceutically acceptable salt thereof, or a solvate thereof, or a prodrug thereof, or a metabolite thereof, or a deuterated derivative thereof as active ingredients, with addition of pharmaceutically acceptable excipients.

The present invention also provides the use of compounds according to anyone of the above, or a stereoisomer thereof, or a pharmaceutically acceptable salt thereof, or a solvate thereof, or a prodrug thereof, or a metabolite thereof, or a deuterated derivative thereof, in preparation of a drug for inducing sedation, hypnosis, and/or anesthesia, and/or for controlling status epilepticus.

The "drug with sedative effects" according to the present invention denotes a drug that effectively aids sleep and improves sleep quality, thereby mitigating the severe harm caused by insomnia, treating insomnia, and enhancing sleep outcomes.

The "drug with hypnotic effects" according to the present invention denotes a drug that induces drowsiness and promotes sleep. It suppresses the central nervous system, causing sedation at low doses and general anesthesia at excessive doses.

The "drug with anesthetic effects" according to the present invention denotes to a drug that induces reversible functional inhibition of the central and/or peripheral nervous systems, characterized primarily by the loss of sensation, particularly pain perception.

Preferably, the anesthesia is general anesthesia.

The "general anesthesia" mentioned in the present invention denotes the temporary inhibition of the central nervous system caused by anesthetics entering the body. Clinically, it is characterized by loss of consciousness, absence of pain perception throughout the body, amnesia, reflex inhibition, and skeletal muscle relaxation.

The "status epilepticus." mentioned in the present invention denotes recurrent epileptic seizures without full recovery of consciousness between episodes, or a single seizure lasting over 30 minutes. Prolonged seizures can cause irreversible brain damage due to hyperthermia, circulatory failure, or neuronal excitotoxicity, leading to high morbidity and mortality. Thus, status epilepticus is a common medical emergency in internal medicine.

Unless otherwise specified, definitions of groups or terms provided herein apply throughout the entire specification. Terms not explicitly defined shall be interpreted according to their generally accepted meanings in the field, consistent with the disclosure and context.

The minimum and maximum number of carbon atoms in a hydrocarbon group are indicated by prefixes. For example, the prefix C_{a-b} alkyl represents an alkyl group containing "a" to "b" carbon atoms. For example, C₁₋₆ alkyl refers to a straight or branched chain alkyl group containing 1 to 6 carbon atoms.

**In** the compound represented by formula I of the present invention, the ring described in "p is an integer from 2 to 8, and two R⁹ groups are connected to form a ring" includes rings such as spiro rings, bridged rings, and fused rings.

**In** this context, "substituted" means that one, two, or more hydrogen atoms in a molecule are replaced by other different atoms or groups, including one, two, or more substitutions on the same or different atoms within the molecule.

"Alkylenes" refers to the group remaining after an alkyl group loses one hydrogen atom. For example, C₁₋₄ alkylene refers to a straight or branched chain alkylene group containing 1 to 4 carbon atoms; C₂ alkylene refers to

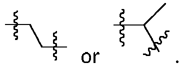

" 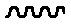 " refers to the connection sites between structural fragments.

"Cycloalkyl" refers to a saturated or unsaturated cyclic hydrocarbon substituent; cyclic hydrocarbon can have one or more rings. For example, "3-6 membered saturated carbocycle" refers to saturated cyclic groups in which the number of carbon atoms in the ring is 3 to 6.

"Heterocyclyl" refers to a saturated or unsaturated cyclic hydrocarbon substituent; cyclic hydrocarbon can have one or more rings, and at least one atom selected from O, S or substituted N, while the remaining ring atoms are carbons. For example: 3-6 membered saturated heterocyclyl refers to saturated heterocyclyl in which the number of carbon atoms in the ring is 3 to 6.

"Aryl" refers to a fully carbon monocyclic or fused polycyclic (i.e. ring sharing adjacent carbon atom pairs) group with a conjugated π electron system, such as phenyl and naphthyl. Said aryl ring can be fused to other cyclic groups (including saturated and unsaturated rings), but can not contain heteroatoms such as nitrogen, oxygen, or sulfur. At the same time, the point connecting with the parent structure must be on the carbon in the ring having the conjugated π electron system. Aryl can be substituted or unsubstituted.

"Heteroaryl" refers to a heteroaromatic group containing one or more heteroatoms. The heteroatoms mentioned herein include oxygen, sulfur, and nitrogen. For example, furanyl, thienyl, pyridinyl, pyrazolyl, pyrrolyl, n-alkylpyrrolyl, pyrimidinyl, pyrazinyl, imidazolyl, tetrazolyl, etc. The heteroaromatic ring can be fused to aryl, heterocyclyl group or cycloalkyl ring, in which the ring connected with the parent structure is heteroaromatic ring. Heteroaryl can be substituted or unsubstituted.

"Bridged ring" refers to a polycyclic cycloalkyl group in which two rings share two non-adjacent carbon atoms.

"Spiro ring" refers to a polycyclic cycloalkyl group in which two rings share one carbon atom.

"Fused ring" refers to a polycyclic cycloalkyl group in which two rings share two adjacent carbon atoms.

In the formula III**,** formula IV-1, formula IV-2, formulaVI-1 or VI-2 of the present invention, "two groups among R^{9a}, R^{9b}, R^{9c}, R^{9d}, R^{9e}, R^{9f}, R^{9h}, and R⁹ⁱ attached to the same carbon atom may join together to form a spiro ring", it means that when two groupsamong R^{9a}, R^{9b}, R^{9c}, R^{9d}, R^{9e}, R^{9f}, R^{9h}, R⁹ⁱ attached to the same carbon atom are connected together with 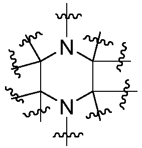 as a whole, a spiro ring is formed; "two groups among R^{9a}, R^{9b}, R^{9c}, R^{9d}, R^{9e}, R^{9f}, R^{9h}, and R⁹ⁱ attached to different carbon atoms may join together to form a bridged ring or a fused ring", it means that when two groups among R^{9a}, R^{9b}, R^{9c}, R^{9d}, R^{9e}, R^{9f}, R^{9h}, and R⁹ⁱ attached to different carbon atoms are connected together with 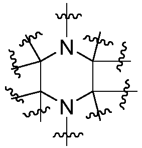 as a whole, a bridge ring or fused ring is formed.

"Halogen" is fluorine, chlorine, bromine, or iodine.

"Deuterated derivative" refers to a compound in which one or more hydrogen atoms are replaced by deuterium.

"Pharmaceutically acceptable" means that a carrier, vehicle, diluent, excipient, and/or the formed salt is generally chemically or physically compatible with other components constituting a pharmaceutical dosage form and physiologically compatible with the recipient.

"Salt" refers to an acid and/or base salt formed by the compound or its stereoisomer with inorganic and/or organic acids and/or bases, including zwitterions (internal salts) and quaternary ammonium salts, such as alkylammonium salts. These salts can be obtained directly during the final isolation and purification of the compound, or by appropriately mixing the compound or its stereoisomer with a certain amount of acid or base (e.g., in equivalent amounts). These salts may form precipitates in solution and be collected by filtration, recovered after solvent evaporation, or lyophilized from an aqueous medium.

The pharmaceutically acceptable salts mentioned in the present invention may be selected from acetate, adipate, aspartate, benzoate, benzene sulfonate, bicarbonate, carbonate, bisulfate, sulfate, borate, camphorsulfonate, citrate, cyclamate, edisylate, ethane sulfonate, formate, fumarate, gluceptate, gluconate, glucuronate, hexafluorophosphate, hydrochloride, hydrobromide, hydroiodide, hydroxyethylsulfonate, lactate, malate, maleate, malonate, mesylate, methylsulfate, naphthylate, napsylate, nicotinate, nitrate, orotate, oxalate, palmitate, pamoate, phosphate, hydrogen phosphate, dihydrogen phosphate, pyroglutamate, saccharate, stearate, succinate, tannate, tartrate, tosylate, trifluoroacetate, xinafoate, methanesulfonate, p-toluenesulfonate, or p-toluenesulfonate.

The compound of the present invention or composition thereof, as well as the use method thereof:
The compounds of the present invention, and various crystal forms, pharmaceutically acceptable inorganic or organic salts, hydrates or solvates thereof, as well as the pharmaceutical composition containing the compound of the present invention as the main active ingredient can be used for sedation, hypnosis and/or general anesthesia. The compound of the present invention can also be used for controlling epileptic persistent state and the like.

The pharmaceutical composition of the present invention includes a compound of the present invention or a pharmaceutically acceptable salt thereof within a safe and effective amount, as well as a pharmaceutically acceptable excipient or carrier thereof.

The administration ways for the compound or pharmaceutical composition of the present invention include (but not limited to) intragastric, intraintestinal, extragastrointestinal (intravenous, intramuscular or subcutaneous), oral and various local administration.

The composition for extragastrointestinal injection (intravenous, intramuscular, subcutaneous) may contain physiologically acceptable sterile aqueous or anhydrous solutions, dispersions, suspensions or emulsions, and sterile powder used for reconstitution into sterile injectable solutions or dispersions. Suitable aqueous and non-aqueous carriers, diluents, solvents or excipients include water, ethanol, polyols and their suitable mixtures.

Solid dosage forms for oral administration include capsules, tablets, pills, powders and granules. **In** these solid dosage forms, the active compound is mixed with at least one conventional inert excipient (or carrier), such as sodium citrate or dicalcium phosphate, or mixed with following ingredients: (a) bulking agent or compatibilizer, such as starch, lactose, sucrose, glucose, mannitol and silicic acid; (b) binding agent, such as hydroxymethylcellulose, alginate, gelatin, polyvinylpyrrolidone, sucrose and gum arabic; (c) moisturing agent, such as glycerin; (d) disintegrating agent, such as agar, calcium carbonate, potato starch or cassava starch, alginate, some complex silicates, and sodium carbonate; (e) solvents, such as paraffin; (f) absorption accelerators, such as quaternary amine compounds; (g) wetting agents, such as cetyl alcohol and glycerin monostearate; (h) adsorbents, such as kaolin; and (i) lubricants, such as talc, calcium stearate, magnesium stearate, solid polyethylene glycol, sodium dodecyl sulfate, or mixtures thereof. **In** capsules, tablets and pills, the dosage form may also include buffers.

The liquid dosage forms used for oral administration include pharmaceutically acceptable emulsion, solution, suspension, syrup or tincture. In addition to the active compounds, the liquid dosage form may comprise inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide and oil, especially cottonseed oil, peanut oil, corn germ oil, olive oil, castor oil and sesame oil or the mixture thereof, etc.

Solid dosage forms such as tablets, sugar pills, capsules, pills and granules can be prepared by coating and shell materials, such as casing and other materials known in the art. They may comprise an opaque agent, and the release of the active compound or compound in the composition may be delayed in a certain part of the digestive tract. Examples of embedding components that can be used are polymers and waxes. If necessary, the active compound may also form a microcapsule form with one or more of above excipients.

The dosage form of the compound of the present invention for local administration includes ointment, powder, patch, spray and inhalant. The active ingredient is mixed in sterile conditions with a biologically acceptable carrier and any preservatives, buffers, or propellants that may be required if necessary.

Except for these inert diluents, the composition may also include auxiliaries such as wetting agents, emulsifiers and suspensions, sweeteners, flavouring agents and perfumes.

Except for the active compounds, the suspension may contain a suspending agent, such as ethoxylated isooctadecanol, polyoxyethylene sorbitol and dehydrated sorbitol ester, microcrystalline cellulose, aluminum methoxide and agar or the mixture thereof, etc.

The compound of the present invention can be administered alone or in combination with other pharmaceutically acceptable compounds.

When the pharmaceutical composition is used, the safe and effective amount of the compound of the present invention is administrated to the mammal (such as human) in need thereof, in which the dosage is the pharmaceutically acceptable safe and effective dosage.

When the pharmaceutical composition is used, the safe and effective amount of the compound of the present invention is administrated to the mammal (such as human) that need to be treated, in which the pharmaceutically effective dosage is given.

Compared with the technology currently available, the compounds provided by the present invention have achieved the following beneficial effects:
1. It is well-known to those skilled in the art that propofol and cyclopropylphenol cannot form salts and are insoluble in water, which limits their clinical applications. However, the compounds of the present invention can form salts, and each salt form has excellent water - solubility and can meet the requirements of the preparation.
2. It is well-known to those skilled in the art that due to the water - insolubility of propofol and cyclopropylphenol, the main dosage forms used clinically are fat emulsions. Fat emulsions are likely to cause a variety of adverse reactions clinically. However, the salt forms of the compounds of the present invention have excellent solubility and the solubility meets the requirements of the preparation, which can effectively overcome the adverse reactions caused by lipid emulsions and improve safety.
3. It is well-known to those skilled in the art that the prodrugs of propofol and cyclopropylphenol have a certain degree of water-solubility, but their onset time is slow and does not meet the requirements of rapid onset and rapid recovery. However, the compounds of the present invention are not prodrugs but active ingredients, and they have a short onset time and can rapidly produce general anesthetic effects.

In summary, the compounds provided by the present invention have excellent salt-forming properties and water solubility, meeting solubility formulation requirements. Additionally, they have a low minimum effective anesthetic dose, enabling rapid onset and recovery. These compounds overcome the drawbacks of propofol, its prodrugs, and fat emulsions. Therefore, they have broad application prospects in preparing drugs with sedative, hypnotic, and/or anesthetic effects and in managing status epilepticus, providing a new option for the clinical development of such drugs and for controlling conditions like status epilepticus.

Clearly, based on the above content of the present invention, various other modifications, substitutions, or changes can be made by using common technical knowledge and conventional methods in the field without departing from the fundamental technical concept of the invention.

The following detailed description, through specific implementation examples, further elaborates on the above content of the present invention. Nevertheless, it should not be construed as limiting the scope of the invention to the examples provided below. All technical solutions realized based on the above content of the invention are within the scope of the present invention.

### Detailed Description of the Invention

The raw materials and equipment used in the implementation of this invention are commercially available products.

The structure of the compounds was determined by nuclear magnetic resonance (NMR) and/or mass spectrometry (MS). NMR shifts (δ) are given in units of 10⁻⁶ (ppm). NMR measurements were performed using a Bruker Avance III 400 spectrometer, with deuterated dimethyl sulfoxide (d₆-DMSO) or deuterated methanol (CD₃OD) as the solvent and tetramethylsilane (TMS) as the internal standard.

Liquid chromatography-mass spectrometry (LCMS) was performed on an Agilent 1260-6110 LCMS system (ESI) with a Waters X-Bridge C18 column (50 mm × 4.6 mm × 3.5 µm). The column temperature was maintained at 40°C, and the flow rate was 2.0 mL/min. The mobile phase consisted of a gradient program: starting with 95% [water + 0.05% trifluoroacetic acid (TFA)] and 5% [acetonitrile (CH₃CN) + 0.05% TFA], transitioning to 0% [water + 0.05% TFA] and 100% [CH₃CN + 0.05% TFA] over 3 minutes, holding for 1 minute, then returning to the initial conditions (95% [water + 0.05% TFA] and 5% [CH₃CN + 0.05% TFA]) over 0.05 minutes, and maintaining these conditions for an additional 0.7 minutes.

### 1) Materials and Reagents

The silica gel plate (HSGF254) for thin layer chromatography was bought from Yantai Xinnuo Chemical Co., Ltd, with the thickness of 1 mm.

Thin layer chromatography (TLC) was bought from Yantai Jiangyou silicone Development Co., Ltd., with the thickness of 0.2±0.03 mm.

Silica gel used for column chromatography was mostly made by Rushan Sun Desiccant Co., Ltd. (Weihai, Shandong) with 100-200 meshes or 200-300 meshes.

### 2) The Main Instruments

Electronic Balance Sartorius BSA124S (manufactured by Sartorius Scientific Instruments (Beijing) Co., Ltd.);
MS-H-PRO⁺ digital control heating type magnetic stirrer (Dragon Lab Instruments Beijing Co., Ltd.)
Contact Voltage Regulator (manufacturer: Zhejiang Tianzheng Electric Co., Ltd);
Temperature Controller (made by Shanghai Lulin Electric Co., Ltd);
Three-function Ultraviolet Analysis (model: ZF-2, manufactured by Shanghai Anting Electronic Instrument Factory);
Rotary Evaporator R-201 (manufactured by Shanghai Shenshun Biological Technology Co., Ltd)
Constant Temperature Water Bath (model: W201D, manufactured by Shanghai Shenshun Biological Technology Co., Ltd)
Circulating Water Vacuum Pump SHB-III (manufactured by Zhengzhou Huicheng Technology Industry and Trade Co., Ltd)
Mobile Water Pump SHB-B95 (manufactured by Zhengzhou Huicheng Technology Industry and Trade Co., Ltd)
Low-temperature Cooling Liquid Circulating Pump (manufactured by Gongyi Yuhua Instrument Co., Ltd)
2XZ-2 vane-type vacuum pump (Linhai Yongwu Vacuum Equipment Co., Ltd.);
VRD-16 bipolar vane-type vacuum pump (Zhejiang Feiji Electromechanical Co., Ltd.);
DGJ-10C vacuum freeze-dryer (Shanghai Biorise Biotechnology Co., Ltd.);
Biotage Isolera One flash preparative liquid-phase chromatograph (Biotage Sweden AB).

### Example 1 Preparation of Compound A1 and A1 Hydrochloride

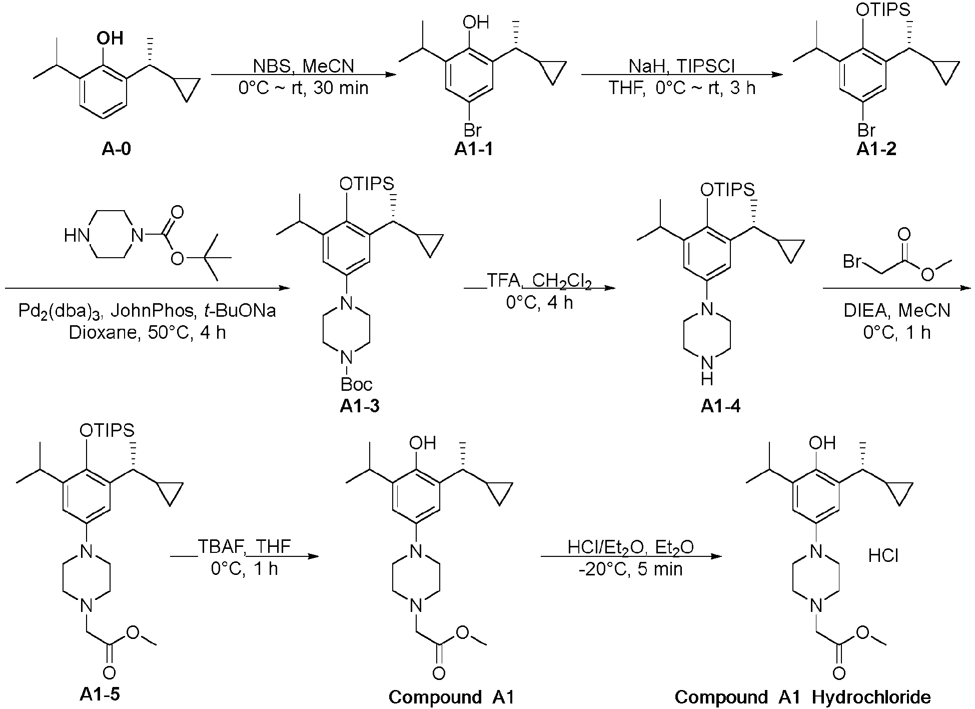

### 1. Preparation of Compound A1-1

At 0°C, NBS (6.71 g, 37.70 mmol) was added dropwise into a solution of A-0 (7.70 g, 37.69 mmol) in acetonitrile (77 mL), and the mixture was stirred at 0°C for 30 minutes. After confirming reaction completion by TLC, H₂O (100 mL) was added. The mixture was extracted with EtOAc (3 × 70 mL). The combined organic layers were washed with saturated brine, dried over anhydrous Na₂SO₄, filtered, and concentrated under reduced pressure to obtain a crude product. The crude product was purified by silica gel column chromatography (dichloromethane/petroleum ether (v/v) = 1/200 to 1/50). TLC (dichloromethane/petroleum ether (v/v) = 1/20) monitoring was performed, and fractions with Rf = 0.4-0.5 were collected to yield Compound **A1-1** as yellow oil (4.21 g, yield 39.4%). ESI[M + H]⁺ = 283.1.

### 2. Preparation of Compound A1-2

At 0°C, NaH (196 mg, 60%, 4.90 mmol) was added in portions to a solution of **A1-1** (1.26 g, 4.45 mmol) in dry tetrahydrofuran (12 mL), after the mixture was stirred for 30 minute, TIPSCl (891 mg, 4.62 mmol) was added to the mixture. The reaction system was warmed to room temperature and stirred for 3 hours. After confirming reaction completion by TLC, water (20 mL) was added to the reaction system. The organic phase was extracted with EtOAc (3 × 30 mL), washed with saturated brine, dried over anhydrous Na₂SO₄, filtered, and concentrated under reduced pressure to obtain a crude product. The crude product was purified by silica gel column chromatography (heptane as eluent), TLC monitoring (ethyl acetate/petroleum ether (v/v) = 1/50) was performed, and the fractions with Rf = 0.5 to 0.6 were collected to yield compound **A1-2** as white solid (1.93 g, yield 98.7%). ESI[M + H]⁺ = 439.3.

### 3. Preparation of Compound A1-3

At room temperature, compound **A1-2** (1.2 g, 2.73 mmol), Pd₂(dba)₃ (125 mg, 0.14 mmol), t-BuONa (394 mg, 4.10 mmol), and JohnPhos (81 mg, 0.27 mmol) were dissolved in 1,4-dioxane (15 mL), the reaction system was purged with nitrogen three times, and then stirred at 50°C for 4 hours under nitrogen protection. After confirming reaction completion by TLC, the crude product was obtained by concentration under reduced pressure and purified by silica gel column chromatography (ethyl acetate/petroleum ether (v/v) = 1/100 to 1/5). TLC monitoring (ethyl acetate/petroleum ether (v/v) = 1/5) was performed, and the fractions with Rf = 0.3 to 0.4 were collected to yield compound **A1-3** as white solid (1.48 g, yield 99.5%). ESI[M + H]⁺ = 545.3.

### 4. Preparation of Compound A1-4

At 0°C, TFA (2 mL) was added to a solution of **A1-3** (1.48 g, 2.72 mmol) in dichloromethane (10 mL), and the mixture was stirred at 0°C for 4 hours. After confirming reaction completion by TLC, the reaction mixture was concentrated under reduced pressure, basified with saturated aqueous sodium bicarbonate solution (20 mL), and extracted with EtOAc (3 × 30 mL), the combined organic layers were dried over anhydrous Na₂SO₄, filtered and concentrated under reduced pressure to obtain a crude produc. The crude product was purified by silica gel column chromatography (dichloromethane / methanol (v/v) = 100/1 - 20/1). TLC monitoring (dichloromethane/methanol (v/v) = 10/1) was performed, and the fractions with Rf = 0.4 to 0.5 were collected to yield compound **A1-4** as white solid (1.20 g, yield 99.2%). ESI[M + H]⁺ = 445.3.

### 5. Preparation of Compound A1-5

At 0°C, DIEA (349 mg, 2.70 mmol) and methyl bromoacetate (303 mg, 1.98 mmol) were successively added to a solution of **A1-4** (800 mg, 1.80 mmol) in acetonitrile (8 mL), the mixture was stirred at 0°C for 1 hour. After confirming reaction completion by TLC, water (10 mL) was added to the reaction system. The organic phase was extracted with EtOAc (3 × 15 mL), dried over anhydrous Na₂SO₄, filtered, and concentrated under reduced pressure to obtain a crude product. The crude product was purified by silica gel column chromatography (ethyl acetate/petroleum ether (v/v) = 1/100 to 1/5). TLC monitoring (ethyl acetate/petroleum ether (v/v) = 1/2) was performed, and the fractions with Rf = 0.5 to 0.6 were collected to yield compound **A1-5** as white solid (692 mg, yield 74.4%). ESI[M + H]⁺ = 517.4.

### 6. Preparation of Compound A1

At 0°C, TBAF (1.34 mL, 1 mol/L in THF, 1.34 mmol) was added to a solution of **A1-5** (692 mg, 1.34 mmol) in tetrahydrofuran (7 mL), and the mixture was stirred at 0°C for 1 hour. After confirming reaction completion by TLC, water (10 mL) was added to the reaction system. The organic phase was extracted with EtOAc (3 × 15 mL), dried over anhydrous Na₂SO₄, filtered, and concentrated under reduced pressure to obtain a crude product. The crude product was purified by silica gel column chromatography (ethyl acetate/petroleum ether (v/v) = 1/100 to 1/2). TLC monitoring (ethyl acetate/petroleum ether (v/v) = 1/2) was performed, and the fractions with Rf = 0.3 to 0.4 were collected to yield **Compound A1** as white solid (200 mg, yield 41.4%). ESI[M + H]⁺ = 361.3.
¹H NMR (400MHz, CD₃OD) δ 6.84 (d, *J* = 2.9Hz,1H), 6.73 (d, *J* = 2.9Hz, 1H), 3.76 (s, 3H), 3.34 (s, 2H), 3.33 - 3.27 (m, 1H), 3.16 - 3.09 (m, 4H), 2.82 - 2.74 (m, 4H), 2.55 - 2.45 (m, 1H), 1.29 (d, *J* = 7.0 Hz, 3H), 1.24 (d, *J* = 6.9 Hz, 3H), 1.23 (d, *J* = 6.9 Hz, 3H), 1.11 - 1.00 (m, 1H), 0.62 - 0.52 (m, 1H), 0.44 - 0.34 (m, 1H), 0.26 - 0.17 (m, 1H), 0.17 - 0.09 (m, 1H).

### 7. Preparation of Compound A1 Hydrochloride

At -20°C, HCl/Et₂O (0.155 mL, 2 mol/L in THF, 0.31 mmol) was slowly added to a solution of **A1** (112 mg, 0.31 mmol) in diethyl ether (5 mL), the mixture was stirred at -20°C for 5 minutes, and concentrated under reduced pressure at low temperature to obtain a crude product. The crude product was washed with diethyl ether to yield the **compound A1 Hydrochloride** as white solid (71.7 mg, yield 58.3%). ESI[M + H]⁺ = 361.3.
¹H NMR (400MHz, CD₃OD) δ 7.01 (s, 1H), 6.89 (s, 1H), 4.06 (s, 2H), 3.84 (s, 3H), 3.54 - 3.36 (m, 8H), 3.35 - 3.25 (m, 1H), 2.52 - 2.41 (m, 1H), 1.26 (d, *J =* 6.9 Hz, 3H), 1.21 (d, *J =* 6.9 Hz, 6H), 1.09 - 0.99 (m, 1H), 0.61 - 0.52 (m, 1H), 0.40 - 0.32 (m, 1H), 0.25 - 0.16 (m, 1H), 0.12 - 0.03 (m, 1H).

### Example 2 Preparation of Compound A2 and Compound A2 hydrochloride, Compound A5 and Compound A5 hydrochloride, Compound A6 and Compound A6 hydrochloride

The preparation methods of **Compound A2, Compound A5,** and **Compound A6** are similar to those for **Compound A1,** starting from **A1-4** and undergoing substitution and deprotection reactions with brominated compounds. The preparation methods of the corresponding **Compound A2 hydrochloride, Compound A5 hydrochloride,** and **Compound A6 hydrochloride** are also similar to that of the **Compound A1 hydrochloride.** The preparation routes are as follows:

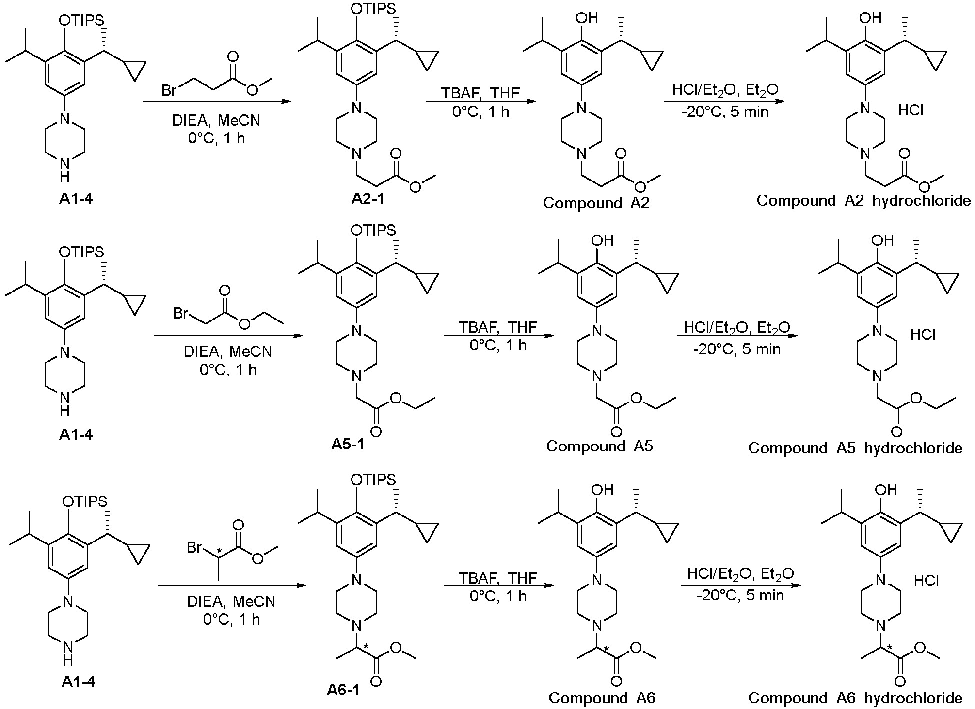

**Compound A2 hydrochloride:** white solid, 71.2 mg, ESI[M + H]⁺=375.3.
¹H NMR (400MHz, d*₆*-DMSO) 10.61 (s, 1H), 6.75 (s, 1H), 6.63 (s, 1H), 3.67 (s, 3H), 3.65 - 3.51 (m, 4H), 3.46 - 3.35 (m, 2H), 3.31 - 3.22 (m, 1H), 3.22 - 3.10 (m, 2H), 3.07 - 2.91 (m, 4H), 2.48 - 2.40 (m, 1H), 1.18 (d, *J =* 6.9 Hz, 3H), 1.14 (d, *J* = 6.8 Hz, 6H), 1.07 - 0.98 (m, 1H), 0.52 - 0.44 (m, 1H), 0.34 - 0.24 (m, 1H), 0.18 - 0.11 (m, 1H), 0.09 - 0.01 (m, 1H).

**Compound A5 hydrochloride:** white solid, 132.9 mg, ESI[M + H]⁺ = 375.3.
¹H NMR (400 MHz, CD₃OD) δ 7.16 (d, *J* = 2.9 Hz, 1H), 7.03 (d, *J =* 2.8 Hz, 1H), 4.32 (q, *J* = 7.1 Hz, 2H), 4.18 (s, 2H), 3.68 - 3.58 (m, 8H), 3.35 - 3.27 (m, 1H), 2.54 - 2.42 (m, 1H), 1.33 (t, *J* = 7.1 Hz, 3H), 1.27 (d, *J* = 6.9 Hz, 3H), 1.22 (d, *J =* 6.9 Hz, 6H), 1.11 - 1.01 (m, 1H), 0.63 - 0.53 (m, 1H), 0.43 - 0.32 (m, 1H), 0.27 - 0.18 (m, 1H), 0.13 - 0.04 (m, 1H).

**Compound A6 hydrochloride:** white solid, 114.7 mg, ESI[M + H]⁺ = 375.3.
¹H NMR (400 MHz, CD₃OD) δ 7.30 (d, *J* = 2.9 Hz, 1H), 7.16 (d, *J =* 2.9 Hz, 1H), 4.28 (q, *J* = 7.2 Hz, 1H), 3.87 (s, 3H), 3.81 - 3.76 (m, 4H), 3.72 - 3.60 (m, 4H), 3.36 - 3.31 (m, 1H), 2.55 - 2.45 (m, 1H), 1.64 (d, *J* = 7.2 Hz, 3H), 1.28 (d, *J* = 6.9 Hz, 3H), 1.23 (d, *J* = 6.9 Hz, 6H), 1.14 - 1.03 (m, 1H), 0.65 - 0.55 (m, 1H), 0.45 - 0.36 (m, 1H), 0.29 - 0.16 (m, 1H), 0.13 - 0.04 (m, 1H).

### Example 3 Preparation of Compound A3 and Compound A3 hydrochloride

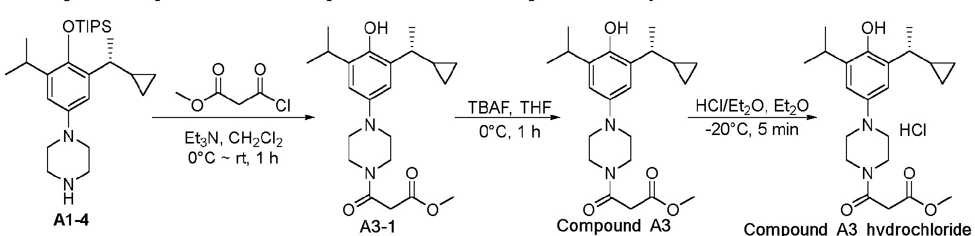

### 1. Preparation of Compound A3-1

At 0°C, Et₃N (205 mg, 2.0 mmol) and methyl 3-chloro-3-oxopropanoate (184 mg, 1.35 mmol) were sequentially added into a solution of **A1-4** (300 mg, 0.67 mmol) in dichloromethane (5 mL), then the mixture was stirred at room temperature for 1 hour. After confirming reaction completion by TLC, water (10 mL) was added to the reaction system. The organic phase was extracted with dichloromethane (3 × 15 mL), dried over anhydrous Na₂SO₄, filtered, and concentrated under reduced pressure to obtain a crude product. The crude product was purified by preparative TLC (ethyl acetate/petroleum ether (v/v) = 1/3), and the fractions with Rf = 0.5 to 0.6 were collected to yield compound **A3-1** as white solid (350 mg, yield 95.2%). ESI[M + H]⁺ = 545.3.

### 2. Preparation of Compound A3

At 0°C, TBAF (0.64 mL, 1 mol/L in THF, 0.64 mmol) was added to a solution of **A3-1** (350 mg, 0.64 mmol) in tetrahydrofuran (5 mL), and the mixture was stirred at 0°C for 1 hour. After confirming reaction completion by TLC, water (10 mL) was added to the reaction system. The organic phase was extracted with EtOAc (3 × 15 mL), dried over anhydrous Na₂SO₄, filtered, and concentrated under reduced pressure to obtain a crude product. The crude product was purified by preparative TLC (ethyl acetate/petroleum ether (v/v) = 1/2) and the fractions with Rf = 0.3 to 0.4 were collected to yield **compound A3** as white solid (224.5 mg, yield 89.9%). ESI[M + H]⁺ = 389.2.

### 3. Preparation of Compound A3 hydrochloride

At -20°C, HCl/Et₂O (0.29 mL, 2 mol/L in THF, 0.58 mmol) of solution was slowly added to a solution of **A3** (224.5 mg, 0.58 mmol) in diethyl ether (5 mL), and the mixture was stirred at -20°C for 5 minutes, and concentrated under reduced pressure at low temperature to obtain a crude product. The crude product was washed with diethyl ether to yield **compound A3 hydrochloride** as white solid (224.6 mg, yield 91.5%). ESI[M + H]⁺ = 389.2.
¹H NMR (400MHz, d*₆*-DMSO) δ 7.44 (s, 1H), 6.70 (d, *J =* 2.8 Hz, 1H), 6.59 (d, *J =* 2.8 Hz, 1H), 3.63 (s, 3H), 3.62 - 3.56 (m, 4H), 3.55 - 3.49 (m, 2H), 3.31 - 3.21 (m, 1H), 3.01 - 2.95 (m, 2H), 2.96 - 2.89 (m, 2H), 2.48 - 2.40 (m, 1H), 1.17 (dd, *J* = 7.0, 3.2 Hz, 3H), 1.13 (d, *J* = 6.8 Hz, 6H), 1.06 - 0.95 (m, 1H), 0.52 - 0.44 (m, 1H), 0.34 - 0.24 (m, 1H), 0.18 - 0.10 (m, 1H), 0.09 - 0.02 (m, 1H).

### Example 4 Preparation of Compounds A4 and Compound A4 hydrochloride

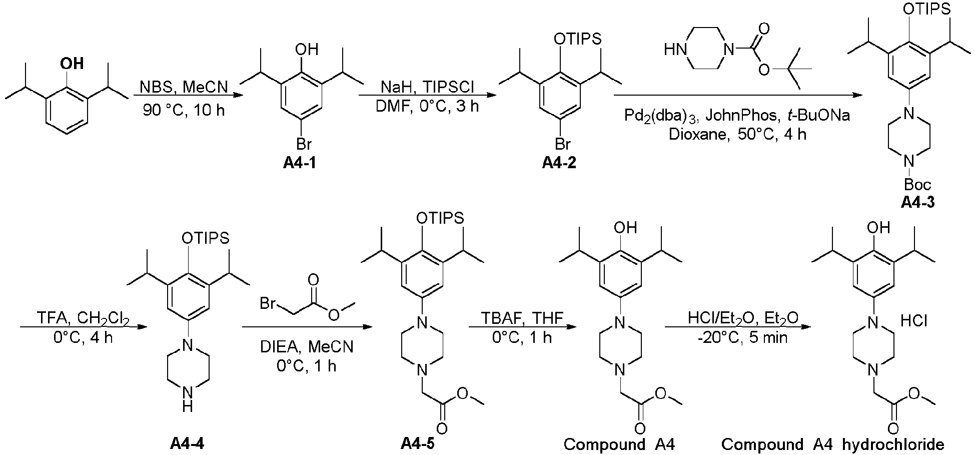

The preparation methods of **Compound A4 and Compound A4 hydrochloride** are similar to those for **Compound A1**, and prepared using propofol as the starting material.

**Compound A4 hydrochloride:** white solid, 135.9 mg, ESI[M + H]⁺ = 335.3.
¹H NMR (400MHz, CD₃OD) δ 6.98 (s, 2H), 4.14 (s, 2H), 3.85 (s, 3H), 3.61 - 3.50 (m, 8H), 3.36 - 3.31 (m, 2H), 1.22 (d, *J* = 6.9 Hz, 12H).

### Example 5 Preparation of Compound A7 and Compound A7 hydrochloride

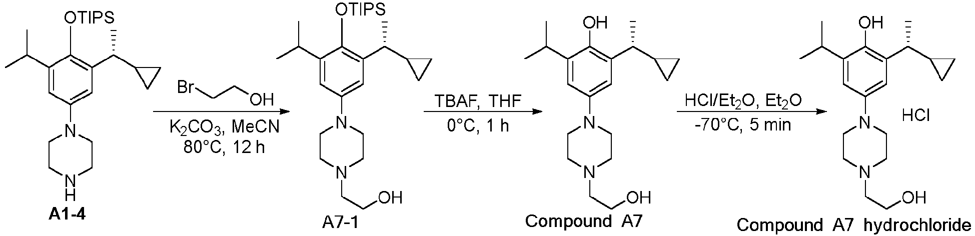

### 1. Preparation of Compound A7-1

At room temperature, K₂CO₃ (93.2 mg, 0.67 mmol) and 2-bromoethanol (61.8 mg, 0.49 mmol) were sequentially added into a solution of **A1-4** (200 mg, 0.45 mmol) in acetonitrile (3 mL), then the mixture was stirred at 80°C for 12 hours. After confirming reaction completion by TLC, water (10 mL) was added to the reaction system. The organic phase was extracted with EtOAc (3 × 15 mL), dried over anhydrous Na₂SO₄, filtered, and concentrated under reduced pressure to obtain a crude product. The crude product was purified by preparative TLC (dichloromethane/methanol (v/v) = 10/1) and the fractions with Rf = 0.5 to 0.6 were collected to yield compound **A7-1** as white solid (115 mg, yield 52.3%). ESI[M + H]⁺ = 489.3.

### 2. Preparation of Compound A7

At 0°C, TBAF (0.24 mL, 1 mol/L in THF, 0.24 mmol) was added to a solution of **A7-1** (115 mg, 0.24 mmol) in tetrahydrofuran (3 mL), and the mixture was stirred at 0°C for 1 hour. After confirming reaction completion by TLC, water (10 mL) was added to the reaction system. The organic phase was extracted with EtOAc (3 × 15 mL), dried over anhydrous Na₂SO₄, filtered, and concentrated under reduced pressure to obtain a crude product. The crude product was purified by preparative TLC (dichloromethane/methanol (v/v) = 10/1) and the fractions with Rf = 0.4 to 0.5 were collected to yield **Compound A7** as white solid (70.6 mg, yield 90.3%). ESI[M + H]⁺ = 333.4.

### 3. Preparation of Compound A7 hydrochloride

At -70°C, HCl/Et₂O (0.16 mL, 2 mol/L in THF, 0.32 mmol) of solution was slowly added to a solution of **A7** (70.6 mg, 0.21 mmol) in diethyl ether (3 mL), and the mixture was stirred at -70°C for 5 minutes, and concentrated under reduced pressure at low temperature to obtain a crude product. The crude product was washed with diethyl ether to yield **Compound A7 hydrochloride** as white solid (76.4 mg, yield 92.9%). ESI[M + H]⁺ = 333.4.
¹H NMR (400 MHz, CD₃OD) δ 7.19 (d, *J* = 2.9 Hz, 1H), 7.06 (d, *J* = 2.9 Hz, 1H), 3.97 - 3.93 (m, 2H), 3.88 - 3.52 (m, 8H), 3.46 - 3.40 (m, 2H), 3.35 - 3.31 (m, 1H), 2.52 - 2.42 (m, 1H), 1.27 (d, *J* = 6.9 Hz, 3H), 1.23 (d, *J* = 6.9 Hz, 6H), 1.12 - 1.00 (m, 1H), 0.63 - 0.53 (m, 1H), 0.44 - 0.33 (m, 1H), 0.27 - 0.15 (m, 1H), 0.13 - 0.05 (m, 1H).

### Example 6 Preparation of Compound A8 and Compound A8 hydrochloride

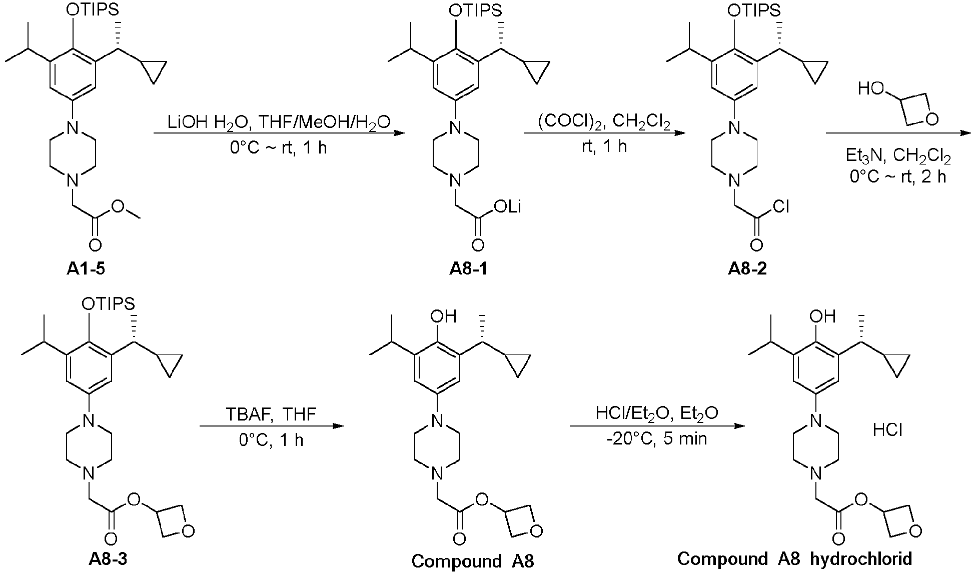

### 1. Preparation of Compound A8-1

At 0°C, LiOH·H2O (146 mg, 3.48 mmol) was added to a solution of **A1-5** (900 mg, 1.74 mmol) in THF/MeOH/H₂O (9 mL, v/v/v = 1/1/1), and the mixture was stirred at room temperature for 1 hour. After confirming reaction completion by TLC, the mixture was concentrated under reduced pressure to obtain a crude product. The crude product was purified by silica gel column chromatography (dichloromethane/methanol (v/v) = 100/1 to 10/1). TLC monitoring (dichloromethane/methanol (v/v) = 10/1) was performed, and the fractions with Rf = 0.4 to 0.5 were collected to yield compound **A8-1** as white solid (730 mg, yield 82.4%). ESI[M + H]⁺ = 503.3.

### 2. Preparation of Compound A8-2

At room temperature, oxalyl chloride (175 mg, 1.38 mmol) was added to a solution of **A8-1** (233 mg, 0.46 mmol) in dichloromethane (5 mL), and the mixture was stirred at room temperature for 1 hour. After confirming reaction completion by TLC, the mixture was concentrated under reduced pressure to obtain a crude product **A8-2** (260 mg). Tthe crude product **A8-2** was used directly in the next step without further purification.

### 3. Preparation of Compound A8-3

At 0°C, Et₃N (93 mg, 0.92 mmol) and oxetan-3-ol (40.9 mg, 0.55 mmol) were added to a solution of the crude product **A8-2** (260 mg) in dichloromethane (3 mL), and the mixture was stirred at room temperature for 2 hours. After confirming reaction completion by TLC, water (10 mL) was added to the reaction system. The organic phase was extracted with dichloromethane (3 × 15 mL), dried over anhydrous Na₂SO₄, filtered, and concentrated under reduced pressure to obtain a crude product. The crude product was purified by preparative TLC (ethyl acetate/petroleum ether (v/v) = 1/2) and the fractions with Rf = 0.3 to 0.4 were collected to yield compound **A8-3** as white solid (76.5 mg, two-step yield 29.9%). ESI[M + H]⁺ = 559.3.

### 4. Preparation of Compound A8

At 0°C, TBAF (0.14 mL, 1 mol/L in THF, 0.14 mmol) was added to a solution of **A8-3** (76.5 mg, 0.14 mmol) in tetrahydrofuran (2 mL), and the mixture was stirred at 0°C for 1 hour. After confirming reaction completion by TLC, water (5 mL) was added to the reaction system. The organic phase was extracted with dichloromethane (3 × 15 mL), dried over anhydrous Na₂SO₄, filtered, and concentrated under reduced pressure to obtain a crude product. The crude product was purified by preparative TLC (ethyl acetate/petroleum ether (v/v) = 1/1) and the fractions with Rf = 0.3 to 0.4 were collected to yield **Compound A8** as white solid (54 mg, yield 98.0%). ESI[M + H]⁺ = 403.3.

### 5. Preparation of Compound A8 hydrochloride

At -20°C, HCl/Et₂O (0.10 mL, 2 mol/L in THF, 0.20 mmol) of solution was slowly added to a solution of **A8** (54 mg, 0.13 mmol) in diethyl ether (3 mL), and the mixture was stirred at -20°C for 5 minutes, and concentrated under reduced pressure at low temperature to obtain a crude product. The crude product was washed with diethyl ether to yield the **Compound A8 hydrochloride** as white solid (34.5 mg, yield 56.3%). ESI[M + H]⁺ = 403.3.
¹H NMR (400 MHz, CD₃OD) δ 7.15 (s, 1H), 7.02 (s, 1H), 5.64 - 5.57 (m, 1H), 4.95-4.90 (m, 2H), 4.70 - 4.64 (m, 2H), 4.13 - 4.04 (m, 2H), 3.62 - 3.54 (m, 4H), 3.51 - 3.41 (m, 4H), 3.35 - 3.31 (m, 1H), 2.55 - 2.41 (m, 1H), 1.27 (d, *J* = 6.9 Hz, 3H), 1.22 (d, *J* = 6.8 Hz, 6H), 1.11 - 1.00 (m, 1H), 0.63 - 0.53 (m, 1H), 0.45 - 0.32 (m, 1H), 0.25 - 0.17 (m, 1H), 0.14 - 0.05 (m, 1H).

### Example 7 Preparation of Compound A6R and Compound A6R Sulfate

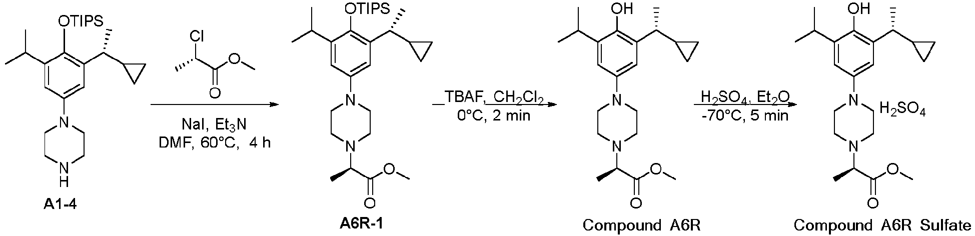

### 1. Preparation of Compound A6R-1

At room temperature, Et₃N (910.7 mg, 9.0 mmol, 2.67 eq), NaI (1.35 g, 9.0 mmol, 2.67 eq) and (S)-2-chloropropionic acid methyl ester (1.10 g, 9.0 mmol, 2.67 eq) were sequentially added into a solution of A1-4 (1.5 g, 3.37 mmol, 1 eq) in dry DMF (20 mL), then the mixture was stirred at 60°C for 4 hours. After TLC (methanol/dichloromethane (v/v) = 1/10) confirmed complete reaction, the mixture was cooled to room temperature, H₂O (30 mL) was added to the reaction system. The organic phase was extracted with EtOAc (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 10%). TLC monitoring (ethyl acetate/petroleum ether (v/v) = 1/5) was performed, and the fractions with Rf = 0.6 were collected to yield compound **A6R-1** as colorless oil (1.685 g, yield 94.2%). ESI[M + H]⁺ = 531.4.

### 2. Preparation of Compound A6R

At 0°C, TBAF (4.9 mL, 1 mol/L in THF, 4.9 mmol, 1.5 eq) was added to a solution of **A6R-1** (1.685 g, 3.17 mmol, 1 eq) in dry tetrahydrofuran (20 mL), and the mixture was stirred at 0°C for 2 minutes. After confirming reaction completion by TLC (ethyl acetate/petroleum ether (v/v) = 1/5), water (30 mL) was added to the reaction system. The organic phase was extracted with EtOAc (3 × 10 mL), washed with saturated brine (20 mL), dried over anhydrous Na₂SO₄, filtered and concentrated under reduced pressure to obtain a crude product. The crude product was purified by flash column chromatography. TLC monitoring (ethyl acetate/petroleum ether (v/v) = 1/5) was performed, and the fractions with Rf = 0.5 were collected to yield **Compound A6R** as colorless oil (1085.8 mg, yield 91.5%). ESI[M + H]⁺ = 375.2.

### 3. Preparation of Compound A6R Sulfate

At room temperature, **Compound A6R** (1085.8 mg, 2.90 mmol, 1.0 eq) was dissolved in diethyl ether (20 mL), and the reaction system was cooled to -70°C using a dry ice-ethanol bath. H₂SO₄ (568.65 mg, 5.80 mmol, 2.0 eq) was slowly added in Et₂O (2 mL) to a solution of **Compound A6R** (112 mg, 0.31 mmol) in diethyl ether (30 mL), the mixture was stirred at -70°C for 5 minutes, and then concentrated under reduced pressure at low temperature to obtain a crude product. Et₂O (30 mL) was added to the crude product, the mixture was stirred, filtered and the solid was washed with Et₂O (3 × 20 mL). The solid was dissolved in ultrapure water (30 mL), and lyophilized for 64 hours to yield **Compound A6R Sulfate** as white solid (1526.42 mg, yield 100%). ESI[M + H]⁺ = 375.2. ¹H NMR (400 MHz, CD₃OD) δ 7.27 (d, *J=* 2.9 Hz, 1H), 7.14 (d, *J=* 2.9 Hz, 1H), 4.25 (q, *J=* 7.2 Hz, 1H), 3.87 (s, 3H), 3.79 - 3.72 (m, 4H), 3.69 - 3.57 (m, 4H), 3.35 - 3.31 (m, 1H), 2.54 - 2.41 (m, 1H), 1.63 (d, *J=* 7.2 Hz, 3H), 1.28 (d, *J=* 6.9 Hz, 3H), 1.23 (d, *J=* 6.9 Hz, 6H), 1.11 - 1.03 (m, 1H), 0.62 - 0.55 (m, 1H), 0.44 - 0.36 (m, 1H), 0.26 - 0.18 (m, 1H), 0.11 - 0.03 (m, 1H).

### Example 8 Preparation of Compound A6S and Compound A6S Sulfate

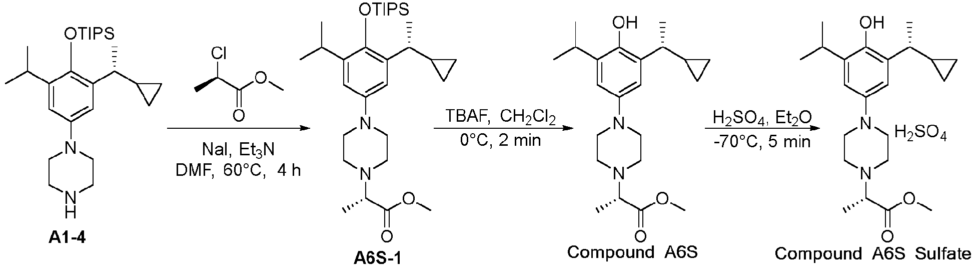

### 1. Preparation of Compound A6S-1

At room temperature, Et₃N (1.82 g, 18.0 mmol, 3 eq), NaI (2.70 g, 18.0 mmol, 3 eq) and (S)-2-chloropropionic acid methyl ester (2.21 g, 18.0 mmol, 3 eq) were sequentially added into a solution of A1-4 (2.67 g, 6.0 mmol, 1 eq) in dry DMF (25 mL), then the mixture was stirred at 60°C for 4 hours. After TLC (methanol/dichloromethane (v/v) = 1/10) confirmed complete reaction, the mixture was cooled to room temperature,H₂O (30 mL) was added to the reaction mixture. The organic phase was extracted with EtOAc (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 10%). TLC monitoring (ethyl acetate/petroleum ether (v/v) = 1/5) was performed, and the fractions with Rf = 0.6 were collected to yield compound **A6S-1** as colorless oil (2.833 g, yield 88.9%). ESI[M + H]⁺ = 531.4.

### 2. Preparation of Compound A6S

At 0°C, TBAF (4.1 mL, 1 mol/L in THF, 4.1 mmol, 1.2 eq) was added to a solution of **A6S-1** (1.805 g, 3.40 mmol, 1 eq) in dry tetrahydrofuran (20 mL), and the mixture was stirred at 0°C for 2 minutes. After TLC (ethyl acetate/petroleum ether (v/v) = 1/5) confirmed complete reaction, water (30 mL) was added to the reaction mixture. The organic phase was extracted with EtOAc (3 × 10 mL), washed with saturated brine (20 mL), dried over anhydrous Na₂SO₄, filtered and concentrated under reduced pressure to obtain a crude product. The crude product was purified by flash column chromatography. TLC monitoring (ethyl acetate/petroleum ether (v/v) = 1/5) was performed, and the fractions with Rf = 0.5 were collected to yield **Compound A6S** as colorless oil (1.337 g, yield100%). ESI[M + H]⁺ = 375.2.

### 3. Preparation of Compound A6R Sulfate

At room temperature, **A6S** (1.337 g, 3.57 mmol, 1.0 eq) was dissolved in diethyl ether (20 mL), and and the reaction system was cooled to -70°C using a dry ice-ethanol bath. H₂SO₄ (568.65 mg, 5.80 mmol, 2.0 eq) was slowly added in Et₂O (2 mL), the mixture was stirred at -70°C for 5 minutes, and then concentrated under reduced pressure at low temperature to obtain a crude product. Et₂O (30 mL) was added to the crude product, the mixture was stirred, filtered and the solid was washed with Et₂O (3 × 20 mL). The solid was dissolved in ultrapure water (30 mL), and lyophilized for 64 hours to yield **Compound A6S Sulfate** as white solid (1856.5 mg, yield 100%). ESI[M + H]⁺ = 375.2.
¹H NMR (400 MHz, CD₃OD) δ 7.27 (d, *J=* 2.9 Hz, 1H), 7.14 (d, *J=* 2.9 Hz, 1H), 4.25 (q, *J=* 7.2 Hz, 1H), 3.87 (s, 3H), 3.80 - 3.71 (m, 4H), 3.69 - 3.57 (m, 4H), 3.36 - 3.30 (m, 1H), 2.52 - 2.43 (m, 1H), 1.63 (d, *J=* 7.2 Hz, 3H), 1.28 (d, *J=* 6.9 Hz, 3H), 1.23 (d, *J=* 6.9 Hz, 6H), 1.12 - 1.04 (m, 1H), 0.62 - 0.55 (m, 1H), 0.44 - 0.35 (m, 1H), 0.26 - 0.17 (m, 1H), 0.12 - 0.03 (m, 1H).

### Example 9 Preparation of Compound A11, Compound A11 Sulfate and Compound A18, Compound A18 Sulfate

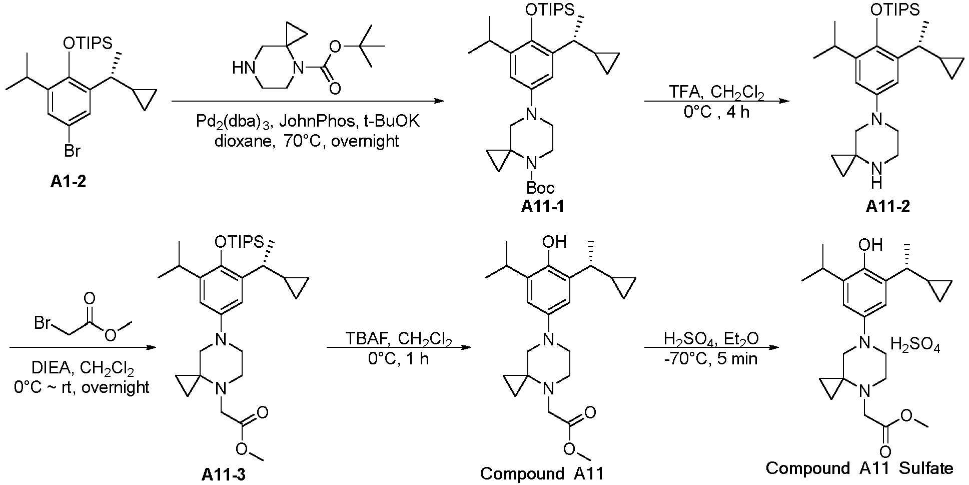

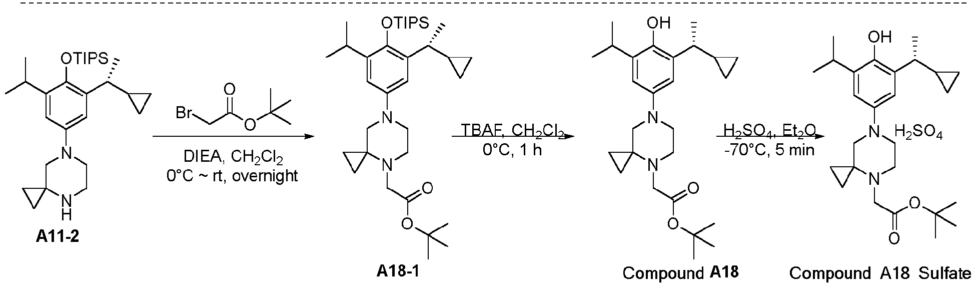

### 1. Preparation of Compound A11-1

At room temperature, compound **A1-2** (6.0 g, 13.65 mmol), 4,7-diazaspiro [2.5] octane-4-carboxylic acid tert-butyl ester (5.0 g, 23.55 mmol), Pd₂(dba)₃ (1.25 g, 1.37 mmol), t-BuOK (3.06 g, 27.30 mmol), and JohnPhos (814.7 mg, 2.73 mmol) were dissolved in 1,4-dioxane (40 mL), the reaction system was purged three times with nitrogen, and the mixture was stirred under nitrogen protection at 70°C overnight. After confirming reaction completion by TLC (n-heptane), the reaction mixture was concentrated under reduced pressure to obtain a crude product. The crude product was purified by flash column chromatography (ethyl acetate/petroleum ether (v/v)=0~15%). TLC (ethyl acetate/petroleum ether (v/v) = 1/5) monitoring was performed, and the fractions with Rf = 0.5 were collected to yield crude compound **A11-1** as colorless oil (5.7 g). ESI[M + H]⁺ = 571.4.

### 2. Preparation of Compound A11-2

At 0°C with an ice-salt bath, TFA (5 mL) was added to a solution of crude compound **A11-1** (5.7 g, 9.98 mmol) in dichloromethane (15 mL), the mixture was stirred at 0°C for 4 hours. After confirming reaction completion by TLC, the mixture was concentrated under reduced pressure, alkalized with saturated aqueous sodium bicarbonate solution (20 mL), and extracted with ethyl acetate (3 × 10 mL). The organic phase was dried over anhydrous Na₂SO₄, filtered and concentrated under reduced pressure to obtain a crude product. The crude product was purified by flash column chromatography (ethyl acetate/petroleum ether (v/v) = 1/1, followed by methanol/dichloromethane (v/v) = 0 ~ 1/1). TLC (methanol/dichloromethane (v/v) = 1/10) monitoring was performed, and the fractions with Rf = 0.5 were collected to yield compound **A11-2** as colorless oil (3.617 g, two-step yield 56.3%). ESI[M + H]⁺ = 471.4.

### 3. Preparation of Compound A11-3

At 0°C with ice-salt bath, DIEA (517.0 mg, 4.0 mmol) and methyl bromoacetate (458.9 mg, 3.0 mmol) were sequentially added to a solution of **A11-2** (941.6 mg, 2.0 mmol) in dichloromethane (20 mL), the mixture was stirred at room temperature overnight. After TLC (methanol/dichloromethane (v/v) = 1/10) confirmed complete reaction, water (20 mL) was added to the reaction system. The organic phase was extracted with ethyl acetate (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 15%). TLC (ethyl acetate/petroleum ether (v/v) = 1/8) was performed, and the fractions with Rf = 0.5 were collected to yield compound **A11-3** as colorless oil (920 mg, yield 77.0%). ESI[M + H]⁺ = 543.4.

### 4. Preparation of Compound A11

At 0°C with ice-salt bath, TBAF (2.0 mL, 1 mol/L in THF, 2.0 mmol) was added to a solution of **A11-3** (920 mg, 1.69 mmol) in dry THF (10 mL), the mixture was stirred at 0°C for 1 hour. After TLC confirmed complete reaction, water (20 mL) was added to the reaction system. The organic phase was extracted with ethyl acetate (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 20%). TLC (ethyl acetate/petroleum ether (v/v) = 1/5) monitoring was performed, and the fractions with Rf = 0.5 were collected to yield crude **Compound A11** as colorless oil (660.2 mg, yield 100%). ESI[M+H]⁺ = 387.2.

### 5. Preparation of Compound A11 Sulfate

At room temperature, **A11** (537 mg, 1.39 mmol, 1.0 eq) was dissolved in diethyl ether (20 mL), and the reaction system was cooled to -70°C using a dry ice-ethanol bath. H₂SO₄ (276.7 mg, 2.82 mmol, 2.0 eq) was slowly added in Et₂O (2 mL) to the reaction system, the mixture was stirred at -70°C for 5 minutes, and then concentrated under reduced pressure at low temperature to obtain a crude product. Et₂O (20 mL) was added to the crude product, the mixture was stirred, filtered and the solid was washed with Et₂O (3 × 20 mL). The solid was dissolved in ultrapure water (30 mL), and lyophilized for for 24 hours to yield **Compound A11 sulfate** as white solid (644.4 mg, yield 100%). ESI[M+H]⁺ = 387.2.
¹H NMR (400 MHz, CD₃OD) δ 7.44 (d, *J=* 2.9 Hz, 1H), 7.31 (d, *J=* 3.0 Hz, 1H), 3.89 (s, 2H), 3.75 (s, 3H), 3.73 - 3.63 (m, 2H), 3.54 - 3.45 (m, 2H), 3.37 - 3.31 (m, 3H), 2.55 - 2.42 (m, 1H), 1.30 (d, *J=* 6.9 Hz, 3H), 1.25 (d, *J=* 6.9 Hz, 6H), 1.14 - 1.05 (m, 3H), 0.94 - 0.86 (m, 2H), 0.64 - 0.56 (m, 1H), 0.45 - 0.36 (m, 1H), 0.31 - 0.17 (m, 1H), 0.14 - 0.03 (m, 1H).

### 6. Preparation of Compound A18 Sulfate

The preparation method of compound **A18** is similar similar to that for **A11. A11-2** (300 mg, 0.64 mmol) was reacted with tert-butyl bromoacetate to obtain **A18-1** (346 mg, 0.59 mmol, yield 92.4%). A18-1 was deprotected with TBAF to obtain compound **A18** (241.2 mg, 0.56 mmol, yield 94.9%). The sulfate salt of compound **A18** was then obtained via salt formation with H₂SO₄/Et₂O to yield **Compound A18 Sulfate** as white solid (197.4 mg, yield 94.9%). ESI[M+H]⁺ = 429.3.
¹H NMR (400MHz, CD₃OD) δ 7.44 (t, *J=* 2.7 Hz, 1H), 7.30 (t, *J=* 2.6 Hz, 1H), 3.89 (s, 2H), 3.80 - 3.43 (m, 6H), 3.37 - 3.32 (m, 1H), 2.55 - 2.42 (m, 1H), 1.50 (s, 5H), 1.30 (d, *J=* 6.9 Hz, 3H), 1.25 (d, *J=* 6.8 Hz, 6H), 1.21 (s, 4H), 1.16 - 1.04 (m, 3H), 0.98 - 0.87 (m, 2H), 0.64 - 0.54 (m, 1H), 0.45 - 0.36 (m, 1H), 0.28 - 0.18 (m, 1H), 0.14 - 0.03 (m, 1H).

### Example 10 Preparation of Compound A12 and Compound A12 Sulfate

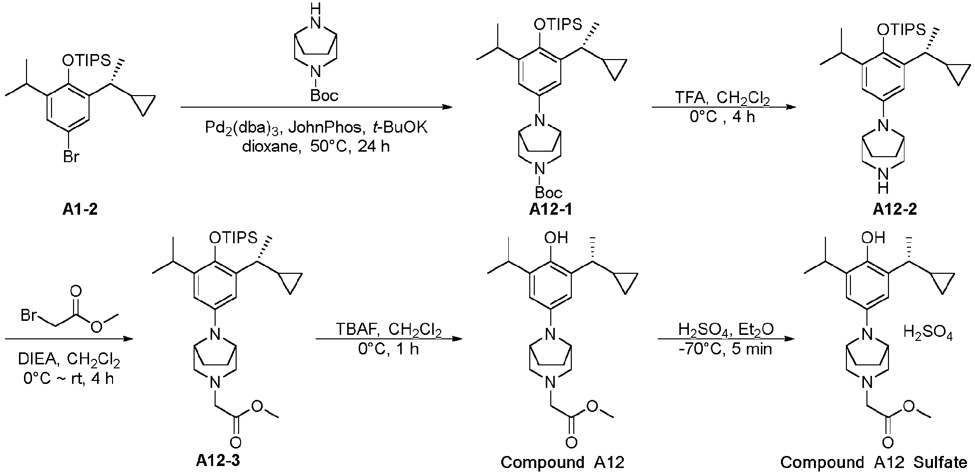

### 1. Preparation of Compound A12-1

At room temperature, compound **A1-2** (2.0 g, 4.55 mmol), tert-butyl (1R,5S)-3,8-diazabicyclo[3.2.1]octane-3-carboxylate (1.93 g, 9.10 mmol), Pd₂(dba)₃ (416.7 mg, 0.455 mmol), t-BuOK (1.02 g, 9.09 mmol) and JohnPhos (271.6 mg, 0.91 mmol) were dissolved in 1,4-dioxane (30 mL), the reaction system was purged three times with nitrogen, and the mixture was stirred under nitrogen protection at 50°C for 24 hours. After confirming reaction completion by TLC (n-heptane), the reaction mixture was concentrated under reduced pressure to obtain a crude product. The crude product was purified by flash column chromatography (ethyl acetate/petroleum ether (v/v)=0~15%). TLC (ethyl acetate/petroleum ether (v/v) = 1/8) monitoring was performed, and the fractions with Rf = 0.5 were collected to yield crude compound **A12-1** as colorless oil (2.328 g). ESI[M + H]⁺ = 571.4.

### 2. Preparation of Compound A12-2

At 0°C with an ice-salt bath, TFA (3 mL) was added to a solution of crude compound **A12-1** (2.328 g) in dichloromethane (15 mL), the mixture was stirred at 0°C for 4 hours. After confirming reaction completion by TLC (n-heptane), the reaction mixture was concentrated under reduced pressure, alkalized with saturated aqueous sodium bicarbonate solution (20 mL), and extracted with ethyl acetate (3 × 10 mL). The organic phase was dried over anhydrous Na₂SO₄, filtered and concentrated under reduced pressure to obtain a crude product. The crude product was purified by flash column chromatography (ethyl acetate/petroleum ether (v/v) = 1/1, followed by methanol/dichloromethane (v/v) = 0 ~ 50%). TLC (methanol/dichloromethane (v/v) = 1/10) monitoring was performed, and the fractions with Rf = 0.5 were collected to yield compound **A12-2** as colorless oil (1.8 g, two-step yield 84.0%). ESI[M + H]⁺= 471.4.

### 3. Preparation of Compound A12-3

At 0°C with ice-salt bath, DIEA (544.0 mg, 4.2 mmol) and methyl bromoacetate (489.5 mg, 3.2 mmol) were sequentially added to a solution of **A12-2** (1.0 g, 2.1 mmol) in dichloromethane (15 mL). The mixture was stirred at room temperature for 4 hours. After confirming reaction completion by TLC (methanol/dichloromethane (v/v) = 1/10), water (20 mL) was added to the reaction system. The organic phase was extracted with dichloromethane (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 15%). TLC (ethyl acetate/petroleum ether (v/v) = 1/8) monitoring was performed, and the fractions with Rf = 0.5 were collected to yield crude compound **A12-3** as colorless oil (1.377 g). ESI[M + H]⁺ = 543.4.

### 4. Preparation of Compound A12

At 0°C with ice-salt bath, TBAF (3.1 mL, 1 mol/L in THF, 3.1 mmol) was added to a solution of **A12-3** (1.377 g, 2.54 mmol) in dry THF (15 mL), the mixture was stirred at 0°C for 1 hour. After confirming reaction completion by TLC, water (20 mL) was added to the reaction system. The organic phase was extracted with ethyl acetate (3 × 10 mL), dried over anhydrous Na₂SO₄, filtered and concentrated under reduced pressure to obtain a crude product. The crude product was purified by flash column chromatography (ethyl acetate/petroleum ether (v/v) = 0 ~ 20%). TLC (ethyl acetate/petroleum ether (v/v) = 1/5) monitoring was performed, and the fractions with Rf = 0.5 were collected to yield crude **Compound A12** as colorless oil (840 mg, two-step yield 100%). ESI[M + H]⁺= 387.3.

### 5. Preparation of Compound A12 Sulfate

At room temperature, **A12** (773.3 mg, 2.0 mmol, 1.0 eq) was dissolved in diethyl ether (20 mL), and the reaction system was cooled to -70°C using a dry ice-ethanol bath. H₂SO₄ (394.2 mg, 4.0 mmol, 2.0 eq) was slowly added in Et₂O (2 mL) to the reaction system, the mixture was stirred at -70°C for 5 minutes, and then concentrated under reduced pressure at low temperature to obtain a crude product. Et₂O (20 mL) was added to the crude product, the mixture was stirred, filtered and the solid was washed with Et₂O (3 × 20 mL). The solid was dissolved in ultrapure water (30 mL), and lyophilized for for 24 hours to yield **Compound A12 Sulfate** as white solid (1060.6 mg, yield 100%). ESI[M + H]⁺ = 387.3.
¹H NMR (400 MHz, CD₃OD) δ 7.13 (d, *J=* 2.5 Hz, 1H), 7.00 (d, *J=* 2.6 Hz, 1H), 4.59 (s, 2H), 3.77 (s, 2H), 3.76 (s, 3H), 3.36 - 3.32 (m, 5H), 2.52 - 2.42 (m, 1H), 2.27 - 2.10 (m, 4H), 1.28 (d, *J=* 6.9 Hz, 3H), 1.23 (d, *J=* 6.9 Hz, 6H), 1.13 - 1.03 (m, 1H), 0.63 - 0.54 (m, 1H), 0.43 - 0.34 (m, 1H), 0.28-0.18 (m, 1H), 0.13 - 0.03 (m, 1H).

### Example 11 Preparation of Compound A13, Compound A13 hydrochloride and Compound A14, Compound A14 hydrochloride

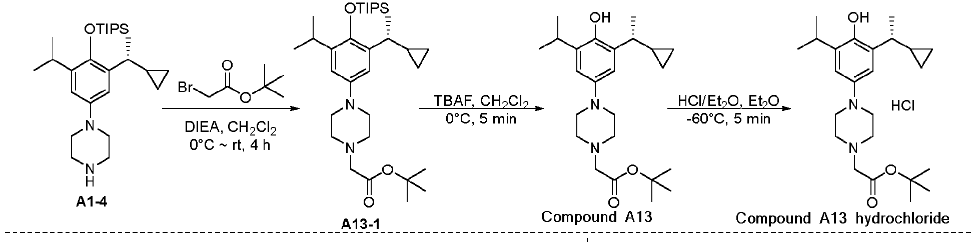

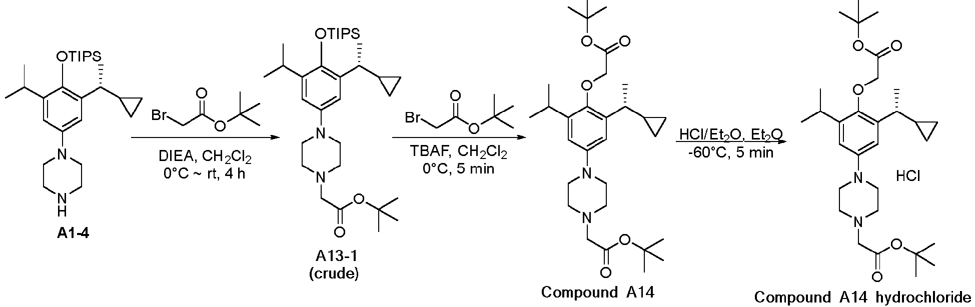

### 1. Preparation of Compound A13-1

At 0°C with an ice-salt bath, DIEA (1.293 g, 10.0 mmol) and tert-butyl bromoacetate (975.25 mg, 5.0 mmol) was sequentially added to a solution of A1-4 (444.78 mg, 1.0 mmol) in dichloromethane (20 mL), the mixture was stirred at room temperature for 4 hours. After confirming reaction completion by TLC (methanol/dichloromethane (v/v) = 1/10), water (20 mL) was added to the reaction system. The organic phase was extracted with dichloromethane (3 × 10 mL), dried over anhydrous Na₂SO₄, filtered and concentrated under reduced pressure to obtain a crude product. The crude product was purified by flash column chromatography (ethyl acetate/petroleum ether (v/v) = 0 ~ 15%). TLC (ethyl acetate/petroleum ether (v/v) = 1/8) monitoring was performed, and the fractions with Rf = 0.5 were collected to yield compound **A13-1** as colorless oil (512 mg, yield 91.6%). ESI[M + H]⁺ = 559.4.

### 2. Preparation of Compound A13

At 0°C with an ice-salt bath, TBAF (1.1 mL, 1 mol/L in THF, 1.1 mmol) was added to a solution of **A13-1** (512 mg, 0.92 mmol) in dry THF (10 mL), the mixture was stirred at 0°C for 5 minutes. After confirming reaction completion by TLC, water (20 mL) was added to the reaction system. The organic phase was extracted with ethyl acetate (3 × 10 mL), dried over anhydrous Na₂SO₄, filtered and concentrated under reduced pressure to obtain a crude product. The crude product was purified by flash column chromatography (ethyl acetate/petroleum ether (v/v) = 0 ~ 20%). TLC (ethyl acetate/petroleum ether (v/v) = 1/5) monitoring was performed, and the fractions with Rf = 0.5 were collected to yield **Compound A13** as colorless oil (360.0 mg, yield 97.2%). ESI[M + H]⁺=403.3.

### 3. Preparation of Compound A13 hydrochloride

At room temperature, A13 (360.0 mg, 0.89 mmol, 1.0 eq) was dissolved in diethyl ether (20 mL), and the reaction system was cooled to -60°C using a dry ice-ethanol bath, the solution of HCl/Et₂O (0.67 mL, 2 mol/L in Et₂O, 1.34 mmol) was slowly added to the reaction system, the mixture was stirred at -60°C for 5 minutes, and concentrated under reduced pressure at low temperature to obtain a crude product. Et₂O (20 mL) was added to the crude product, the mixture was then stirred and filtered, the solid was washed with Et₂O (3 × 20 mL). The solid was dissolved in ultrapure water (30 mL) and lyophilized for 24 hours to yield **Compound A13 hydrochloride** as white solid (395 mg, yield 100%). ESI[M + H]⁺ =403.3.
¹H NMR (400 MHz, CD₃OD) δ 7.19 (d, *J=* 2.8 Hz, 1H), 7.06 (d, *J=* 2.8 Hz, 1H), 4.17 (s, 2H), 3.75 - 3.58 (m, 8H), 3.35 - 3.32 (m, 1H), 2.54 - 2.41 (m, 1H), 1.55 (s, 9H), 1.27 (d, *J=* 6.9 Hz, 3H), 1.22 (d, *J=* 6.9 Hz, 6H), 1.11 - 1.01 (m, 1H), 0.62 - 0.54 (m, 1H), 0.43 - 0.34 (m, 1H), 0.25 - 0.18 (m, 1H), 0.12 - 0.05 (m, 1H).

### 4. Preparation of Compound A14

The preparation method of **A13-1** is similar to the first step of this example. A1-4 (444.78 g, 1.0 mmol) was reacted with tert-butyl bromoacetate, and followed by TBAF-mediated desilylation to yield A13-1. During the purification of **A13-1,** incomplete removal of residual tert-butyl bromoacetate led to further reaction of the remaining tert-butyl bromoacetate with the phenolic hydroxyl group under TBAF desilylation conditions, to yield compound **A14** (263.2 mg, two-step yield 50.9%).

### 5. Preparation of Compound A14 hydrochloride

At room temperature, **A14** (263.2 mg, 0.51 mmol, 1.0 eq) was dissolved in diethyl ether (20 mL), and the reaction system was cooled to -60°C using a dry ice-ethanol bath. HCl/Et₂O (0.38 mL, 2 mol/L in Et₂O, 0.76 mmol) was slowly added to the reaction system, the mixture was stirred at -60°C for 5 minutes, and concentrated under reduced pressure at low temperature to obtain a crude product, Et₂O (20 mL) was added to the crude product, the mixture was stirred and filtered, the solid was washed with Et₂O (3 × 20 mL). The solid was dissolved in ultrapure water (30 mL), and lyophilized for 24 hours to yield **Compound A14 hydrochloride** as white solid (241.9 mg, yield 100%). ESI[M + H]⁺ = 517.4.
¹H NMR (400 MHz, CD₃OD) δ 6.88 (d, *J=* 2.9 Hz, 1H), 6.78 (d, *J=* 2.9 Hz, 1H), 4.23 - 4.12 (m, 4H), 3.90 - 3.31 (m, 8H), 3.35 - 3.18 (m, 1H), 2.45 - 2.33 (m, 1H), 1.55 (s, 9H), 1.51 (s, 9H), 1.27 (d, *J=* 7.0 Hz, 3H), 1.22 (d, *J=* 6.9 Hz, 3H), 1.21 (d, *J=* 6.9 Hz, 3H), 1.05 - 0.93 (m, 1H), 0.61 - 0.51 (m, 1H), 0.38 - 0.29 (m, 1H), 0.26 - 0.17 (m, 1H), 0.17 - 0.08 (m, 1H).

### Example 12 Preparation of Compound A15 and Compound A15 Sulfate

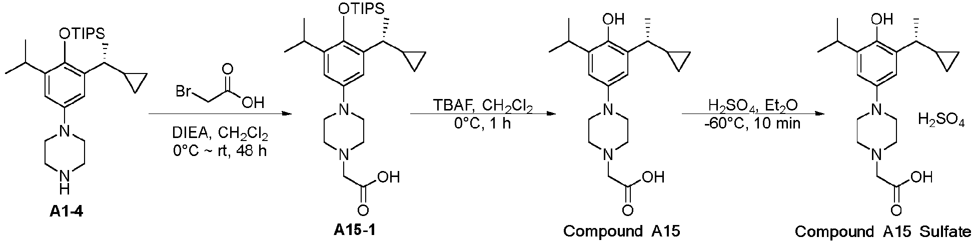

### 1. Preparation of Compound A15-1

At 0°C with an ice-salt bath, DIEA (360.54 mg, 2.81 mmol) and bromoacetic acid (130.27 mg, 0.94 mmol) was sequentially added to a solution of **A1-4** (417 g, 0.94 mmol) in dichloromethane (50 mL), the mixture was stirred at room temperature for 48 hours. After confirming reaction completion by TLC (methanol/dichloromethane (v/v) = 1/10), the reaction mixture was concentrated under reduced pressure to obtain a crude product. The crude product was purified by flash column chromatography (methanol/dichloromethane (v/v) = 0 ~ 30%) and Prep-TLC (methanol/dichloromethane (v/v) = 1/10). TLC (methanol/dichloromethane (v/v) = 1/10) monitoring was performed, and the fractions with Rf = 0.5 were collected to yield compound **A15-1** as colorless oil (209.9 mg, yield 44.4%).
ESI[M + H]⁺=503.4.

### 2. Preparation of Compound A15

At 0°C with an ice-salt bath, TBAF (0.8 mL, 1 mol/L in THF, 0.8 mmol) was added to a solution of **A15-1** (209.9 mg, 0.42 mmol) in dry THF (10 mL). The mixture was stirred at 0°C for 1 hour. After confirming reaction completion by TLC, the reaction mixture was concentrated under reduced pressure to obtain a crude product. The crude product was purified by flash column chromatography (methanol/dichloromethane (v/v) = 0~30%) and Prep-TLC (methanol/dichloromethane (v/v) = 1/10). TLC (methanol/dichloromethane (v/v) = 1/10) monitoring was performed and the fractions with Rf = 0.4 were collected to yield **Compound A15** as colorless oil (121.7 mg, yield 83.6%). ESI[M + H]⁺ = 347.3.

### 3. Preparation of Compound A15 Sulfate

At room temperature, **A15** (121.7 mg, 0.35 mmol, 1.0 eq) was dissolved in diethyl ether (10 mL), and the reaction system was cooled to -70°C using a dry ice-ethanol bath. The solution of H₂SO₄ (68.9 mg, 0.7 mmol, 2.0 eq) in Et₂O (2 mL) was slowly added to the reaction system, the mixture was stirred at -60°C for 10 minutes, and concentrated under reduced pressure at low temperature to obtain a crude product. Et₂O (10 mL) was added to the crude product, the mixture was then stirred and filtered, the solid was washed with Et₂O (3 × 10 mL). The solid was dissolved in ultrapure water (20 mL), and lyophilized for 24 hours to yield **Compound A15 Sulfate** as white solid (138.1 mg, yield 100%). ESI[M + H]⁺ = 347.3.
1H NMR (400 MHz, CD₃OD) δ 7.08 (d, *J=* 2.8 Hz, 1H), 6.95 (d, *J =* 2.8 Hz, 1H), 4.18 (s, 2H), 3.67 - 3.53 (m, 8H), 3.35 - 3.31 (m, 1H), 2.51 - 2.42 (m, 1H), 1.27 (d, *J=* 6.9 Hz, 3H), 1.22 (d, *J*= 6.8 Hz, 6H), 1.11 - 1.01 (m, 1H), 0.61 - 0.52 (m, 1H), 0.43 - 0.33 (m, 1H), 0.24 - 0.16 (m, 1H), 0.12 - 0.04 (m, 1H).

### Example 13 Preparation of Compounds A16 and Compound A16 Sulfate

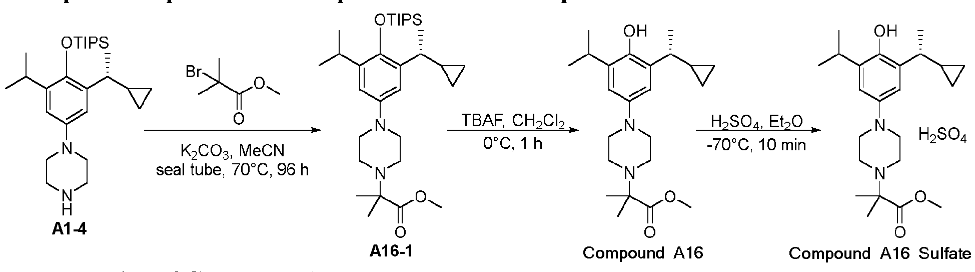

### 1. Preparation of Compound A16-1

At room temperature, K₂CO₃ (2.07 g, 15.0 mmol) and methyl 2-bromo-2-methylpropionate (1.81 g, 10.0 mmol) was sequentially added to a solution of **A1-4** (444.78 g, 1.0 mmol) in MeCN (10 mL), the mixture was stirred in a sealed tube at 70°C for 4 hours. After confirming reaction completion by TLC (methanol/dichloromethane (v/v) = 1/10), water (20 mL) was added to the reaction system. The organic phase was extracted with ethyl acetate (3 × 10 mL), dried over anhydrous Na₂SO₄, filtered and concentrated under reduced pressure to obtain a crude product. The crude product was purified by flash column chromatography (ethyl acetate/petroleum ether (v/v) = 0 ~ 15%). TLC (ethyl acetate/petroleum ether (v/v) = 1/8) monitoring was performed, and the fractions with Rf = 0.5 were collected to yield compound **A16-1** as colorless oil (253.0 mg, yield 46.4%). ESI[M + H]⁺=545.4.

### 2. Preparation of Compound A16

At 0°C with an ice-salt bath, TBAF (0.6 mL, 1 mol/L in THF, 0.6 mmol) was added to a solution of **A15-1** (253.0 mg, 0.46 mmol) in dry THF (10 mL), the mixture was stirred at 0°C for 1 hour. After confirming reaction completion by TLC, the reaction mixture was concentrated under reduced pressure to obtain a crude product. The crude product was purified by flash column chromatography (ethyl acetate/petroleum ether (v/v) = 0 ~ 20%). TLC (ethyl acetate/petroleum ether (v/v) = 1/8) monitoring was performed, and the fractions with Rf = 0.5 were collected to yield **Compound A16** as colorless oil (152.6 mg, yield 85.4%). ESI[M + H]⁺ = 389.2.

### 3. Preparation of Compound A16 Sulfate

At room temperature, **A16** (135.6 mg, 0.35 mmol, 1.0 eq) was dissolved in ether (10 mL), and the reaction system was cooled to -70°C using a dry ice-ethanol bath. The solution of H₂SO₄ (68.6 mg, 0.7 mmol, 2.0 eq) in Et₂O (2 mL) was slowly added to the reaction system, the mixture was stirred at -70°C for 10 minutes, and concentrated under reduced pressure at low temperature to obtain a crude product. Et₂O (20 mL) was added to the crude product, the mixture was then stirred and filtered, the solid was washed with Et₂O (3 × 10 mL). The solid was washed (20 mL), and lyophilized for 24 hours to yield **Compound A16 Sulfate** as white solid (189.1 mg, yield 100%). ESI[M + H]⁺ = 389.2.
¹H NMR (400MHz, CD₃OD) δ 7.23 (d, *J=* 2.9 Hz, 1H), 7.09 (d, *J=* 2.9 Hz, 1H), 3.88 (s, 3H), 3.78 - 3.67 (m, 4H), 3.64 - 3.51 (m, 4H), 3.37 - 3.32 (m, 1H), 2.54 - 2.39 (m, 1H), 1.66 (s, 6H), 1.28 (d, *J=* 6.9 Hz, 3H), 1.23 (d, *J=* 6.8 Hz, 6H), 1.12 - 1.02 (m, 1H), 0.64 - 0.54 (m, 1H), 0.43 - 0.34 (m, 1H), 0.26 - 0.18 (m, 1H), 0.12 - 0.03 (m, 1H).

### Example 14 Preparation of Compound A17 and Compound A17 hydrochloride

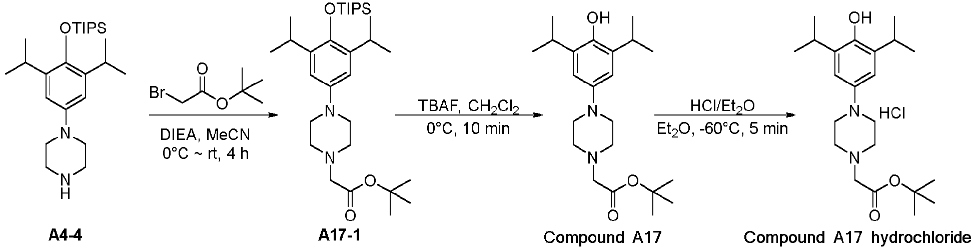

### 1. Preparation of Compound A17-1

At 0°C with an ice-salt bath, DIEA (932.7 mg, 7.2 mmol) and tert-butyl bromoacetate (702.2 mg, 3.6 mmol) was sequentially added to a solution of **A4-4** (300.0 mg, 0.72 mmol) in dichloromethane (20 mL), the mixture was stirred at room temperature for 4 hours. After confirming reaction completion by TLC (methanol/dichloromethane (v/v) = 1/10), water (20 mL) was added to the reaction system. The organic phase was extracted with dichloromethane (3 × 10 mL), dried over anhydrous Na₂SO₄, filtered and concentrated under reduced pressure to obtain a crude product. The crude product was purified by flash column chromatography (ethyl acetate/petroleum ether (v/v) = 0 ~ 15%). TLC (ethyl acetate/petroleum ether (v/v) = 1/8) monitoring was performed, and the fractions with Rf = 0.5 were collected to yield crude compound **A17-1** as colorless oil (405.6 mg). ESI[M + H]⁺ = 533.4.

### 2. Preparation of Compound A17

At 0°C with an ice-salt bath, TBAF (0.9 mL, 1 mol/L in THF, 0.9 mmol) was added to a solution of **A17-1** (405.6 mg, 0.76 mmol) in dry THF (10 mL), the mixture was stirred at 0°C for 10 minutes. After confirming reaction completion by TLC, water (20 mL) was added to the reaction system. The organic phase was extracted with ethyl acetate (3 × 10 mL), dried over anhydrous Na₂SO₄, filtered and concentrated under reduced pressure to obtain a crude product, the crude product was purified by flash column chromatography (ethyl acetate/petroleum ether (v/v) = 0 ~ 20%). TLC (ethyl acetate/petroleum ether (v/v) = 1/5) monitoring was performed, and the fractions with Rf = 0.5 were collected to yield **Compound A17** as colorless oil (188.8 mg, two-step yield 69.6%). ESI[M + H]⁺= 377.3.

### 3. Preparation of Compound A17 hydrochloride

At room temperature, **A17** (188.8 mg, 0.50 mmol, 1.0 eq) was dissolved in ether (20 mL), and the reaction system was cooled to -60°C using a dry ice-ethanol bath. HCl/Et₂O (0.38 mL, 2 mol/L in Et₂O, 0.76 mmol) was slowly added to the reaction system, the mixture was stirred at -60°C for 5 minutes, and concentrated under reduced pressure at low temperature to obtain a crude product. Et₂O (20 mL) was added to the crude product, the mixture was stirred and filtered, the solid was washed with Et₂O (3 × 20 mL). The solid was dissolved in ultrapure water (20 mL), and lyophilized for 24 hours to yield **Compound A17 hydrochloride** as white solid (211.6 mg, yield 100%). ESI[M + H]⁺ = 377.3.
¹H NMR (400MHz, CD₃OD) δ 6.94 (s, 2H), 4.12 (s, 2H), 3.62 - 3.52 (m, 8H), 3.36 - 3.32 (m, 2H), 1.55 (s,95H), 1.21 (d, *J=* 6.9 Hz, 12H).

### Example 15 Preparation of Compound A19 and Compound A19 Sulfate, Compound A19S and Compound A19S Sulfate, Compound A19R and Compound A19R Sulfate

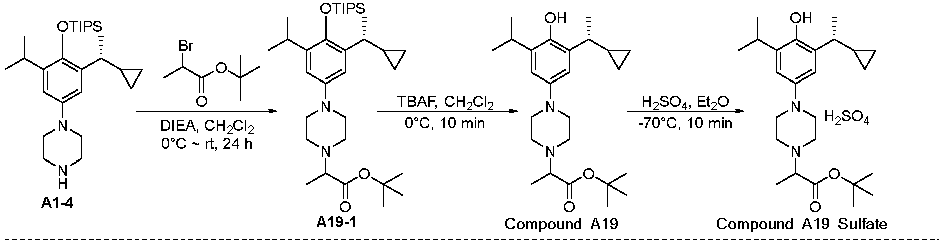

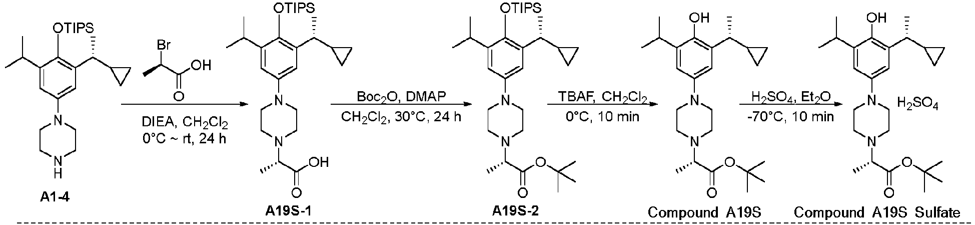

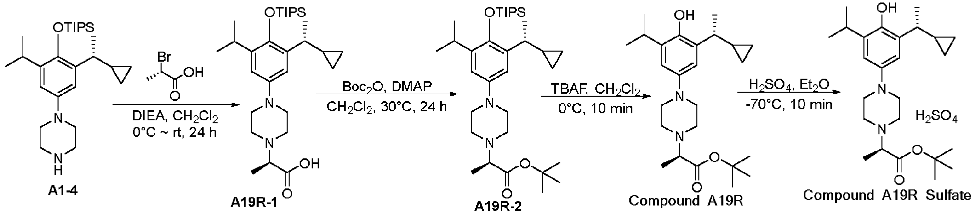

### 1. Preparation of Compound A19S-1

At room temperature, DIEA (122.3 mg, 0.944 mmol) and (R)-2-bromopropionic acid (144.4 mg, 0.944 mmol) was added to a solution of **A1-4** (420.0 mg, 0.944 mmol) in dichloromethane (15 mL). The mixture was stirred at room temperature for 24 hours. After confirming reaction completion by TLC (methanol/dichloromethane (v/v) = 1/10), the mixture was concentrated under reduced pressure at low temperature to obtain a crude product. The crude product was purified by flash column chromatography (methanol/dichloromethane (v/v) = 1/100 ~ 1/10). TLC (methanol/dichloromethane (v/v) = 1/10) monitoring was performed, and the fractions with Rf = 0.3 were collected to yield compound **A19S-1** as colorless oil (227.3 mg, yield 44.2%). ESI[M + H]⁺ = 516.4.

### 2. Preparation of Compound A19S-2

At room temperature, DMAP (23.0 mg, 0.19 mmol) and Boc2O (412 mg, 1.888 mmol) were added to a solution of **A19S-1** (187 mg, 0.364 mmol) in dichloromethane (10 mL), the mixture was stirred at room temperature for 24 hours. After confirming reaction completion by TLC (methanol/dichloromethane (v/v) = 1/10), water (30 mL) was added to the reaction mixture. The organic phase was extracted with dichloromethane (3 × 10 mL), dried over anhydrous Na₂SO₄, filtered and concentrated under reduced pressure to obtain a crude product. The crude product was purified by flash column chromatography (ethyl acetate/petroleum ether (v/v) = 0 ~ 15%). TLC (ethyl acetate/petroleum ether (v/v) = 1/10) monitoring was performed, and the fractions with Rf = 0.7 were collected to yield compound **A19S-2** as colorless oil (132 mg, yield 63.3%). ESI[M + H]⁺=573.4.

### 3. Preparation of Compound A19S

At room temperature, TBAF (0.3 mL, 1 mol/L in THF, 0.3 mmol) was added to a solution of **A19S-2** (132 mg, 0.23 mmol) in dry THF (8 mL), the mixture was stirred at 0°C for 10 minutes. After confirming reaction completion by TLC, water (20 mL) was added to the reaction system. The organic phase was extracted with ethyl acetate (3 × 10 mL), dried over anhydrous Na₂SO₄, filtered and concentrated under reduced pressure to obtain a crude product. The crude product was purified by Prep-TLC (ethyl acetate/petroleum ether (v/v) = 1/10), and the fractions with Rf = 0.5 were collected to yield **Compound A19S** as colorless oil (77 mg, yield 92.4%). ESI[M + H]⁺= 417.3.

### 4. Preparation of Compound A19S Sulfate

At room temperature, **A19S** (77 mg, 0.185 mmol, 1.0 eq) was dissolved in ether (10 mL), and the reaction system was cooled to -70°C using a dry ice-ethanol bath, solution of H₂SO₄ (36.3 mg, 0.37 mmol, 2.0 eq) in Et₂O (2 mL) was slowly added to the reaction system, the mixture was stirred at -70°C for 10 minutes, and concentrated under reduced pressure at low temperature to obtain a crude product. Et₂O (10 mL) was added to the crude product, the mixture was then stirred and filtered, the solid was washed with Et₂O (3 × 10 mL). The solid was dissolved in ultrapure water (20 mL), and lyophilized for 24 hours to yield **Compound A19S Sulfate** as white solid (83.3 mg, yield 100%). ESI[M + H]⁺ = 417.3.
¹H NMR (400MHz, CD₃OD) δ 7.16 (d, *J =* 2.9 Hz, 1H), 7.03 (d, *J =* 2.9 Hz, 1H), 4.13 (q, *J =* 7.2 Hz, 1H), 3.73 - 3.55 (m, 8H), 3.36 - 3.32 (m, 1H), 2.52 - 2.42 (m, 1H), 1.61 (d, *J =* 7.2 Hz, 3H), 1.55 (s, 9H), 1.27 (d, *J =* 6.9 Hz, 3H), 1.25 - 1.18 (m, 6H), 1.10 - 1.02 (m, 1H), 0.61 - 0.54 (m, 1H), 0.43 - 0.35 (m, 1H), 0.25 - 0.17 (m, 1H), 0.12 - 0.03 (m, 1H).

The preparation method of **Compound A19, Compound A19 Sulfate** and **Compound A19R, Compound A19R Sulfate** are similar to those for **A19S** and **A19S Sulfate.**

### Example 16 Preparation of Compound A20 and Compound A20 Sulfate, Compound A20S and Compound A20S Sulfate, Compound A20R and Compound A20R Sulfate

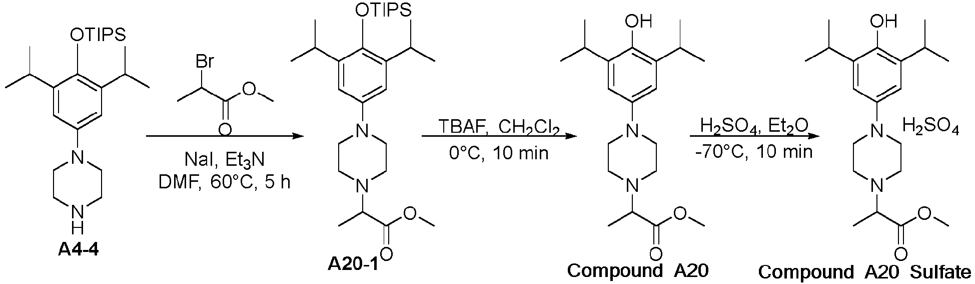

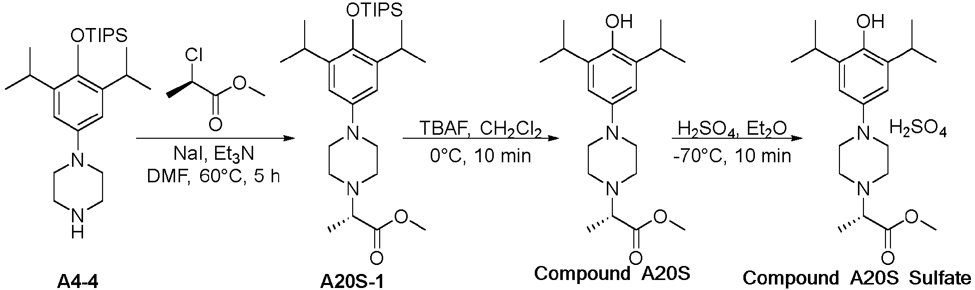

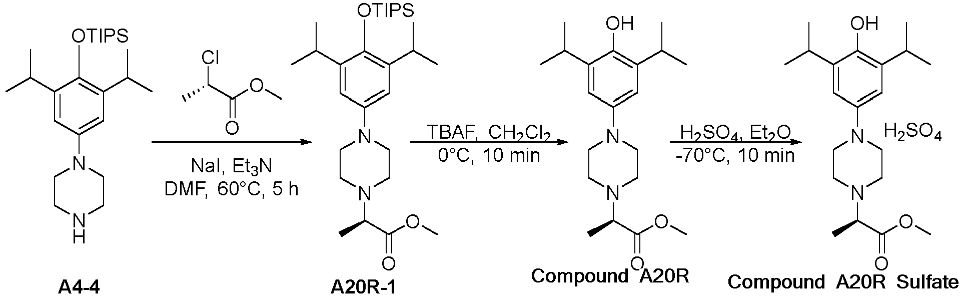

### 1. Preparation of Compound A20S-1

At room temperature, Et₃N (218.4 mg, 2.158 mmol, 3 eq), NaI (323.5 mg, 2.158 mmol, 3 eq), and methyl (S)-2-chloropropionate (264.5 mg, 2.158 mmol, 3 eq) was sequentially added to a solution of A4-4 (300 mg, 0.716 mmol, 1 eq) in dry DMF (10 mL), the mixture was stirred at 60°C for 5 hours. After confirming reaction completion by TLC (methanol/dichloromethane (v/v) = 1/10), the mixture was cooled to room temperature, water (30 mL) was added. The organic phase was extracted with ethyl acetate (3 × 10 mL), dried over anhydrous Na₂SO₄, filtered and concentrated under reduced pressure to obtain a crude product. The crude product was purified by flash column chromatography (ethyl acetate/petroleum ether (v/v) = 0 ~ 20%). TLC (ethyl acetate/petroleum ether (v/v) = 1/5) monitoring was performed, and the fractions with Rf = 0.6 were collected to yield compound **A20S-1** as colorless oil (331.7 mg, yield 91.8%). ESI [M + H]⁺ = 505.4.

### 2. Preparation of Compound A20S

At 0°C with an ice-salt bath, TBAF (0.8 mL, 1 mol/L in THF, 0.8 mmol, 1.2 eq) was added to a solution of **A20S-1** (331 mg, 0.656 mmol, 1 eq) in dry THF (20 mL), the mixture was stirred at 0°C for 10 minutes. After confirming reaction completion by TLC (ethyl acetate/petroleum ether (v/v) = 1/5), water (20 mL) was added to the reaction system. The organic phase was extracted with ethyl acetate (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 15%). TLC (ethyl acetate/petroleum ether (v/v) = 1/5) monitoring was performed, and the fractions with Rf = 0.5 were collected to yield **Compound A20S** as colorless oil (214 mg, yield 93.6%). ESI [M + H]⁺ = 349.2.

### 3. Preparation of Compound A20S Sulfate

At room temperature, **A20S** (214 mg, 0.614 mmol, 1.0 eq) was dissolved in ether (20 mL), and cool the reaction system to -70°C using a dry ice-ethanol bath, solution of H₂SO₄ (120.45 mg, 1.228 mmol, 2.0 eq) in Et₂O (2 mL) was slowly added to the system, the mixture was stirred at -70°C for 10 minutes, and concentrated under reduced pressure at low temperature to obtain a crude product. Et₂O (10 mL) was added to the crude product, the mixture was then stirred and filtered, the solid was washed with Et₂O (3 × 10 mL). The solid was dissolved in ultrapure water (20 mL), and lyophilized for 24 hours to yield **Compound A20S Sulfate** (315.3 mg, yield 100%) as white solid. ESI [M + H]⁺ = 349.2.
¹H NMR (400MHz, CD₃OD) δ 7.09 (s, 2H), 4.21 (q, *J =* 7.2 Hz, 1H), 3.86 (s, 3H), 3.74 - 3.66 (m, 4H), 3.65 - 3.49 (m, 4H), 3.38 - 3.32 (m, 2H), 1.61 (d, *J =* 7.2 Hz, 3H), 1.23 (d, *J =* 6.9 Hz, 12H).

The preparation methods of **Compound A20, Compound A20 Sulfate, Compound A20R, Compound A20R Sulfate** are similar to those for **Compound A20S and Compound A20S Sulfate.**

### Example 17 Preparation of Compound A21 and Compound A21 hydrochloride, Compound A21S and Compound A21S hydrochloride, Compound A21R and Compound A21R hydrochloride, Compound A27S and Compound A27S hydrochloride

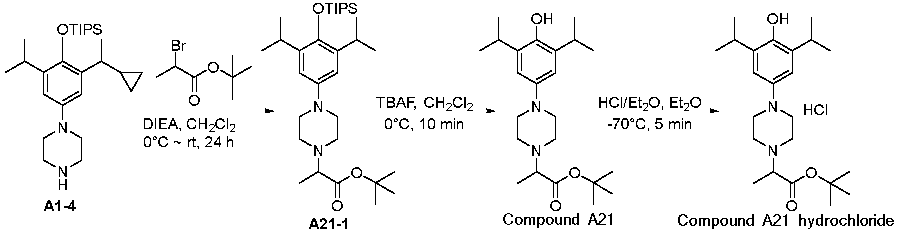

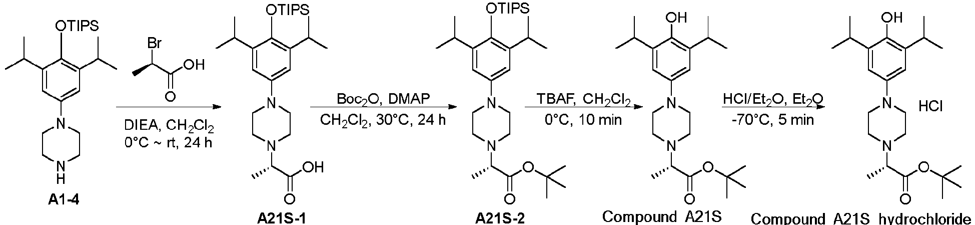

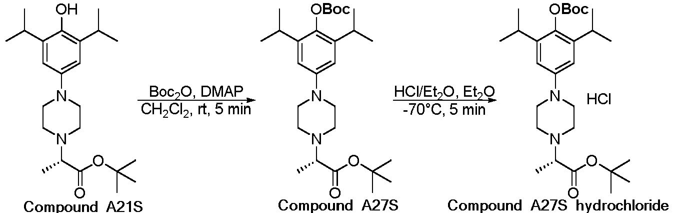

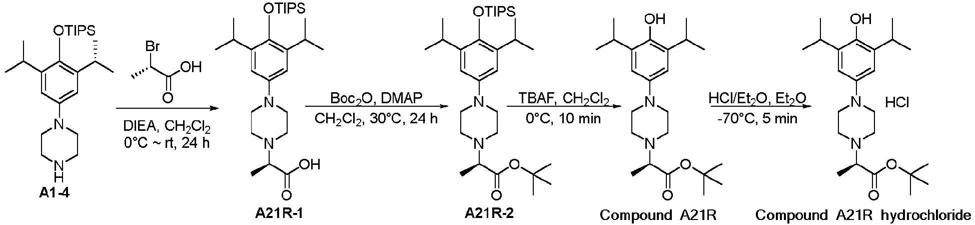

The preparation methods for **Compounds A21, A21S,** and **A21R** are similar to those described in **Example 15** (**Compounds A19, A19S,** and **A19R**)**.** The preparation methods for **A21 hydrochloride, A21S hydrochloride,** and **A21R hydrochloride** are similar to those for **A17 hydrochloride in Example 14.** Compound **A4-4** is used as the starting material.

During the isolation and purification of compound **A21S-2,** residual Boc₂O was not completely removed, resulting in the isolation of 75.1 mg of the compound **A27S** as colorless oily. The preparation method for **Compound A27S hydrochloride** is similar to that for **Compound A17 hydrochloride** in Example 14.

**A21S hydrochloride:** white solid, 75.6 mg. ESI [M + H]⁺ = 391.3.
¹H NMR (400MHz, CD₃OD) δ 7.03 (s, 2H), 4.14 (q, *J =* 7.2 Hz, 1H), 3.70 - 3.52 (m, 8H), 3.39 - 3.32 (m, 2H), 1.61 (d, *J* = 7.2 Hz, 3H), 1.55 (s, 9H), 1.22 (d, *J =* 6.9 Hz, 12H).

A27S **hydrochloride:** white solid, 64.9 mg. ESI [M + H]⁺ = 491.3.
¹H NMR (400MHz, CD₃OD) δ 6.79 (s, 2H), 4.29 - 3.94 (m, 1H), 3.93 - 3.03 (m, 8H), 3.01 - 2.92 (m, 2H), 1.64 - 1.56 (m, 3H), 1.55 (s, 9H), 1.52 (s, 9H), 1.19 (d, J = 6.9 Hz, 12H).

### Example 18 Preparation of Compound A22 and Compound A22 Sulfate, Compound A22S and Compound A22S Sulfate, Compound A22R and Compound A22R Sulfate

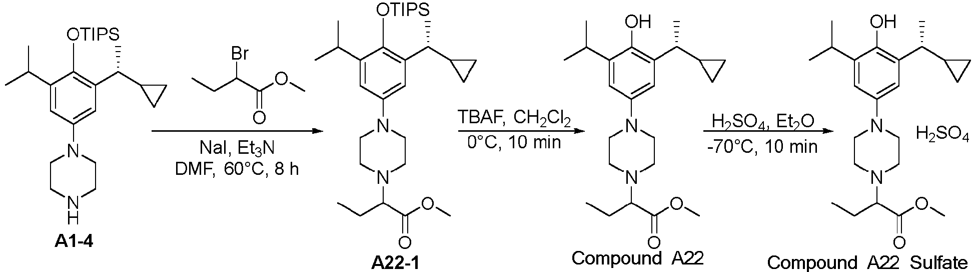

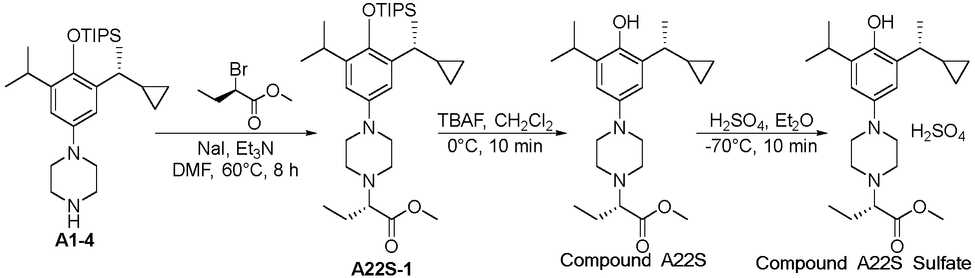

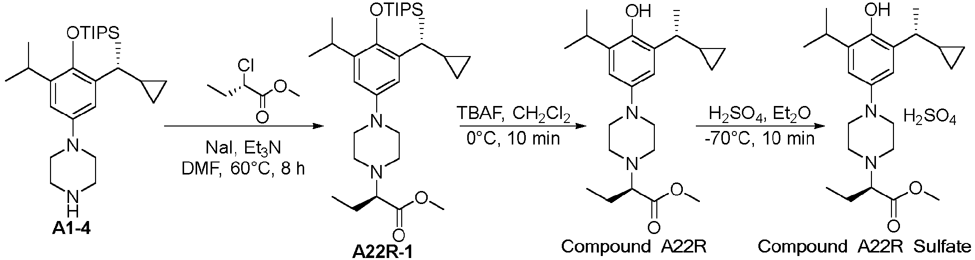

### 1. Preparation of Compound A22S-1

At room temperature, Et₃N (204.76 mg, 2.024 mmol, 3 eq), NaI (303.30 mg, 2.024 mmol, 3 eq), and methyl (R)-2-bromobutyrate (366.31 mg, 2.024 mmol, 3 eq) was sequentially added to a solution of A1-4 (300.0 mg, 0.675 mmol, 1 eq) in dry DMF (8 mL), the mixture was stirred at 60°C for 8 hours. After confirming reaction completion by TLC (methanol/dichloromethane (v/v) = 1/10), the mixture was cooled to room temperature, water (30 mL) was added. The organic phase was extracted with ethyl acetate (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 8%). TLC (ethyl acetate/petroleum ether (v/v) = 1/8) monitoring was performed, and the fractions with Rf = 0.6 were collected to yield compound **A22S-1** as colorless oil (296.0 mg, yield 80.5%). ESI [M + H]⁺ = 545.4.

### 2. Preparation of Compound A22S

At 0°C with an ice-salt bath, TBAF (0.7 mL, 1 mol/L in THF, 0.7 mmol) was added to a solution of **A22S-1** (296 mg, 0.543 mmol) in dry THF (8 mL), the mixture was stirred at 0°C for 10 minutes. After confirming reaction completion by TLC, water (20 mL) was added to the reaction system. The organic phase was extracted with ethyl acetate (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 7%). TLC (ethyl acetate/petroleum ether (v/v) = 1/5) monitoring was performed, and the fractions with Rf = 0.4 were collected to yield crude **Compound A22S** as colorless oil (247.86 mg). ESI [M + H]⁺ = 389.2.

### 3. Preparation of Compound A22S Sulfate

At room temperature, **A22S** (247.86 mg, 0.638 mmol, 1.0 eq) was dissolved in ether (20 mL), and the reaction system was cooled to -70°C using a dry ice-ethanol bath, solution of H₂SO₄ (125.12 mg, 1.276 mmol, 2.0 eq) in Et₂O (2 mL) was slowly added to the reaction system, the mixture was stirred at -70°C for 10 minutes, and concentrated under reduced pressure at low temperature to obtain a crude product. Et₂O (10 mL) was added to the crude product, the mixture was then stirred and filtered, the solid was washed with Et₂O (3 × 10 mL). The solid was dissolved in ultrapure water (20 mL), and lyophilized for 24 hours to yield **Compound A22S Sulfate** as white solid (278.28 mg, yield 100%). ESI [M + H]⁺ = 389.2.
¹H NMR (400MHz, CD₃OD) δ 7.30 (d, *J =* 2.9 Hz, 1H), 7.16 (d, *J =* 2.9 Hz, 1H), 3.84 (s, 3H), 3.73 - 3.68 (m, 4H), 3.65 - 3.60 (m, 1H), 3.49 - 3.42 (m, 4H), 3.36 - 3.32 (m, 1H), 2.54 - 2.43 (m, 1H), 2.02 - 1.93 (m, 2H), 1.28 (d, *J =* 6.9 Hz, 3H), 1.24 (d, *J =* 6.8 Hz, 6H), 1.09 (dd, *J =* 7.8, 2.9 Hz, 1H), 1.04 (t, *J =* 7.4 Hz, 3H), 0.64 - 0.56 (m, 1H), 0.44 - 0.36 (m, 1H), 0.27 - 0.19 (m, 1H), 0.12 - 0.03 (m, 1H).
The preparation methods for **Compound A22** and **Compound A22 sulfate,** as well as **Compound A22R** and **Compound A22R Sulfate,** are similar to those for **Compound A22S** and **Compound A22S Sulfate.**

### Example 19 Preparation of Compound A23 and Compound A23 Sulfate, Compound A23S and Compound A23S Sulfate, Compound A23R and Compound A23R Sulfate

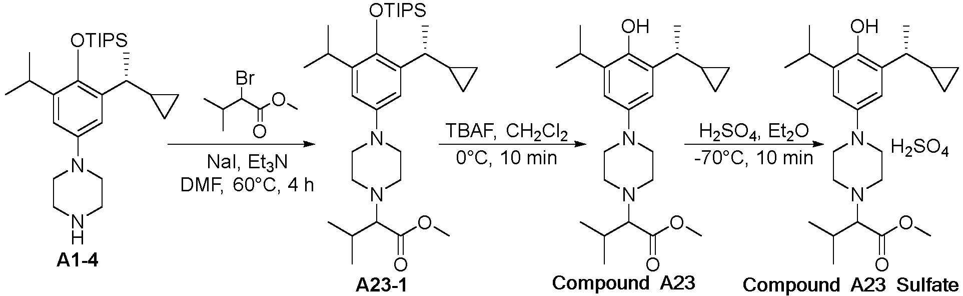

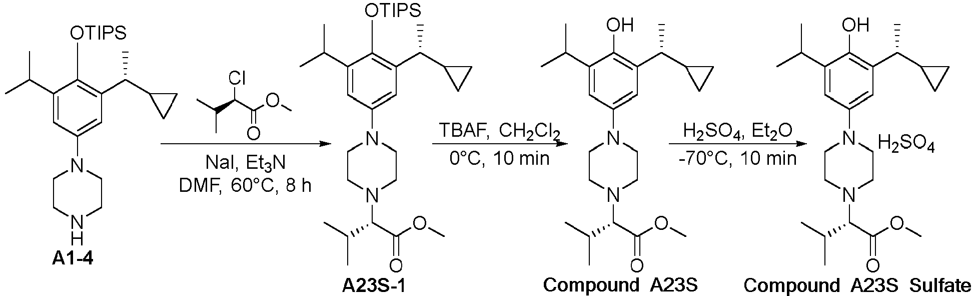

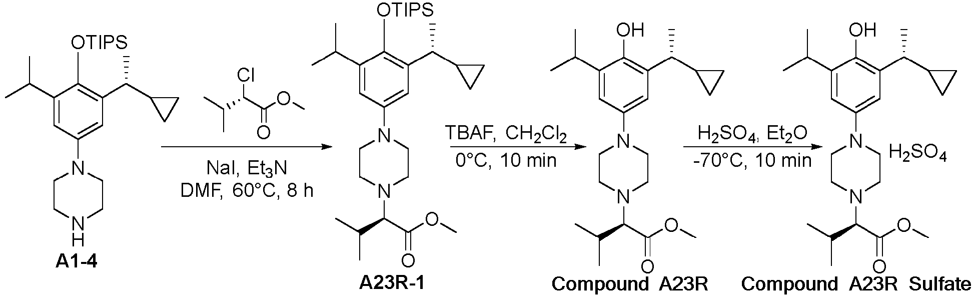

### 1. Preparation of Compound A23-1

At room temperature, Et₃N (184.27 mg, 1.821 mmol, 3 eq), NaI (272.95 mg, 1.821 mmol, 3 eq), and methyl 2-bromo-3-methylbutanoate (355.20 mg, 1.821 mmol, 3 eq) was sequentially added to a solution of A1-4 (270.0 mg, 0.607 mmol, 1 eq) in dry DMF (10 mL), the mixture was stirred at 60°C for 4 hours. After confirming reaction completion by TLC (methanol/dichloromethane (v/v) = 1/10), the mixture was cooled to room temperature, water (30 mL) was added. The organic phase was extracted with ethyl acetate (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 8%). TLC (ethyl acetate/petroleum ether (v/v) = 1/10) monitoring was performed, and the fractions with Rf = 0.6 were collected to yield compound **A23-1** as colorless oil (103.0 mg, yield 30.4%). ESI [M + H]⁺ = 559.4.

### 2. Preparation of Compound A23

At 0°C with an ice-salt bath, TBAF (0.25 mL, 1 mol/L in THF, 0.25 mmol) was added to a solution of **A23-1** (103.0 mg, 0.184 mmol) in dry THF (5 mL). The mixture was stirred at 0°C for 10 minutes. After confirming reaction completion by TLC, water (20 mL) was added to the reaction system. The organic phase was extracted with ethyl acetate (3 × 5 mL), wash with saturated brine, dried over anhydrous Na₂SO₄, filtered and concentrated under reduced pressure to obtain a crude product. The crude product was purified by flash column chromatography (ethyl acetate/petroleum ether (v/v) = 0 ~ 7%). TLC (ethyl acetate/petroleum ether (v/v) = 1/10) monitoring was performed, and the fractions with Rf = 0.3 were collected to yield **Compound A23** as colorless oil (46.1 mg, yield 62.2%). ESI [M + H]⁺ = 403.3.

### 3. Preparation of Compound A23 Sulfate

At room temperature, **A23** (46.1 mg, 0.115 mmol, 1.0 eq) was dissolved in ether (20 mL), and cool the reaction system to -70°C using a dry ice-ethanol bath, solution of H₂SO₄ (22.46 mg, 0.229 mmol, 2.0 eq) in Et₂O (2 mL) was slowly added to the reaction system, the mixture was stirred at -70°C for 10 minutes, and concentrated under reduced pressure at low temperature to obtain a crude product. Et₂O (10 mL) was added to the crude product, the mixture was then stirred and filtered, the solid was washed with Et₂O (3 × 10 mL). The solid was dissolved in ultrapure water (20 mL), and lyophilized for 24 hours to yield **Compound A23 Sulfate** as white solid (54.18 mg, yield 100%). ESI [M + H]⁺ = 403.3.
¹H NMR (400MHz, CD₃OD) δ 7.38 (d, *J =* 2.9 Hz, 1H), 7.24 (d, *J =* 2.9 Hz, 1H), 3.79 (s, 3H), 3.75 - 3.64 (m, 4H), 3.36 - 3.31 (m, 1H), 3.26 - 3.11 (m, 5H), 2.53 - 2.42 (m, 1H), 2.25 - 2.10 (m, 1H), 1.29 (d, *J =* 6.9 Hz, 3H), 1.24 (d, *J =* 6.9 Hz, 6H), 1.11 (d, *J =* 6.6 Hz, 3H), 1.09 - 1.03 (m, 1H), 0.95 (d, *J =* 6.6 Hz, 3H), 0.64 - 0.55 (m, 1H), 0.45 - 0.36 (m, 1H), 0.27 - 0.18 (m, 1H), 0.12 - 0.03 (m, 1H).
The preparation methods for **Compound A23** and **Compound A23 Sulfate,** as well as **A23R** and **Compound A23R Sulfate,** are similar to those for **Compound A23S** and **Compound A23S Sulfate.**

### Example 20 Preparation of Compound A24 and Compound A24 Sulfate, Compound A24S and Compound A24S Sulfate, Compounds A24R and Compound A24R Sulfate, Compound A26R and Compound A26R Sulfate

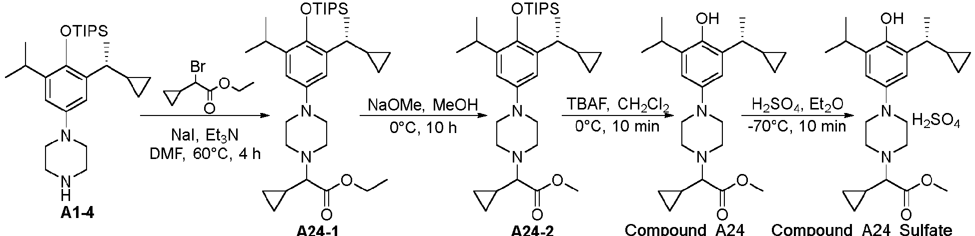

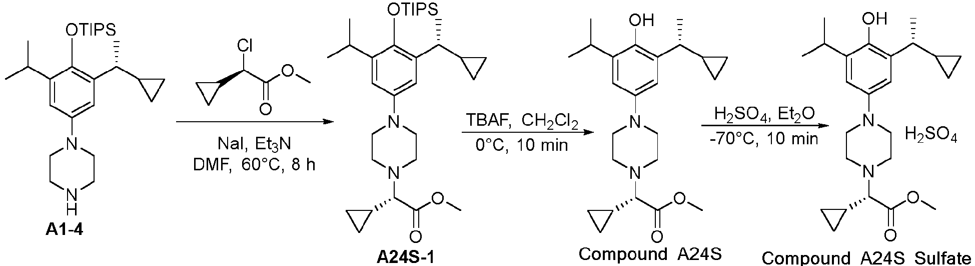

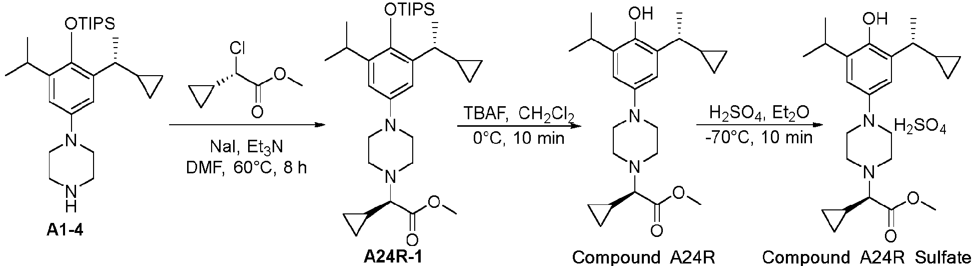

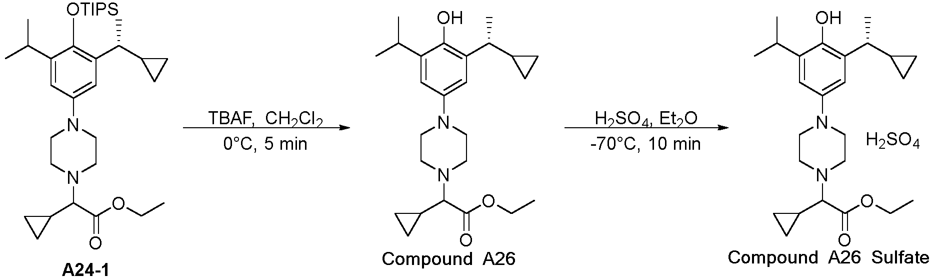

### 1. Preparation of Compound A24-1

At room temperature, Et₃N (341.6 mg, 3.376 mmol, 3 eq), NaI (337.0 mg, 2.248 mmol, 2 eq), and ethyl 2-bromo-2-cyclopropylacetate (465.5 mg, 2.248 mmol, 2 eq) was sequentially added to a solution of **A1-4** (500.0 mg, 1.125 mmol, 1 eq) in dry DMF (10 mL), the mixture was stirred at 60°C for 4 hours. After confirming reaction completion by TLC (methanol/dichloromethane (v/v) = 1/10), the mixture was cooled to room temperature, water (30 mL) was added. The organic phase was extracted with ethyl acetate (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 5%). TLC (ethyl acetate/petroleum ether (v/v) = 1/8) monitoring was performed, and the fractions with Rf = 0.5 were collected to yield compound **A24-1** as colorless oil (512.7 mg, yield 79.9%). ESI[M + H]⁺ = 571.4.

### 2. Preparation of Compound A24-2

At 0°C with an ice-salt bath, NaOMe (0.25 mL, 0.25 mmol) was added to a solution of **A24-1** (240.0 mg, 0.42 mmol) in MeOH (5 mL), the mixture was stirred at 0°C for 10 hours. After confirming reaction completion by TLC, the mixture was concentrated under reduced pressure to obtain a crude product. The crude product was purified by flash column chromatography (ethyl acetate/petroleum ether (v/v) = 0 ~ 7%). TLC (ethyl acetate/petroleum ether (v/v) = 1/8) monitoring was performed, and the fractions with Rf = 0.4 were collected to yield compound **A24-2** as colorless oil (43.2 mg, yield 18.5%). ESI[M + H]⁺ =557.4.

### 3. Preparation of Compound A24

At 0°C with an ice-salt bath, TBAF (0.1 mL, 1 mol/L in THF, 0.1 mmol) was added to solution of **A24-2** (43.2 mg, 0.078 mmol) in dry THF (5 mL). The mixture was stirred 0°C for 10 minutes. After confirming reaction completion by TLC, water (20 mL) was added to the reaction system. The organic phase was extracted with ethyl acetate (3 × 10 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 7%). TLC (ethyl acetate/petroleum ether (v/v) = 1/10) momnitoring was performed, and the fractions with Rf = 0.3 were collected to yield **Compound A24** as colorless oil (21.0 mg, yield 67.2%). ESI[M + H]⁺ = 401.3.

### 4. Preparation of Compound A24 Sulfate

At room temperature, **A24** (21.0 mg, 0.0524 mmol, 1.0 eq) was dissolved in ether (10 mL), reaction system was cooled to -70°C using a dry ice-ethanol bath. Solution of H₂SO₄ (10.30 mg, 0.105 mmol, 2.0 eq) in Et₂O (1 mL) was slowly added to the reaction system, the mixture was stirred at -70°C for 10 minutes, and concentrated under reduced pressure at low temperature to obtain a crude product. Et₂O (10 mL) was added to the crude product, the mixture was then stirred and filtered, the solid was washed with Et₂O (3 × 10 mL). The solid was dissolved in ultrapure water (20 mL), and lyophilized for 24 hours to yield **Compound A24 Sulfate** as white solid (26.0 mg, yield 100%). ESI[M + H]⁺ = 401.3.
¹H NMR (400MHz, CD₃OD) δ 7.17 (d, *J =* 2.9 Hz, 1H), 7.04 (d, *J =* 2.9 Hz, 1H), 3.88 (s, 3H), 3.75 - 3.62 (m, 6H), 3.57 - 3.49 (m, 2H), 3.40 (d, *J =* 10.2 Hz, 1H), 3.35 - 3.32 (m, 1H), 2.52 - 2.43 (m, 1H), 1.27 (d, *J* = 7.0 Hz, 3H), 1.23 (d, *J =* 6.9 Hz, 3H), 1.22 (d, *J =* 6.9 Hz, 3H), 1.21 - 1.17 (m, 1H), 1.12 - 1.01 (m, 1H), 1.01 - 0.91 (m, 1H), 0.86 - 0.77 (m, 1H), 0.76 - 0.67 (m, 1H), 0.67 - 0.53 (m, 2H), 0.43 - 0.34 (m,1H), 0.25 - 0.18 (m, 1H), 0.12 - 0.04 (m, 1H).
The preparation methods for **Compound A24S** and **Compound A24S Sulfate,** as well as **Compound A24R** and **Compound A24R Sulfate,** are similar to those for **Compound A24** and

### Compound A24 Sulfate.

### 5. Preparation of Compound A26 and Compound A26 Sulfate

At 0°C with an ice-salt bath, TBAF (0.5 mL, 1 mol/L in THF, 0.5 mmol) was added to solution of **A24-1** (232.3 mg, 0.407 mmol) in dry THF (5 mL), the mixture was stirred at 0°C for 5 minutes. After confirming reaction completion by TLC, water (10 mL) was added. The organic phase was extracted with ethyl acetate (3 × 5 mL), washed with saturated brine (20 mL), dried over anhydrous Na₂SO₄, filtered and concentrated under reduced pressure to obtain a crude product. The crude product was purified by flash column chromatography (ethyl acetate/petroleum ether (v/v) = 0 ~ 8%). TLC (ethyl acetate/petroleum ether (v/v) = 1/10) monitoring was performed, and the fractions with Rf = 0.3 were collected to yield **Compound A26** as colorless oil (160.0 mg, yield 94.8%). ESI[M + H]⁺ = 415.2.

At room temperature, **Compound A26** (160.0 mg, 0.386 mmol, 1.0 eq) was dissolved in ether (10 mL), the reaction system was cooled to -70°C using a dry ice-ethanol bath. Solution of H₂SO₄ (75.70 mg, 0.772 mmol, 2.0 eq) in Et₂O (1 mL) was slowly added to the reaction system, the mixture was stirred at -70°C for 10 minutes. Et₂O (10 mL) was added to the crude product, the mixture was then stirred and filtered, the solid was washed with Et₂O (3 × 10 mL). The solid was dissolved in ultrapure water (20 mL), and lyophilized for 24 hours to yield **Compound A26 Sulfate** as white solid (220.59 mg, yield 100%). ESI[M + H]⁺ = 415.2.
¹H NMR (400MHz, CD₃OD) δ 7.23 (d, *J =* 2.9 Hz, 1H), 7.09 (d, *J =* 2.9 Hz, 1H), 4.44 - 4.26 (m, 2H), 3.88 - 3.76 (m, 2H), 3.76 - 3.69 (m, 4H), 3.66 - 3.57 (m, 2H), 3.47 (d, *J =* 10.2 Hz, 1H), 3.37 - 3.31 (m, 1H), 2.53 - 2.42 (m, 1H), 1.35 (t, *J =* 7.1 Hz, 3H), 1.28 (d, *J =* 6.9 Hz, 3H), 1.23 (d, *J =* 6.9 Hz, 6H), 1.22 - 1.19 (m, 1H), 1.14 - 1.03 (m, 1H), 1.02 - 0.93 (m, 1H), 0.89 - 0.80 (m, 1H), 0.81 - 0.72 (m, 1H), 0.71 - 0.63 (m, 1H), 0.63 - 0.53 (m, 1H), 0.43 - 0.34 (m, 1H), 0.26 - 0.17 (m, 1H), 0.12 - 0.03 (m, 1H).

### Example 21 Preparation of Compound A25S

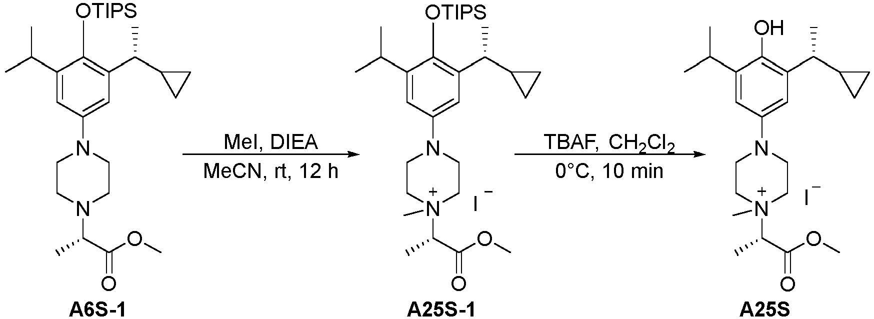

At room temperature, **A6S-1** (200 mg, 0.377 mmol), MeI (106.95 mg, 0.753 mmol, 2 eq), and DIEA (146.4 mg, 1.133 mmol) was sequentially dissolved to the dry DMF (10 mL), and the mixture was stirred at room temperature for 12 hours. After confirming reaction completion by TLC (ethyl acetate/petroleum ether (v/v) = 1/10), concentrated under reduced pressure to obtain a crude product. The crude product was purified by flash column chromatography (methanol/dichloromethane (v/v) = 0 ~ 50%). TLC (methanol/dichloromethane (v/v) = 1/10) monitoring was performed, and the fractions with Rf = 0.5 were collected to yield crude compound **A25S-1** (301 mg).

At 0°C with an ice-salt bath, TBAF (0.1 mL, 1 mol/L in THF, 0.1 mmol) was added to solution of the crude compound **A25S-1** (300 mg) in dry THF (5 mL). The mixture was stirred at 0°C for 10 minutes. After confirming reaction completion by TLC, water (10 mL) was added. The organic phase was extracted with ethyl acetate (3 × 10 mL), washed with saturated brine (20 mL), dried over anhydrous Na₂SO₄, filtered and concentrated under reduced pressure to obtain a crude product. The crude product was purified by flash column chromatography (methanol/dichloromethane (v/v) = 0 ~ 50%), TLC (methanol/dichloromethane (v/v) = 1/10) monitoring was performed, and the fractions with Rf = 0.5 were collected to yield compound **A25S** as colorless oil (70.7 mg, two-step yield 36.3%). ESI[M - I] = 389.2.
¹H NMR (400MHz, CD₃OD) δ 7.48 (d, *J =* 3.3 Hz, 1H), 7.34 (d, *J =* 3.3 Hz, 1H), 4.46 - 4.32 (m, 2H), 3.97 - 3.85 (m, 2H), 3.60 (s, 3H), 3.52 - 3.47 (m, 1H), 3.45 (s, 3H), 3.37 (m, 1H), 3.12 (m, 2H), 3.04 - 2.82 (m, 2H), 2.58 - 2.39 (m, 1H), 1.31 (m, 6H), 1.27 - 1.24 (m, 6H), 1.12 (m, 1H), 0.66 - 0.57 (m, 1H), 0.42 (m, 1H), 0.25 (m, 1H), 0.07 (m, 1H).
The following examples illustrate the beneficial effects of the present invention:

### Example 1 Salt Type Screening and Solubility Determination Test of Compound A6S of the Present Invention

### 1. Experiment method

**Compound A6S** (1 eq) was dissolved in Et₂O (2mL) at room temperature, and the corresponding acid (2 eq) was dissolved in Et₂O (NO. 1- NO.4) or MeOH (NO.5- NO.10). The above acid solution was slowly added to the reaction system containing **Compound A6S** using a syringe. NO.1- NO.3 was mixed and stirred at room temperature for 1 hour, and NO.5- NO.10 was mixed and stirred at room temperature for 16 hours. The mixture was filtered under suction to get the solid, then the solid was washed with Et₂O, and dried to obtain the corresponding salt form of **Compound A6S.** Then, the solubility of each salt form was tested in ultrapure water.

**Table 1. Screening and Solubility of Salt Forms of Compound A6S of the Present Invention**

| **NO.** | **A6S (mg)** | **Acid** | **The dosage of acid** | **Salt formation (Yes/No)** | **Solubility (ultrapure water)** |
|---|---|---|---|---|---|
| **1** | 17.1 | Hydrochloric acid | 0.09 mL (2M in Et₂O) | Yes | >30 mg/mL |
| **2** | 17.8 | Sulfuric acid | 9.32 mg | Yes | >200 mg/mL |
| **3** | 17.1 | Methanesulfonic acid | 8.78 mg | Yes | >10 mg/mL |
| **4** | 15.5 | Phosphoric acid | 8.11 mg | Yes | >8 mg/mL |
| **5** | 18.2 | p-Toluenesulfonic acid hydrate | 18.49 mg | Yes | >10 mg/mL |
| **6** | 17.6 | Benzenesulfonic acid | 14.87 mg | Yes | > 10mg/mL |
| 7 | 18.9 | Oxalic acid dihydrate | 12.72 mg | Yes | >10 mg/mL |
| **8** | 20.28 | Maleic acid | 96.91 mg | Yes | >10 mg/mL |

### 2. Experiment result

As can be seen in Table 1, the **Compound A6S** of the present invention can form the various types of salts, including hydrochloride, sulfate, methanesulfonate, phosphate, p-toluenesulfonate, benzenesulfonate, oxalate, and maleate. Moreover, each salt form exhibits excellent water solubility, with solubility in ultrapure water exceeding 8 mg/mL, meeting the requirements for formulation.

### Example 2 Solubility Determination of Salt Forms of the Compound in the Present Invention

### 1. Experiment method

A weighed amount of 10 mg of the compound's salt form was placed in a vessel at 25°C ± 2°C. Ultrapure water was added incrementally. The mixture was vigorously shaken for 30 seconds every 5 minutes and observed for 30 minutes. When no undissolved particles or droplets remained in the test system, the water solubility of the salt form was recorded according to the following criteria:
A: Water volume used is less than 0.1 mL;
B: Water volume used is greater than or equal to 0.1 mL and less than 1.0 mL;
C: Water volume used is greater than or equal to 1.0 mL and less than 5.0 mL.

### 2. Experiment result

**Table 2. Solubility of Salt Forms of Compounds in the Present Invention**

| **Compound** | **Compound Salt** | **Solubility** |
|---|---|---|
| A1 | Hydrochloride | A |
| A2 | Hydrochloride | A |
| A3 | Hydrochloride | C |
| A4 | Hydrochloride | A |
| A5 | Hydrochloride | A |
| A6 | Hydrochloride | A |
| A6R | Sulfate | A |
| A6S | Sulfate | A |
| A7 | Hydrochloride | B |
| A8 | Hydrovhloride | B |
| A11 | Sulfate | A |
| A12 | Sulfate | A |
| A13 | Hydrochloride | A |
| A14 | Hydrochloride | C |
| A15 | Sulfate | A |
| A16 | Sulfate | A |
| A17 | Hydrochloride | C |
| A18 | Sulfate | A |
| A19S | Sulfate | A |
| A20S | Sulfate | A |
| A21S | Hydrochloride | C |
| A22S | Sulfate | A |
| A23 | Sulfate | C |
| A24 | Sulfate | C |
| A26 | Sulfate | A |
| A27S | Hydrochloride | C |

It is well-known to those skilled in the art, propofol and ciprofol can not form salts and are insoluble in water. However, as can be seen in Table 2, the representative salt forms of the compound of the present invention have excellent water-solubility. There is no need to prepare the compounds of the present invention into lipid emulsions, meanwhile, the compounds of the present invention can overcome various adverse reactions caused by lipid emulsions.

### Example 3 Determination of Pharmacological Data and Safety Data for Compounds of the Present Invention

### 1. Experiment method

(1) Determination of the anesthetic effect (determination of the minimum effective anesthetic dose) of the representative compound of the present invention in rats via tail vein injection:
   SD rats were used, with a single intravenous injection via the tail vein (administration rate of 0.02 ml/s and administration volume of 0.6 ml per rat) in the experiment. The initial dose of the test compound started at1 mg/kg, and the actual administration dose was calculated based on the body weight measured before the experiment for each animal. The increase or decrease in subsequent doses was determined based on the results of the experimental animals (whether the LORR (Loss of righting reflex) occureed). The dose at which the LORR first occur was determined as the minimum effective anesthetic dose. The minimum effective anesthetic dose is further classified as: A ≤ 5 mg/kg; 5 mg/kg < B ≤ 10 mg/kg; 10 mg/kg < C ≤ 20 mg/kg; D > 20 mg/kg in this invention.
(2) Determination of the anesthetic effect (determination of median effective dose, ED₅₀) and the pharmacological characteristics of equivalent doses of the representative compound of the present invention in rats via tail vein injection:
   SD rats were used, with a single intravenous injection via the tail vein (administration rate of 0.02 ml/s and administration volume of 0.6 ml per rat) in the experiment. The Up and down method was used to determine the ED₅₀ of the compound causing the LORR. The initial dose of the test compound started at 5 mg/kg, and the actual administration dose was calculated based on the body weight measured before the test for each animal. The increase or decrease in subsequent doses was determined based on the results of the experimental animals (whether the LORR occurred). After observing five consecutive consistent directional changes in LORR occurrence and recovery, the ED₅₀ was calculated using the Dixon-Mood formula. After determining the ED₅₀, the onset time, duration of anesthesia, and emergence time of anesthesia were determined at an injection dose of 2ED₅₀.
(3) Determination of the effects on blood pressure, heart rate, and respiratory rate in rats of the representative compounds of the present invention via tail vein injection at a injection dose of 2ED₅₀:
   According to the ED₅₀ of the compounds, the SD rats were still selected, to be catheterized in their tail artery, and a single injection of 2ED₅₀ dose was given to the tail vein (with an administration rate of 0.02 ml/s and an administration volume of 0.6 ml per rat). Arterial blood pressure, heart rate, and respiratory rate of the rats were continuously monitored during the whole course of the expriment. The effects of the compounds on blood pressure, heart rate and respiratory rate of rats at 2ED₅₀ dose were observed.
(4) Determination of the minimum lethal dose of the compound of the present invention in rats via tail vein injection:
   SD rats were used, with a single intravenous injection via the tail vein (administration rate of 0.02 ml/s and administration volume of of 0.5-1.0 ml per 100 g body weight) in the experiment. The initial dose of the test compound started at 4ED₅₀, and the actual administration dose was calculated based on the body weight measured before the test for each animal. The increase or decrease in subsequent doses was determined based on the results of the experimental animals (whether death occurred). The dose at which death first occurred was determined as the minimum lethal dose. In the present invention, the ratio of the minimum lethal dose to the minimum effective anesthetic dose is defined as the approximate therapeutic index. The approximate therapeutic index is further classified as: A when the index is ≥5; B when the index is <5 but ≥3; and C when the index is <3.
(5) Determination of the drug withdrawal recovery time of the compound of the present invention after stopping administration following continuous infusion for different infusion duration in rats:
   SD rats were used, with a single intravenous injection of 1.5ED₅₀ via the tail vein, followed by another 1ED₅₀ dose after 1 minute. After administration, the tail vein catheter was connected to a syringe on the infusion pump via an extension tube. The initial infusion rate for each rat was determined based on the specific compound, and the infusion rate was halved after 5 minutes, with adjustments made every 5 minutes thereafter based on the rat's response. When the predetermined infusion time was reached, the infusion was stopped, and the time to recovery of LORR, as well as the time and type of adverse reactions observed during the test were recorded.

In the above experiments, in addition to recording the dose at which the LORR occurred, the onset time and emergence time of anesthesia, the duration of the LORR, and the duration of sedative effects could also be recorded. Meanwhile, circulatory effects experiments can be added to observe the effects of the compound on circulation and respiration in rats.

### 2. Test result

**Table 3. Pharmacological Data of a Single Intravenous Injection of the Salt Form of the Compound of the Present Invention**

| **Compound** | **Tested salt form** | **Vehicle for animal testing** | **Minimal effective anesthetic dose(mg/kg)** | **Onset time (min)** | **Righting Reflex Duration (min)** | **Sedation Duration (min)** |
|---|---|---|---|---|---|---|
| A1 | Hydrochloride | 0.9% physiological saline | B | 0.22-0.83 | 0.58-1.57 | 8.93-16.45 |
| A2 | Hydrochloride | 0.9% physiological saline | C | 0.22-0.23 | 0.52-1.03 | 12.73-13.92 |
| A3 | Hydrochloride | 31.8% DMSO | C | 0.37-0.52 | 1.02-2.08 | 6.65-8.50 |
| A4 | Hydrochloride | 0.9% physiological saline | C | 0.17-0.25 | 0.50-0.70 | 8.27-18.48 |
| A5 | Hydrochloride | 0.9% physiological saline | A | 0.13-0.37 | 0.60-2.13 | 14.80-16.37 |
| A6 | Hydrochloride | 0.9% physiological saline | A | 0.18-0.27 | 0.53-2.65 | 14.90-22.30 |
| A6R | Sulfate | Pure water | A | 0.15-0.70 | 1.72-3.82 | 7.02-14.77 |
| A6S | Sulfate | 0.9% physiological saline | B | 0.17-0.35 | 0.57-2.13 | 9.62-20.72 |
| A6S | Sulfate | Pure water | B | 0.47-0.73 | 1.08-5.67 | 7.93-15.12 |
| A7 | Hydrochloride | 0.9% physiological saline | D | / | / | / |
| A8 | Hydrochloride | 0.9% physiological saline | D | / | / | / |
| A11 | Sulfate | Pure water | C | 0.12-0.27 | 0.68-3.12 | 12.05-14.53 |
| A12 | Sulfate | Pure water | C | 0.50-0.77 | 1.22-7.40 | 9.90-34.25 |
| A13 | Hydrochloride | 0.9% physiological saline | A | 0.17-0.25 | 2.75-21.13 | 18.20-69.72 |
| A14 | Hydrochloride | 66.7% DMSO | C | 0.18-1.63 | 1.95-11.67 | 19.40-28.45 |
| A15 | Sulfate | Pure water | D | / | / | / |
| A16 | Sulfate | 0.9% physiological saline | B | 0.13-0.18 | 7.32-24.47 | 34.35-64.98 |
| A17 | Hydrochloride | 79.2% DMSO | A | 0.15-0.63 | 3.67-9.38 | 24.42-35.32 |
| A18 | Sulfate | Pure water | B | 0.23-0.98 | 3.02-19.25 | 16.47-35.57 |
| A19S | Sulfate | Pure water | B | 0.70-0.98 | 2.21-5.57 | 14.55-21.30 |
| A20S | Sulfate | Pure water | B | 0.18-0.43 | 2.18-7.48 | 15.75-23.72 |
| A21S | Hydrochloride | 66.7% DMSO | B | 0.25-0.42 | 12.02-22.80 | 28.72-56.33 |
| A23 | Sulfate | 60% DMSO | B | 0.20-0.22 | 4.93-15.08 | 30.48-39.13 |
| A24 | Sulfate | 82.8% DMSO | B | 0.17-0.18 | 5.53-9.67 | 19.33-38.80 |
| A25S | Quaternary ammonium | 70% DMSO | D | / | / | / |
| A26 | Sulfate | Pure water | A | 0.42-0.43 | 7.03-10.48 | 14.75-21.28 |

As can be seen from Table 3, most of the salt forms selected in the expriment of representative compounds of the present invention can be directly dissolved in pure water or normal saline, with low minimum effective anesthetic dose, short onset time, and rapid general anesthetic effect.

**Table 4. Safety Data of the Compounds of the Present Invention.**

| **Compound** | **Tested salt form** | **Vehicle for animal testing** | **Approximate therapeutic index** | **The impact of equivalent anesthetic doses on circulation** | **The impact of equivalent anesthetic doses on respiration** | **Recovery after continuous infusion** |
|---|---|---|---|---|---|---|
| Propofol | / | Lipid emulsion | B | Significant inhibitory effect | Significant inhibitory effect | Long, dose-depen dent |
| A1 | Hydrochloride | 0.9% physiological saline | A | Have an inhibitory effect, but it is lighter than propofol | Have an inhibitory effect, but it is lighter than propofol | Short, dose-depen dent and superior to propofol. |
| A2 | Hydrochloride | 0.9% physiological saline | B | | | |
| A3 | Hydrochloride | 31.8%DMSO | B | | | |
| A4 | Hydrochloride | 0.9% physiological saline | B | Have an inhibitory effect, but it is lighter than propofol | Have an inhibitory effect, but it is lighter than propofol | |
| A5 | Hydrochloride | 0.9% physiological saline | B | | | |
| A6 | Hydrochloride | 0.9% physiological saline | B | | | |
| A6R | Sulfate | Pure water | A | Have an inhibitory effect, but it is lighter than propofol | Have an inhibitory effect, but it is lighter than propofol | Short, dose-depen dent and superior to propofol. |
| A6S | Sulfate | 0.9% physiological saline | B | | | Long, dose-depen dent and similar to propofol. |
| A11 | Sulfate | Pure water | B | | | |
| A12 | Sulfate | Pure water | A | | | |
| A13 | Hydrochloride | 0.9% physiological saline | A | | | |
| A17 | Hydrochloride | 79.2%DMSO | A | | | |
| A18 | Sulfate | Pure water | B | | | |
| A20S | Sulfate | Pure water | B | | | |

As can be seen from Table 4, the representative compounds of the present invention demonstrates not only rapid inducetion of general anesthesia and allow for quick recovery but also exhibit better safety profiles compared to propofol, including lighter cardiovascular depression and respiratory suppression. During continuous infusion, these compounds demonstrate shorter anesthesia recovery times and improved safety.

As can be seen from Tables 3 and 4, the representative compounds of the present invention exhibit excellent anesthetic efficacy and/or superior safety profiles, indicating broad prospects for clinical application.

In summary, the present invention provides a piperazine substituted phenol derivative represented by formula I. This derivative not only exhibits excellent salt-forming properties and excellent water solubility, meeting the requirements for formulation, but also demonstrates a low minimum effective anesthetic dose, enabling rapid onset and recovery. It overcomes the drawbacks of propofol fat emulsions and the limitations of water-soluble prodrugs of propofol. Additionally, it has better safety compared with propofol. The compound has broad application prospects in preparation of drugs with sedative, hypnotic, and/or anesthetic effects, and drugs for controlling status epilepticus. It provides a new option for the preparation of clinical drugs with sedative, hypnotic, and/or anesthetic effects, and for controlling status epilepticus.

## Claims

1. A compound of formula I, or a stereoisomer thereof, a pharmaceutically acceptable salt thereof, a solvate thereof, a prodrug thereof, a metabolite thereof, or a deuterated derivative thereof: 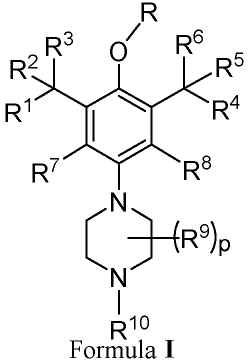
wherein, R is selected from hydrogen, COR^{a}, COCH(NH₂)R^{a}, COOR^{h1}, CH₂COOR^{h1}, a protecting group, PO(OR^{h1})(OR^{h2}) or CH₂OPO(OR^{h1}) (OR^{h2}); R^{a} is hydrogen, C₁₋₄ alkyl, C₂₋₄ alkenyl, C₂₋₄ alkynyl, 3-6 membered cycloalkyl, 3-6 membered heterocyclyl, aryl or heteroaryl; R^{h1}, R^{h2} are each independently selected from hydrogen, C₁₋₄ alkyl, 3-6 membered cycloalkyl, 3-6 membered heterocyclyl, aryl or heteroaryl;
R¹, R², R³, R⁴, R⁵, and R⁶ are each independently selected from hydrogen, halogen, C₁₋₄ alkyl or 3-6 membered cycloalkyl, provided that R¹, R², R³, R⁴, R⁵, and R⁶ are not simultaneously methyl;
R⁷, R⁸ are each independently selected from hydrogen, halogen, C₁₋₄ alkyl;
p is an integer from 0 to 8, and each R⁹ is independently selected from hydrogen, C₁₋₄ alkyl, halogenated C₁₋₄ alkyl; or p is an integer from 2 to 8, and two R⁹ groups are connected to form a ring, with the remaining R⁹ each independently selected from hydrogen, C₁₋₄ alkyl, halogenated C₁₋₄alkyl; or p is an integer from 1 to 9, and one R⁹ is connected to the nitrogen atom attached to R¹⁰ to form a quaternary ammonium salt, with the remaining R⁹ each independently selected from hydrogen, C₁₋₄ alkyl, halogenated C₁₋₄ alkyl;
R¹⁰ is selected from hydrogen, C₁₋₄ alkyl, (CR^{b1}R^{b2})mCOOR^{c}, (CR^{b1}R^{b2})mCONR^{d1}R^{d2}, (CR^{b1}R^{b2})nOR^{f}, (CR^{b1}R^{b2})nNR^{d1}R^{d2}, (CR^{b1}R^{b2})nOCOR^{e}, (CR^{b1}R^{b2})nNR^{d1}COR^{e}, (CR^{b1}R^{b2})mCO(CR^{b3}R^{b4})tCOOR^{c}, or (CR^{b1}R^{b2})mCO(CR^{b3}R^{b4})ₜCONR^{d1}R^{d2};
wherein, R^{b1}, R^{b2}, R^{b3}, and R^{b4} are each independently selected from hydrogen, halogen, C₁₋₄ alkyl, halogenated C₁₋₄ alkyl, C₂₋₄ alkenyl, C₂₋₄ alkynyl, 3-6 membered cycloalkyl, 3-6 membered heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is hydrogen, C₁₋₄ alkyl, halogenated C₁₋₄alkyl, C₂₋₄ alkenyl, C₂₋₄ alkynyl, 3-6 membered cycloalkyl, 3-6 membered heterocyclyl, aryl, or heteroaryl;
R^{e} is hydrogen, C₁₋₄ alkyl, C₂₋₄ alkenyl, C₂₋₄ alkynyl, 3-6 membered cycloalkyl, 3-6 membered heterocyclyl, aryl, or heteroaryl;
the alkyl, alkenyl, alkynyl, cycloalkyl, heterocyclyl, aryl, or heteroaryl in R^{b1}, R^{b2}, R^{b3}, R^{b4}, R^{c}, and R^{e} are unsubstituted or optionally substituted with one or more groups selected from halogen, C₁-₄ alkyl, C₁-₄ alkoxy, halogenated C₁-₄ alkyl, C₂-₄ alkenyl, C₂-₄ alkynyl, 3-6 membered cycloalkyl, 3-6 membered heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₄ alkyl;
R^{f} is selected from hydrogen, C₁-₄ alkyl, or halogenated C₁-₄ alkyl;
Rⁱ is selected from hydrogen, C₁-₄ alkyl, C₂-₄ alkenyl, C₂-₄ alkynyl, 3-6 membered cycloalkyl, or 3-6 membered heterocyclyl;
m is 0, 1, 2, 3, or 4;
n is 2, 3, or 4;
t is 1, 2, 3, or 4.

2. The compound according to claim 1, or a stereoisomer thereof, or a pharmaceutically acceptable salt thereof, or a solvate thereof, or a prodrug thereof, or a metabolite thereof, or a deuterated derivative thereof, **characterized in that** said compound has a structure of formula II: 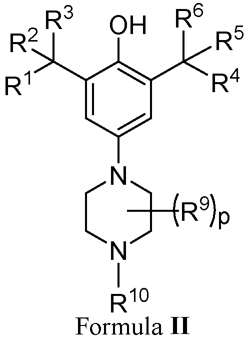
wherein, R¹, R², R³, R⁴, R⁵, R⁶ are each independently selected from hydrogen, halogen, C₁₋₄ alkyl or 3-6 membered saturated cycloalkyl;
p is an integer from 0 to 8, each R⁹ is independently selected from hydrogen, C₁₋₄ alkyl; or p is an integer from 2 to 8, and two R⁹ groups are connected to form a ring, with the remaining R⁹ each independently selected from hydrogen, C₁₋₄ alkyl; or p is an integer from 1 to 9, and one R⁹ is connected to the nitrogen atom attached to R¹⁰ to form a quaternary ammonium salt, with the remaining R⁹ each independently selected from hydrogen, C₁₋₄ alkyl, halogenated C₁₋₄ alkyl;
R¹⁰ is selected from hydrogen, C₁₋₄ alkyl, (CR^{b1}R^{b2})mCOOR^{c}, (CR^{b1}R^{b2})mCONR^{d1}R^{d2}, (CR^{b1}R^{b2})nOR^{f}, (CR^{b1}R^{b2})nNR^{d1}R^{d2}, (CR^{b1}R^{b2})nOCOR^{e}, (CR^{b1}R^{b2})nNR^{d1}COR^{e}, (CR^{b1}R^{b2})mCO(CR^{b3}R^{b4})tCOOR^{c}, or (CR^{b1}R^{b2})mCO(CR^{b3}R^{b4})tCONR^{d1}R^{d2};
wherein, R^{b1}, R^{b2}, R^{b3}, and R^{b4} are each independently selected from hydrogen, halogen, C₁₋₄ alkyl, halogenated C₁₋₄ alkyl, C₂₋₄ alkenyl, C₂₋₄ alkynyl, 3-6 membered cycloalkyl, 3-6 membered heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₄ alkyl, halogenated C₁₋₄ alkyl, C₂₋₄ alkenyl, C₂₋₄ alkynyl, 3-6 membered cycloalkyl, 3-6 membered heterocyclyl, aryl, or heteroaryl;
R^{e} is selected from hydrogen, C₁₋₄ alkyl, C₂₋₄ alkenyl, C₂₋₄ alkynyl, 3-6 membered cycloalkyl, 3-6 membered heterocyclyl, aryl, or heteroaryl;
the alkyl, alkenyl, alkynyl, cycloalkyl, heterocyclyl, aryl, or heteroaryl in R^{b1}, R^{b2}, R^{b3}, R^{b4}, R^{c}, and R^{e} are unsubstituted or optionally substituted with one or more groups selected from halogen, C₁-₄ alkyl, C₁-₄ alkoxy, halogenated C₁-₄ alkyl, C₂-₄ alkenyl, C₂-₄ alkynyl, 3-6 membered cycloalkyl, 3-6 membered heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₄ alkyl;
R^{f} is selected from hydrogen, C₁-₄ alkyl, or halogenated C₁-₄ alkyl;
Rⁱ is selected from hydrogen, C₁-₄ alkyl, C₂-₄ alkenyl, C₂-₄ alkynyl, 3-6 membered cycloalkyl, or 3-6 membered heterocyclyl;
m is 0, 1, 2, 3, or 4;
n is 2, 3, or 4;
t is 1, 2, 3, or 4.

3. The compound according to claim 2, or a stereoisomer thereof, or a pharmaceutically acceptable salt thereof, or a solvate thereof, or a prodrug thereof, or a metabolite thereof, or a deuterated derivative thereof, **characterized in that** said compound has a structure of formula III: 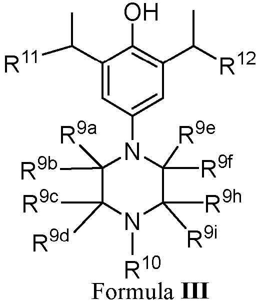
wherein, R¹¹, R¹² are each independently selected from methyl, 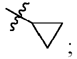
R^{9a} R^{9b}, R^{9c}, R^{9d}, R^{9e}, R^{9f}, R^{9h}, R⁹ⁱ are each independently selected from hydrogen, C₁-₄ alkyl; or, two groups among R^{9a}, R^{9b}, R^{9c}, R^{9a}, R^{9e}, R^{9f}, R^{9h}, and R⁹ⁱ attached to the same carbon atom may join together to form a spiro ring, with the remaining groups each independently selected from hydrogen or C₁-₄ alkyl; or, two groups among R^{9a}, R^{9b}, R^{9c}, R^{9d}, R^{9e}, R^{9f}, R^{9h}, and R⁹ⁱ attached to different carbon atoms may join together to form a bridged ring or a fused ring, with the remaining groups each independently selected from hydrogen or C₁₋₄ alkyl;
R¹⁰ is as described in claim 2.

4. The compound according to claim 3, or a stereoisomer thereof, or a pharmaceutically acceptable salt thereof, or a solvate thereof, or a prodrug thereof, or a metabolite thereof, or a deuterated derivative thereof, **characterized in that** said compound has a structure of formula IV-1 or formula IV-2: 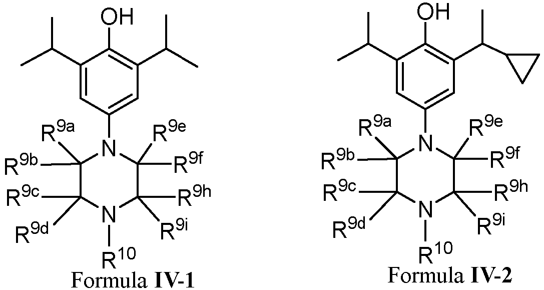
wherein, R^{9a}, R^{9b}, R^{9c}, R^{9a}, R^{9e}, R^{9f}, R^{9h}, and R⁹ⁱ are each independently selected from hydrogen or C₁-₃ alkyl; or, two groups among R^{9a}, R^{9b}, R^{9c}, R^{9a}, R^{9e}, R^{9f}, R^{9h}, and R⁹ⁱ attached to the same carbon atom may join together to form a spiro ring, with the remaining groups each independently selected from hydrogen or C₁-₃ alkyl; or, two groups among R^{9a}, R^{9b}, R^{9c}, R^{9a}, R^{9e}, R^{9f}, R^{9h}, and R⁹ⁱ attached to different carbon atoms may join together to form a bridged ring or a fused ring, with the remaining groups each independently selected from hydrogen or C₁-₃ alkyl; said spiro ring, bridged ring, or fused ring contains 2, 3, or 4 heteroatoms selected from N, O, or S;
R¹⁰ is selected from hydrogen, C₁₋₄ alkyl, (CR^{b1}R^{b2})mCOOR^{c}, (CR^{b1}R^{b2})mCONR^{d1}R^{d2}, (CR^{b1}R^{b2})nOR^{f}, (CR^{b1}R^{b2})nNR^{d1}R^{d2}, (CR^{b1}R^{b2})nOCOR^{e}, (CR^{b1}R^{b2})nNR^{d1}COR^{e}, (CR^{b1}R^{b2})mCO(CR^{b3}R^{b4})tCOOR^{c}, or (CR^{b1}R^{b2})mCO(CR^{b3}R^{b4})tCONR^{d1}R^{d2};
wherein, R^{b1}, R^{b2}, R^{b3}, and R^{b4} are each independently selected from hydrogen, halogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₄ alkyl, halogenated C₁₋₄ alkyl, C₂₋₄ alkenyl, C₂₋₄ alkynyl, 3-6 membered cycloalkyl, 3-6 membered heterocyclyl, aryl, or heteroaryl;
R^{e} is selected from hydrogen, C₁₋₃ alkyl, C₂₋₃ alkenyl, C₂₋₃ alkynyl, 3-6 membered saturated cycloalkyl, 3-6 membered saturated heterocyclyl, aryl, or heteroaryl;
the alkyl, alkenyl, alkynyl, saturated cycloalkyl, saturated heterocyclyl, cycloalkyl, heterocyclyl, aryl, or heteroaryl in R^{b1}, R^{b2}, R^{b3}, R^{b4}, R^{c}, and R^{e} are unsubstituted or optionally substituted with one or more groups selected from halogen, C₁₋₃ alkyl, C₁₋₃ alkoxy, halogenated C₁₋₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₃ alkyl;
R^{f} is selected from hydrogen, C₁-₃ alkyl, or halogenated C₁-₃ alkyl;
Rⁱ is selected from hydrogen, C₁-₃ alkyl, C₂-₃ alkenyl, C₂-₃ alkynyl, 3-6 membered saturated cycloalkyl, or 3-6 membered saturated heterocyclyl;
m is 0, 1, 2, 3, or 4;
n is 2, 3, or 4;
t is 1, 2, 3, or 4.

5. The compound according to claim 4, or a stereoisomer thereof, or a pharmaceutically acceptable salt thereof, or a solvate thereof, or a prodrug thereof, or a metabolite thereof, or a deuterated derivative thereof, **characterized in that** said compound has a structure of formula IV-1 or formula IV-2: 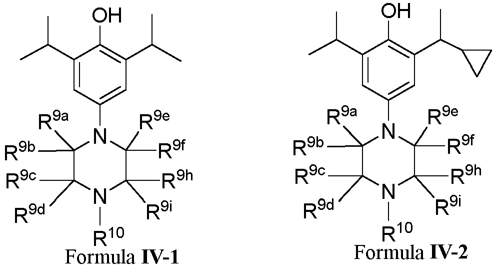
wherein, R^{9a}, R^{9b}, R^{9c}, R^{9d}, R^{9e}, R^{9f}, R^{9h}, and R⁹ⁱ are each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to the same carbon atom may join together to form a spiro ring, with the remaining groups each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to different carbon atoms may join together to form a bridged ring or a fused ring, with the remaining groups each independently selected from hydrogen or C₁-₃ alkyl; said spiro ring, bridged ring, or fused ring contains 2, 3, or 4 heteroatoms selected from N, O, or S;
R¹⁰ is selected from hydrogen, C₁₋₄ alkyl, (CR^{b1}R^{b2})mCOOR^{c}, (CR^{b1}R^{b2})mCONR^{d1}R^{d2}, (CR^{b1}R^{b2})ₙOR^{f}, (CR^{b1}R^{b2})nNR^{d1}R^{d2}, (CR^{b1}R^{b2})ₙOCOR^{e}, (CR^{b1}R^{b2})ₙNR^{d1}COR^{e}, (CR^{b1}R^{b2})ₘCO(CR^{b3}R^{b4})ₜCOOR^{c}, or (CR^{b1}R^{b2})ₘCO(CR^{b3}R^{b4})ₜCONR^{d1}R^{d2};
wherein, R^{b1}, R^{b2}, R^{b3}, and R^{b4} are each independently selected from hydrogen, halogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₃ alkyl, halogenated C₁₋₃ alkyl, C₂₋₃ alkenyl, C₂₋₃ alkynyls, 3-6 membered saturated cycloalkyl, 3-6 membered saturated heterocyclyl, aryl, or heteroaryl;
R^{e} is selected from hydrogen, C₁₋₃ alkyl, C₂₋₃ alkenyl, C₂₋₃ alkynyl, 3-6 membered saturated cycloalkyl, 3-6 membered saturated heterocyclyl, aryl, or heteroaryl;
the alkyl, alkenyl, alkynyl, saturated cycloalkyl, saturated heterocyclyl, aryl, or heteroaryl in R^{b1}, R^{b2}, R^{b3}, R^{b4}, R^{c}, and R^{e} are unsubstituted or optionally substituted with one or more groups selected from halogen, C₁-₃ alkyl, C₁-₃ alkoxys, halogenated C₁-₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₃ alkyl;
R^{f} is selected from hydrogen, C₁-₃ alkyl, or halogenated C₁-₃ alkyl;
Rⁱ is selected from hydrogen, C₁-₃ alkyl, C₂-₃ alkenyl, C₂-₃ alkynyl, 3-6 membered saturated cycloalkyl, or 3-6 membered saturated heterocyclyl;
m is 0, 1, 2, 3, or 4;
n is 2, 3, or 4;
t is 1, 2, 3, or 4.

6. The compound according to anyone of claims 3-5, or a stereoisomer thereof, or a pharmaceutically acceptable salt thereof, or a solvate thereof, or a prodrug thereof, or a metabolite thereof, or a deuterated derivative thereof, **characterized in that** said 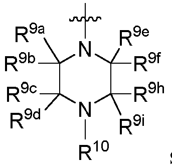 structural fragment is one of the following structures:

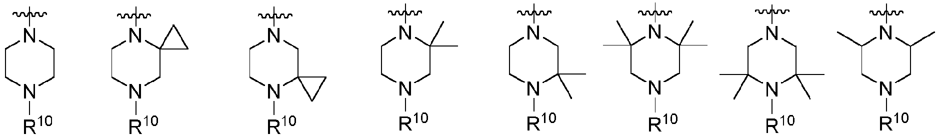

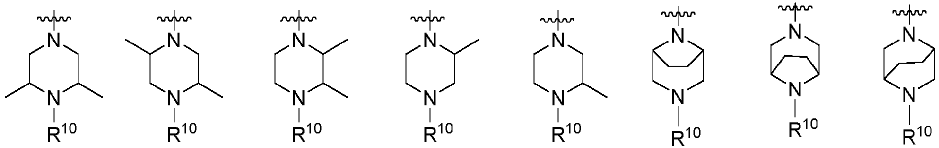

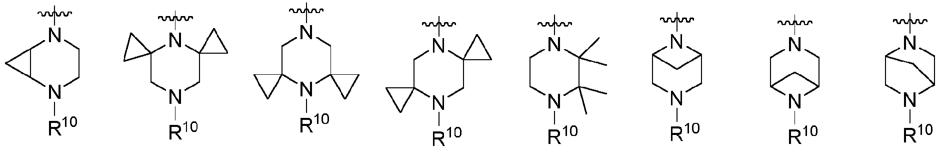

7. The compound according to claim 5, or a stereoisomer thereof, or a pharmaceutically acceptable salt thereof, or a solvate thereof, or a prodrug thereof, or a metabolite thereof, or a deuterated derivative thereof, **characterized in that** said compound has a structure of formula V-1 or formula V-2: 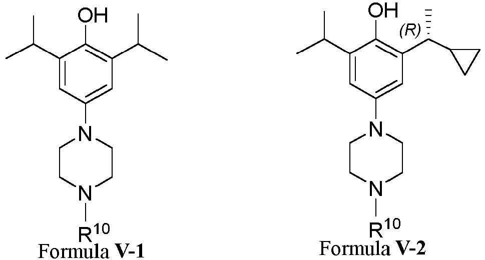
wherein, R¹⁰ is selected from hydrogen, C₁₋₄ alkyl, (CR^{b1}R^{b2})ₘCOOR^{c}, (CR^{b1}R^{b2})ₘCONR^{d1}R^{d2}, (CR^{b1}R^{b2})ₙOR^{f}, (CR^{b1}R^{b2})nNR^{d1}R^{d2}, (CR^{b1}R^{b2})ₙOCOR^{e}, (CR^{b1}R^{b2})ₙNR^{d1}COR^{e}, (CR^{b1}R^{b2})ₘCO(CR^{b3}R^{b4})ₜCOOR^{c}, or (CR^{b1}R^{b2})mCO(CR^{b3}R^{b4})ₜCONR^{d1}R^{d2};
wherein, R^{b1}, R^{b2}, R^{b3}, and R^{b4} are each independently selected from hydrogen, halogen, C₁₋₂ alkyl, halogenated C₁₋₂ alkyl, C₂₋₃ alkenyl, C₂₋₃ alkynyl, 3-6 membered saturated cycloalkyl, 3-6 membered saturated heterocyclyl, phenyl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₂ alkyl, halogenated C₁₋₂ alkyl, C₂₋₃ alkenyl, C₂₋₃ alkynyl, 3-6 membered saturated cycloalkyl, 3-6 membered saturated heterocyclyl, phenyl, or heteroaryl;
R^{e} is selected from hydrogen, C₁₋₂ alkyl, C₂₋₃ alkenyl, C₂₋₃ alkynyl, 3-6 membered saturated cycloalkyl, 3-6 membered saturated heterocyclyl, phenyl, or heteroaryl;
the alkyl, alkenyl, alkynyl, saturated cycloalkyl, saturated heterocyclyl, phenyl, or heteroaryl in R^{b1}, R^{b2}, R^{b3}, R^{b4}, R^{c}, and R^{e} are unsubstituted or optionally substituted with one or more groups selected from halogen, C₁-₂ alkyl, C₁-₂ alkoxy, halogenated C₁-₂ alkyl, vinyl, ethynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₂ alkyl;
R^{f} is selected from hydrogen, C₁-₂ alkyl, or halogenated C₁-₂ alkyl;
Rⁱ is selected from hydrogen, C₁-₂ alkyl, vinyl, ethynyl, 3-6 membered saturated cycloalkyl, or 3-6 membered saturated heterocyclyl;
m is 0, 1, 2, 3, or 4;
n is 2, 3, or 4;
t is 1, 2, 3, or 4.

8. The compound according to claim 7, or a stereoisomer thereof, or a pharmaceutically acceptable salt thereof, or a solvate thereof, or a prodrug thereof, or a metabolite thereof, or a deuterated derivative thereof, **characterized in that** said 3-6-membered saturated cycloalkyl is selected from 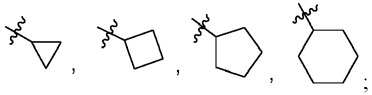 said 3-6-membered saturated heterocyclyl is selected from 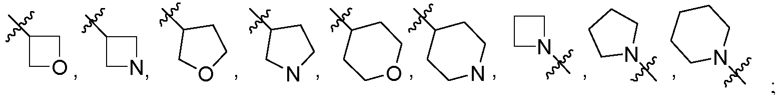 said 3-6-membered cyclic structure is selected from cyclopropyl, cyclobutyl, cyclopentyl, cyclohexy; said halogen is selected from fluorine, chlorine, bromine.

9. The compound according to claim 5, or a stereoisomer thereof, or a pharmaceutically acceptable salt thereof, or a solvate thereof, or a prodrug thereof, or a metabolite thereof, or a deuterated derivative thereof, **characterized in that** said compound has a structure of formula VI-1 or formula VI-2: 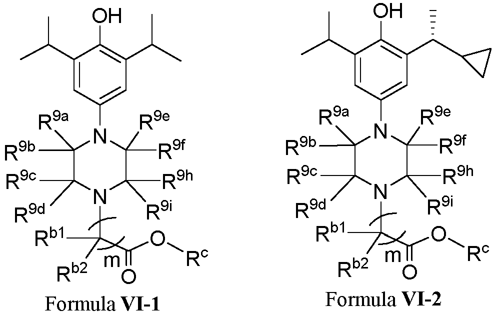
wherein, R^{9a}, R^{9b}, R^{9c}, R^{9d}, R^{9e}, R^{9f}, R^{9h}, and R⁹ⁱ are each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to the same carbon atom may join together to form a spiro ring, with the remaining groups each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to different carbon atoms may join together to form a bridged ring or a fused ring, with the remaining groups each independently selected from hydrogen or C₁-₃ alkyl; said spiro ring, bridged ring, or fused ring contains 2, 3, or 4 heteroatoms selected from N, O, or S;
m is 0, 1, 2, 3, or 4;
R^{b1}, R^{b2} are each independently selected from hydrogen, halogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, or heteroaryl;
the alkyl, alkenyl, alkynyl, saturated cycloalkyl, saturated heterocyclyl, aryl, or heteroaryl in R^{b1}, R^{b2}, and R^{c} are unsubstituted or optionally substituted with one or more groups selected from halogen, C₁-₃ alkyl, C₁-₃ alkoxy, halogenated C₁-₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₃ alkyl;
R^{f} is selected from hydrogen, C₁-₃ alkyl, or halogenated C₁-₃ alkyl;
Rⁱ is selected from hydrogen, C₁-₃ alkyl, C₂-₃ alkenyl, C₂₋₃ alkynyl, 3-6 membered saturated cycloalky, or 3-6 membered saturated heterocyclyl.

10. The compound according to claim 1 or 4, or a stereoisomer thereof, or a pharmaceutically acceptable salt thereof, or a solvate thereof, or a prodrug thereof, or a metabolite thereof, or a deuterated derivative thereof, **characterized in that** said compound has a structure of formula VI-1 or formula VI-2: 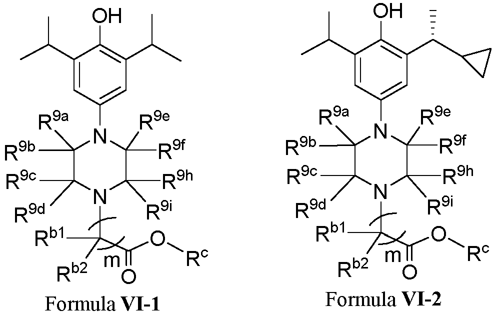
wherein, R^{9a}, R^{9b}, R^{9c}, R^{9d}, R^{9e}, R^{9f}, R^{9h}, and R⁹ⁱ are each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to the same carbon atom may join together to form a spiro ring, with the remaining groups each independently selected from hydrogen or C₁-₃ alkyl; or, two groups among R^{9a}, R^{9b}, R^{9c}, R^{9d}, R^{9e}, R^{9f}, R^{9h}, and R⁹ⁱ attached to different carbon atoms may join together to form a bridged ring or a fused ring, with the remaining groups each independently selected from hydrogen or C₁-₃ alkyl; said spiro ring, bridged ring, or fused ring contains 2, 3, or 4 heteroatoms selected from N, O, or S;
m is 0, 1, 2, 3, or 4;
R^{b1}, R^{b2} are each independently selected from hydrogen, halogen, C₁₋₃ alkyl, halogenated C₁₋₃ alkyl, C₂₋₃ alkenyl, C₂₋₃ alkynyl, 3-6 membered saturated cycloalkyl, 3-6 membered saturated heterocyclyl, aryl, heteroaryl, CN, OR^{f}, SR^{f}, or NR^{d1}R^{d2}; or, R^{b1} and R^{b2}, together with the carbon atom to which they are attached, form a 3-6 membered cyclic structure containing 0 or 1 heteroatom selected from N, O, or S;
R^{c} is selected from hydrogen, C₁₋₄ alkyl, halogenated C₁₋₄ alkyl, C₂₋₃ alkenyl, C₂₋₃ alkynyl, 3-6 membered saturated cycloalkyl, 3-6 membered saturated heterocyclyl, aryl, or heteroaryl;
the alkyl, alkenyl, alkynyl, saturated cycloalkyl, saturated heterocyclyl, aryl, or heteroaryl in R^{b1}, R^{b2}, and R^{c} are unsubstituted or optionally substituted with one or more groups selected from halogen, C₁-₃ alkyl, C₁-₃ alkoxy, halogenated C₁-₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2} are each independently selected from hydrogen or C₁-₃ alkyl;
R^{f} is selected from hydrogen, C₁-₃ alkyl, or halogenated C₁-₃ alkyl;
Rⁱ is selected from hydrogen, C₁-₃ alkyl, C₂-₃ alkenyl, C₂-₃ alkynyl, 3-6 membered saturated cycloalky, or 3-6 membered saturated heterocyclyl.

11. The compound according to claim 10, or a stereoisomer thereof, or a pharmaceutically acceptable salt thereof, or a solvate thereof, or a prodrug thereof, or a metabolite thereof, or a deuterated derivative thereof, **characterized in that** said 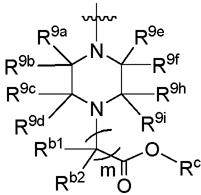 structural fragment is one of the following structures: 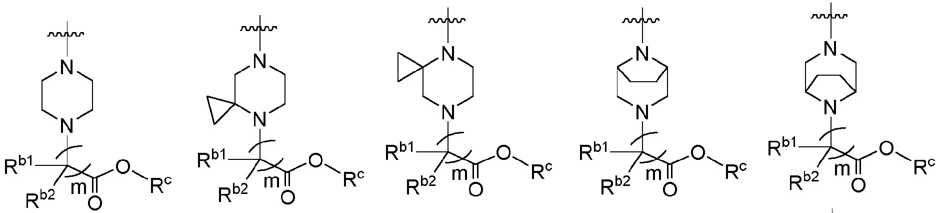 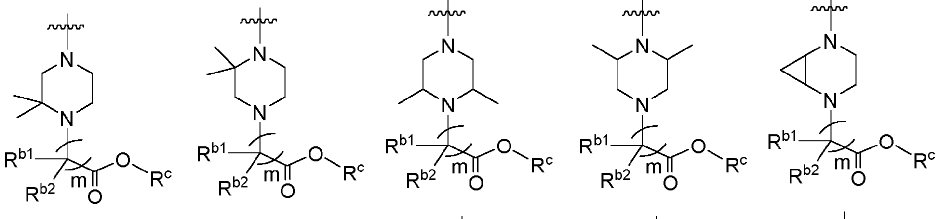 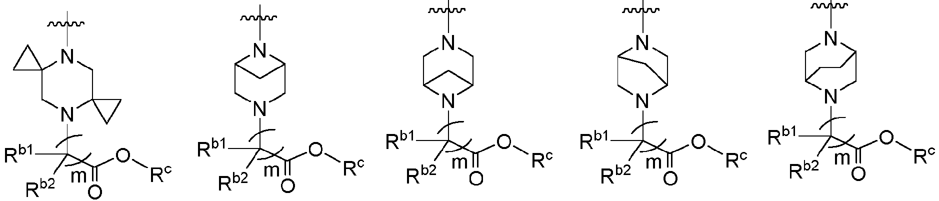
m is 1, 2 or 3;
R^{b1}, R^{b2} are each independently selected from hydrogen, halogen, C₁₋₃ alkyl, halogenated C₁₋₃ alkyl, 3-6 membered saturated cycloalkyl, 4-6 membered saturated heterocyclyl; or, R^{b1} and R^{b2}, together with the carbon atom to which they are attached, form a 3-4 membered cyclic structure containing 0 or 1 heteroatom selected from N, O, or S;
R^{c} is selected from hydrogen, C₁₋₄ alkyl, halogenated C₁₋₄ alkyl, 3-6 membered saturated cycloalkyl, 3-6 membered saturated heterocyclyl, aryl, or heteroaryl;
the alkyl, saturated cycloalkyl, saturated heterocyclyl, aryl, or heteroaryl in R^{b1}, R^{b2}, and R^{c} are unsubstituted or optionally substituted with one or more groups selected from halogen, C₁-₃ alkyl, C₁-₃ alkoxy, halogenated C₁-₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2}, R^{f}, and Rⁱ are as described in claim 10.

12. The compound according to claim 10, or a stereoisomer thereof, or a pharmaceutically acceptable salt thereof, or a solvate thereof, or a prodrug thereof, or a metabolite thereof, or a deuterated derivative thereof, **characterized in that** said compound has a structure of formula VII-1 or formula VII-2: 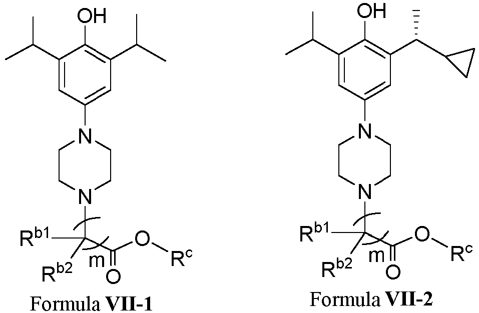
wherein, m is 1, 2;
R^{b1}, R^{b2} are each independently selected from hydrogen, halogen, C₁₋₃ alkyl, halogenated C₁₋₃ alkyl, 3-6 membered saturated cycloalkyl, 4-6 membered saturated heterocyclyl;
R^{c} is selected from hydrogen, C₁₋₄ alkyl, halogenated C₁₋₄ alkyl, 3-6 membered saturated cycloalkyl, 3-6 membered saturated heterocyclyl, aryl, or heteroaryl;
the alkyl, saturated cycloalkyl, saturated heterocyclyl, aryl, or heteroaryl in R^{b1}, R^{b2}, and R^{c} are unsubstituted or optionally substituted with one or more groups selected from halogen, C₁-₃ alkyl, C₁-₃ alkoxy, halogenated C₁-₃ alkyl, C₂-₃ alkenyl, C₂-₃ alkynyl, 3-6 membered saturated cycloalkyl, 3-6 membered saturated heterocyclyl, CN, NO₂, OR^{f}, SR^{f}, NR^{d1}R^{d2}, CORⁱ, COOR^{f}, OCORⁱ, CONR^{d1}R^{d2}, NR^{d1}CORⁱ, NR^{d1}SO₂Rⁱ, or SO₂Rⁱ;
R^{d1}, R^{d2}, R^{f}, and Rⁱ are as described in claim 10.

13. The compound according to anyone of claims 10-12, or a stereoisomer thereof, or a pharmaceutically acceptable salt thereof, or a solvate thereof, or a prodrug thereof, or a metabolite thereof, or a deuterated derivative thereof, **characterized in that**, in formula VI-1, formula VI-2, formula VII-1, or formula VII-2:
m is 1 or 2;
R^{b1}, R^{b2} are each independently selected from hydrogen, methyl, ethyl, n-Propyl, isopropyl, cyclopropyl;
R^{c} is selected from methyl, ethyl, n-propyl, isopropyl, tert-butyl, CF₃, cyclopropyl,

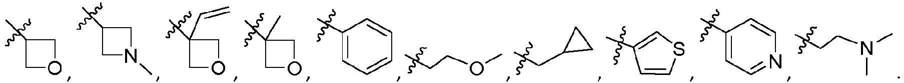

14. The compound according to claim 1, or a stereoisomer thereof, or a pharmaceutically acceptable salt thereof, or a solvate thereof, or a prodrug thereof, or a metabolite thereof, or a deuterated derivative thereof, **characterized in that** the structure of said compound is selected from: 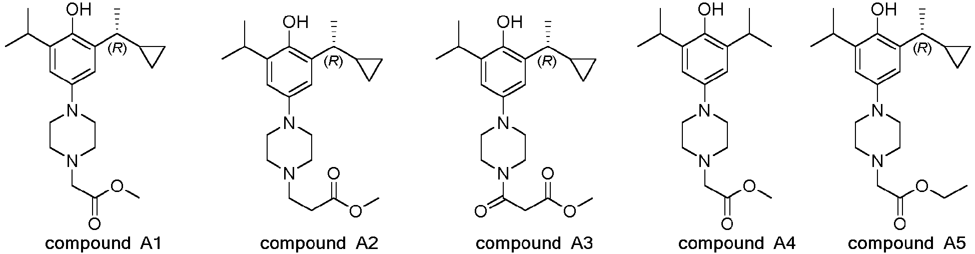 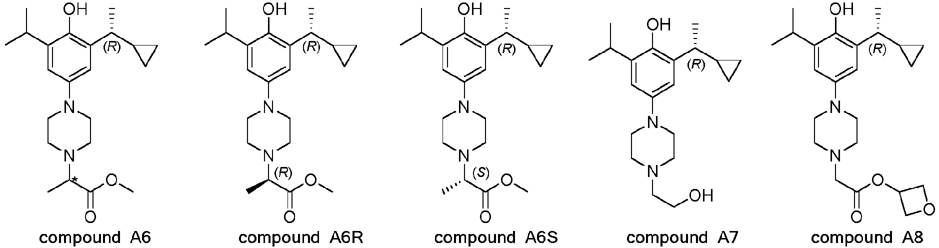 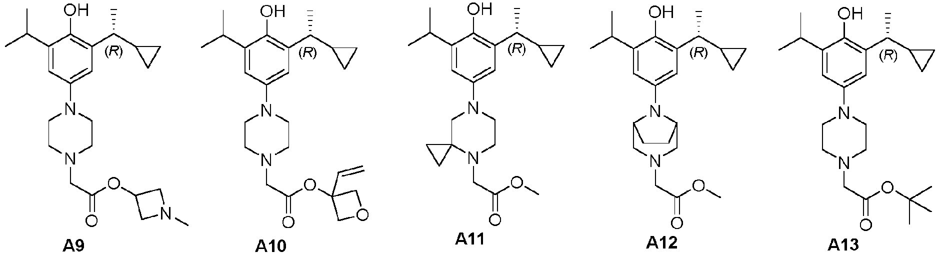 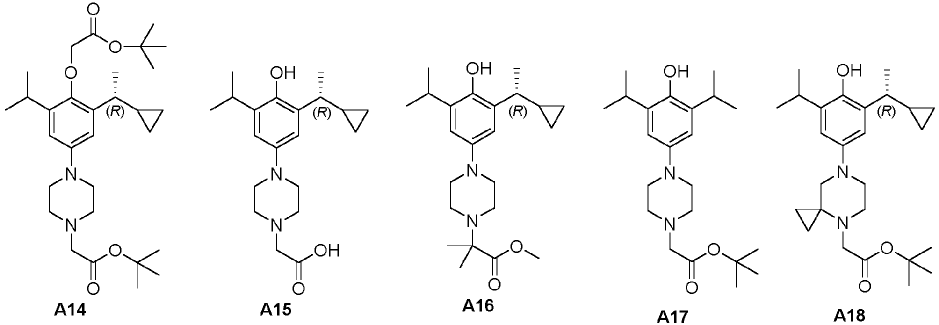 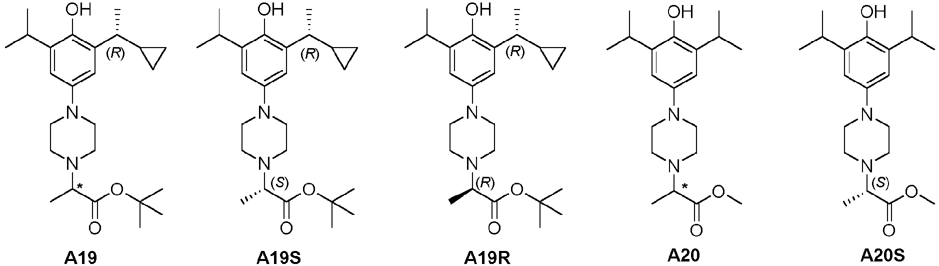 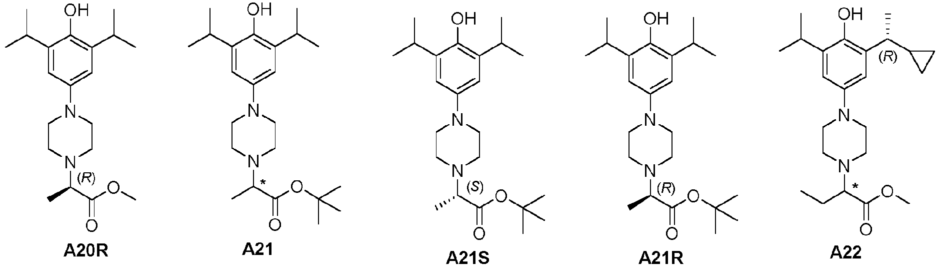 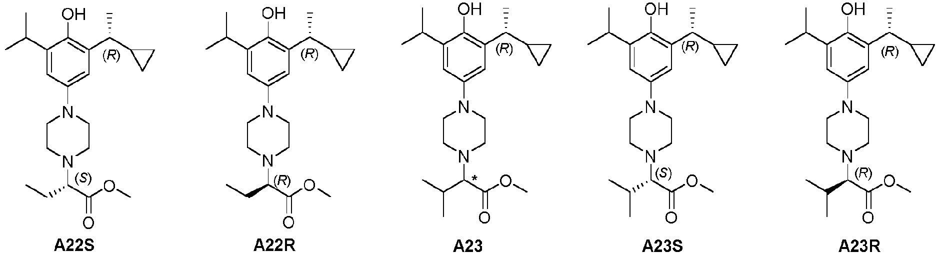 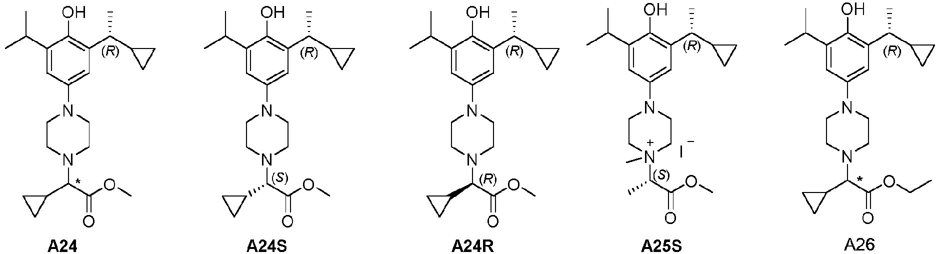 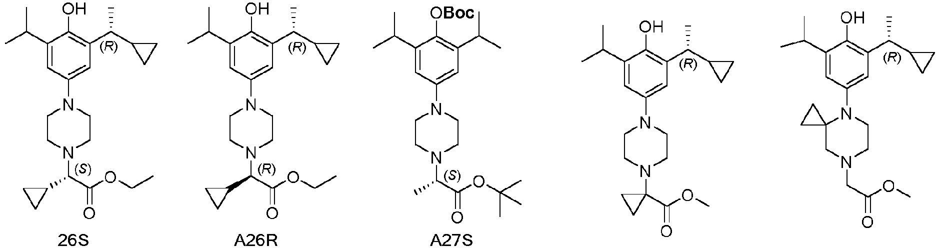 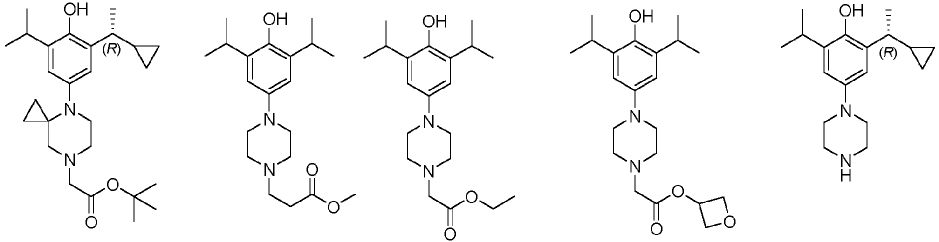 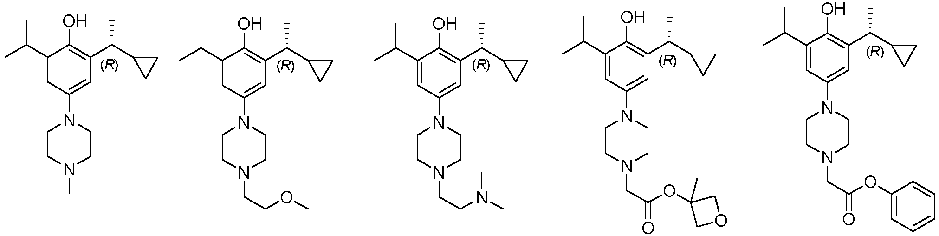 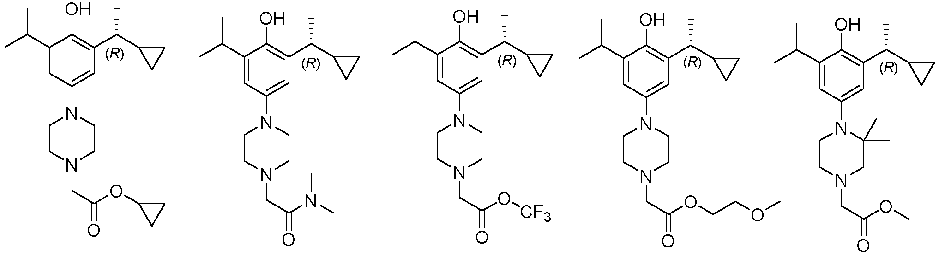 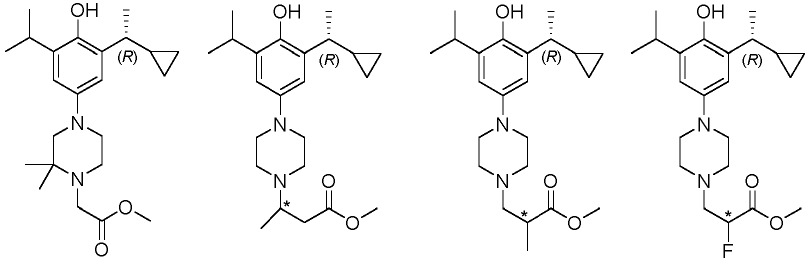 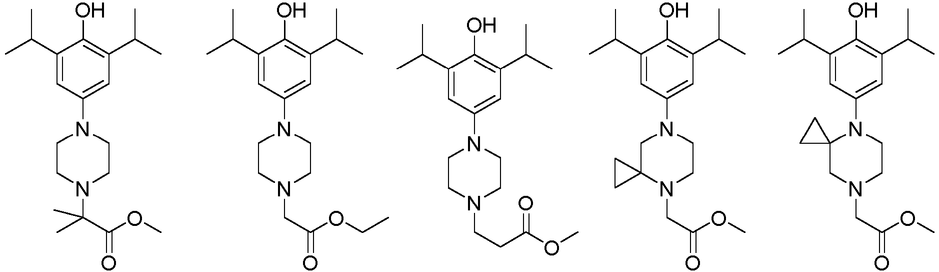 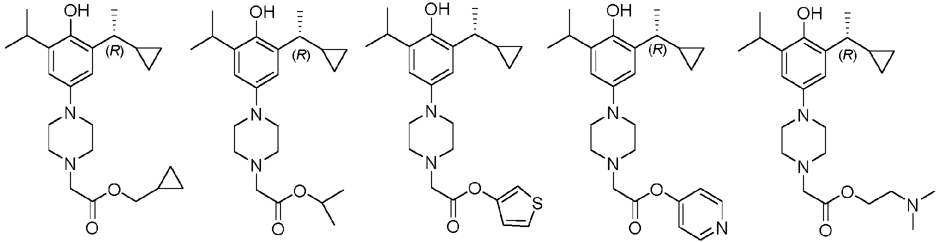 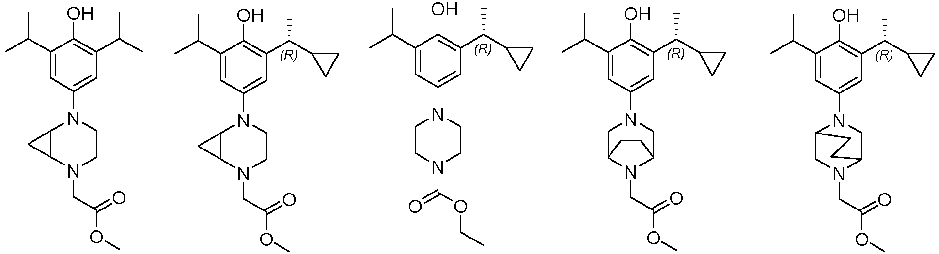 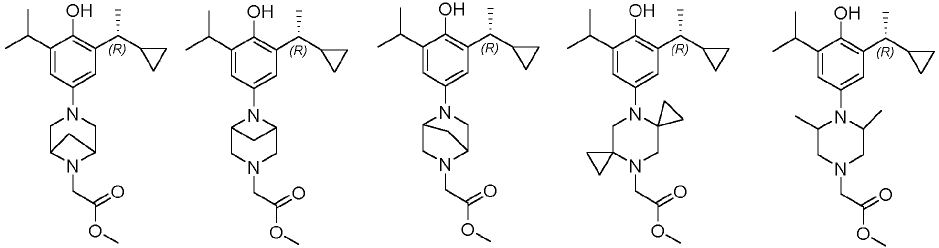 "*" means a chiral center.

15. The compound according to anyone of claims 1-14, or a stereoisomer thereof, or a pharmaceutically acceptable salt thereof, or a solvate thereof, or a prodrug thereof, or a metabolite thereof, or a deuterated derivative thereof, **characterized in that** said pharmaceutically acceptable salt is selected from acetate, adipate, aspartate, benzoate, benzene sulfonate, bicarbonate, carbonate, bisulfate, sulfate, borate, camphorsulfonate, citrate, cyclamate, edisylate, ethane sulfonate, formate, fumarate, glucoheptonate, gluconate, glucuronate, hexafluorophosphate, hydrochloride, hydrobromide, hydroiodide, hydroxyethylsulfonate, lactate, malate, maleate, malonate, mesylate, methylsulfate, naphthylate, napsylate, nicotinate, nitrate, orotate, oxalate, palmitate, pamoate, phosphate, hydrogen phosphate, dihydrogen phosphate, pyroglutamate, saccharate, stearate, succinate, tannate, tartrate, tosylate, trifluoroacetate, xinafoate, methanesulfonate, or p-toluenesulfonate.

16. A drug, **characterized in that** said drug is prepared by using the compound according to anyone of claims 1-15, or a stereoisomer thereof, or a pharmaceutically acceptable salt thereof, or a solvate thereof, or a prodrug thereof, or a metabolite thereof, or a deuterated derivative thereof as active ingredients, with addition of pharmaceutically acceptable excipients.

17. Use of compound according to anyone of claims 1-15, or a stereoisomer thereof, or a pharmaceutically acceptable salt thereof, or a solvate thereof, or a prodrug thereof, or a metabolite thereof, or a deuterated derivative thereof, in preparation of a drug for inducing sedation, hypnosis, and/or anesthesia, and/or for controlling status epilepticus.
